# EUROPEAN PATENT APPLICATION

(11) **EP 1 293 572 A1**
(43) Date of publication of application: **19.03.2003**
(21) Application number: 01122252.8
(22) Date of filing: 17.09.2001
(51) Int. Cl.: C12N 15/82, C12N 15/29, C07K 14/415, C12N 5/10, C07K 14/16, C12Q 1/68, G01N 33/53, A01H 5/00

(54) **Method of altering plant secondary metobolite biosynthesis in plant cells**

(71) Applicant: Syngenta Participations AG, 4058 Basel (CH)
(72) Inventor: Welsch, Ralf, 7910 Freiburg (DE); Beyer, Peter, 79423 Heitersheim (DE)
(74) Representative: Bastian, Werner Maria

(57) **Abstract**

The present invention relates to methods and materials, particularly nucleic acid binding proteins, for altering the levels of plant secondary metabolites such as vitamins and/or their precursors. It further relates to plants, which have been modified using such materials and methods.

## Description

The present invention relates to methods and materials, particularly nucleic acid binding proteins, for altering the levels of plant secondary metabolites such as vitamins and/or their precursors. It further relates to plants, which have been modified using such materials and methods.

### Background of the invention

Plant secondary metabolites represent a very large and diverse group of compounds and include the alkaloids (such as terpenoid indole alkaloids and indole alkaloids), phenolic compounds (such as quinones, lignans, and flavonoids), terpenoid compounds (such as monoterpenoids, iridoids and sesquiterpenoids), vitamins and their precursors (such as carotenoid compounds and tocotrienols/tocopherols), and the like.

Many plant secondary metabolites have value as pharmaceuticals, food- colouring agents, flavours and fragrances.

PCT/NL00/00075 (WO 00/46383) describes a method of modulating certain secondary metabolite biosynthesese in plant cells and focuses on the generation of alkaloids in Catharanthus roseus plants transgenic for a nucleic acid binding proteins, namely, AP-2 domain transcription factors. The transcription factors of the prior art do not appear to be related to transcription factors of the present invention since inter alia, i) they do not possess ATCTA binding domains ii) display a low percentage identity with transcription factors of the present invention and iii) are associated with secondary metabolite pathways that do not appear to be related to the secondary metabolite pathways of interest in the present invention. In addition, it is noted that the technical teaching in the prior art specification and figures relates to work conducted in a Catharanthus roseus background.

PCT/NL00/00075 teaches that transcription factors are proteins that bind to DNA in a sequence specific manner and recognize specific elements located in the promoter and/or enhancer regions of the corresponding genes (page 35, lines 23 - 30). Transcription factors therefore comprise DNA binding domains that are capable of binding to specific elements of their target genes. The specification also teaches that transcription factors are classified in families based on conserved features of their binding domains and that these binding domains vary within families. The differences can be very small. As a result of such differences it is stated in PCT/NL00/00075 that it is impossible to predict if a certain DNA sequence will bind a certain transcription factor family member with high affinity or not, or conversely, if any given amino acid sequence of a transcription factor will bind to a DNA sequence with high affinity.

The characterizing of the action of transcription factors with corresponding DNA binding domains in order to obtain desirable alterations in plant secondary metabolite levels is therefore not a simple task and is fraught with problems. A problem is that it is not possible to predict with any high degree of expectation what benefits the use, generally over-expression, of a gene encoding any one particular transcription factor will provide in terms of altered levels of desired compounds.

### Disclosure of the invention

The present inventors have succeeded in characterizing previously unrecognised DNA sequence motifs in several promoter regions of naturally occurring genes and have demonstrated for the first time that the binding of some of these previously unrecognised DNA motifs with certain transcription factors that appear to have a high affinity for such motifs can give rise to altered levels of secondary plant metabolites selected from isoprenoid-related compounds such as vitamin A and precursors thereof, and of tocotrienol/tocopherol-related compounds such as vitamin E and precursors thereof, and vitamin K-related compounds, in plants transgenic for such transcription factors. Typically, the levels of these compounds are elevated relative to the corresponding wild type or native levels. By using such transcription factors the inventors have found an elegant means of being able to alter the levels of certain vitamins and/or their precursors by stimulating the synthesis of vitamins such as vitamins A, E and/or K through the natural metabolic pathway(s) of the plant.

The present inventors have now discovered that transcription factors having at least one ATCTA DNA-binding domain, and preferably two ATCTA DNA-binding domains, preferably located in tandem, are suitable for altering the levels of certain vitamins and/or their precursors in plant cells.

The invention results from the cloning of the RAP2.2 transcription factor gene and the provision of homologues thereof.

In various aspects the invention relates to nucleic acid encoding a polypeptide with RAP2.2 function. "RAP.2 function" refers to the ability of the *Rap.2* gene and polypeptide expression products thereof to stimulate the production of carotenoid compounds and/or tocotrienol/tocopherol compounds in plant cell tissues. The term "RAP2.2 function" may be used to refer to sequences which confer a RAP2.2 phenotype in a plant as defined herein, the term "RAP2.2 function" may be used to refer to forms of *RAP2.2* sequences which are capable of conferring a *RAP2.2* phenotype in a plant. *Rap2.2* function can be determined by assessing the level of total carotenoids and/or tocotrienol/tocopherol compounds, for example, as described hereinafter in the examples against the relevant total carotenoid and/or tocotrienol/tocopherol levels found in corresponding wild type or native plants of the same species.

As used herein, the term "plant" generally includes eukaryotic alga, embryophytes including *Bryophyta, Pteridophyta* and Spermatophyta such as *Gymnospermae* and *Angiospermae*, the latter including *Magnoliopsida, Rosopsida* (eu-"dicots"), *Liliopsida* ("monocots"). Representative and preferred examples include grain seeds, e.g. rice, wheat, barley, oats, amaranth, flax, triticale, rye, corn, and other grasses; oil seeds, such as oilseed *Brassica* seeds, cotton seeds, soybean, safflower, sunflower, coconut, palm, and the like; other edible seeds or seeds with edible parts including pumpkin, squash, sesame, poppy, grape, mung beans, peanut, peas, beans, radish, alfalfa, cocoa, coffee, hemp, tree nuts such as walnuts, almonds, pecans, chick-peas etc.. Furthermore, potatoes, carrots, sweet potatoes, tomato, pepper, cassava, willows, oaks, elm, maples, apples, bananas; ornamental flowers such as lilies, orchids, sedges, roses, buttercups, petunias, phlox, violets, sunflowers, and the like. Generally, the present invention is applicable in ornamental species as well as species cultivated for food, fibre, wood products, tanning materials, dyes, pigments, gums, resins, latex products, fats, oils, drugs, beverages, and the like. Preferably, the target plant selected for transformation is cultivated for food, such as, for example, grains, roots, legumes, nuts, vegetables, tubers, fruits, spices and the like.

Preferably, suitable dicots include members of the Cruciferae such as Arabidopsis and the closely related Brassicas, tobacco, tomato, potato and grape vine. Particularly preferred dicots are tomato and Brassicas such as green cabbage, cauliflower, Brussels sprouts, curly kale, broccoli, white cabbage and red cabbage. Preferably, suitable monocots include members of the Graminaeae such as wheat, barley, rice, corn, oat, amaranth, flax, triticale, rye, and other grasses. Further included are coconut, palm and banana.

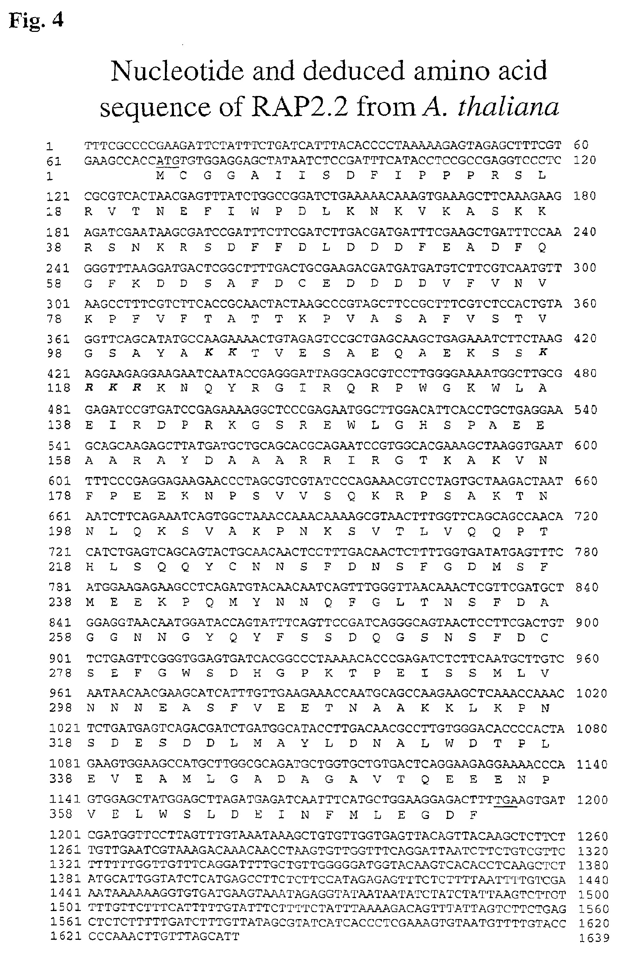

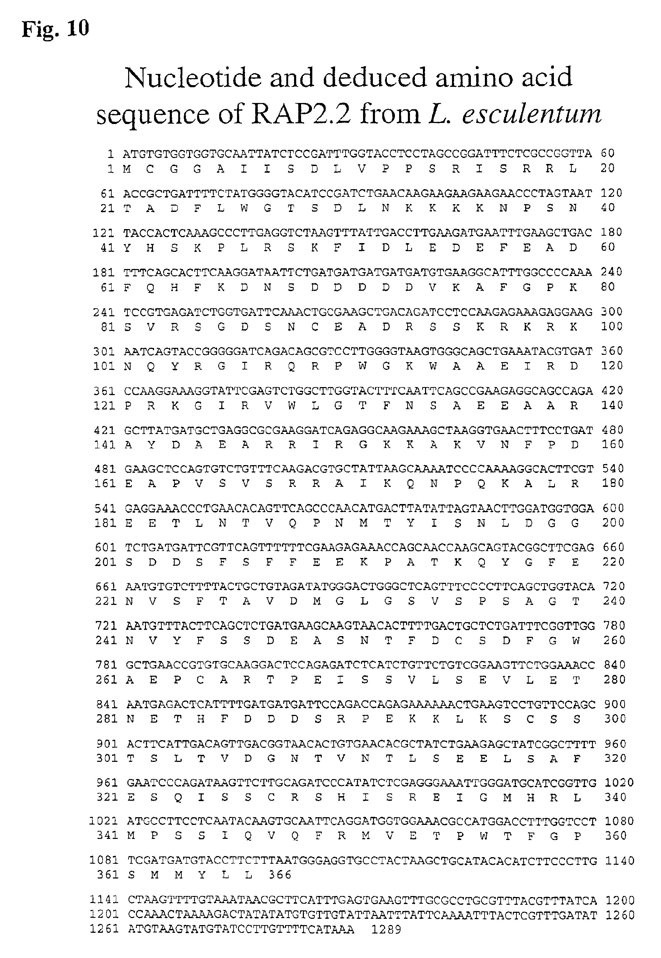

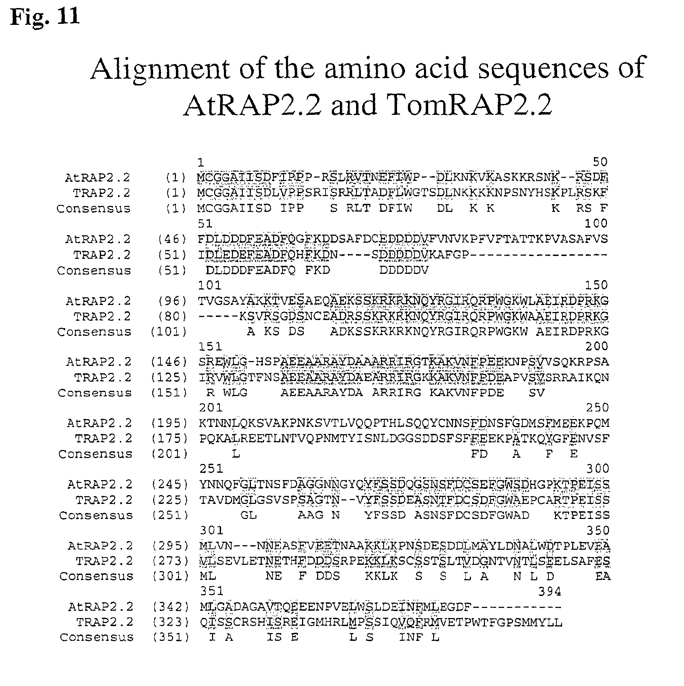

A polynucleotide according to the invention may encode a polypeptide including the consensus amino acid sequence shown in Figure 11 (section 2) and capable of binding to a promoter region comprising at least one ATCTA domain wherein said promoter region is operably associated with a coding sequence of a gene of a carotenoid synthesis pathway and/or a tocotrienol/tocopherol synthesis pathway such that when the said polypeptide is bound to the promoter region carotenoid and/or tocotrienol/tocopherol synthesis is enhanced. Preferably, vitamin A, vitamin E and/or vitamin K synthesis is enhanced. Preferably, the polynucleotide encodes an amino acid sequence comprising the tomato or Arabidopsis polypeptide sequence shown in Figure 11 (section 2). The coding sequence may be selected from those shown included in Figures 4 or 10 (section 2) or it may be a mutant, variant, derivative or allele of the sequences shown. The sequences may differ from those of Figures 4 and 10 (section 2) shown by a change which is one or more of addition, insertion, deletion and substitution of one or more nucleotides of the sequences shown. Changes to a nucleotide sequence may result in an amino acid change at the protein level, or not, as determined by the genetic code. Thus, nucleic acid according to the present invention may include sequences different from the sequences shown in Figures 4 or 10 (section 2) yet encode a polypeptide with the same amino acid sequence (i.e. the coding sequence may be "degeneratively equivalent").

Thus *RAP2.2* mutants, variants, fragments, derivatives, alleles and homologues thereof which when expressed give rise to a corresponding increase in the levels of carotenoids and/or tocotrienols/tocopherols for plant tissues transgenic therefore. In the agronomic situation the major interest will be one of raising the vitamin content in edible plants, such as vegetables, of for example vitamins A, E and/or K, in plant tissues relative to the vitamin content of conventionally available edible plants.

In particular, homologues of the particular *Rap2.2* sequences provided herein (see e.g. Figures 4 and 10 (section 2)) are provided by the present invention as are mutants, variants, fragments and derivatives of such homologues (and comments made above in relation to such mutants etc also apply in relation to mutants etc of homologues). Such homologues are readily obtainable by use of the disclosures made herein. Thus the present invention also extends to nucleic acid molecules which include a nucleic acid sequence encoding a *RAP2.2* homologue obtainable using a nucleotide sequence derived from, or as shown in Figure 4 or Figure 10 (section 2), or obtainable using amino acid sequences as shown in Figure 4 or Figure 10 (section 2). The *RAP2.2* homologue may at the nucleotide level have homology with a nucleotide sequence of Figure 4 or 10 (section 2), or may encode a polypeptide which has homology with the polypeptides of which the amino acid sequences are shown in Figures 4 or 10 (section 2), preferably at least about 50%, 51%, 52%, 53%, 54% or 55%, or at least about 60%, or at least about 65%, or at least about 70%, or at least about 75%, or at least about 80% homology, or at least about 90% homology. Most preferably, at least about 95% or greater homology. (Determination of homology at the amino acid level is discussed further below.)

In certain embodiments, a polypeptide allele, variant, derivative, mutant derivative, mutant or homologue of the specific sequences may show little overall homology, say about 20%, or about 25%, or about 30%, or about 35%, or about 40% or about 45%, with the tomato or Arabidopsis amino acid sequences of Figures 4 and 10 (section 2). Preferably, any such polypeptide alleles, variants, derivatives, mutant derivatives, mutants or homologues have 95%, 96%, 97%, 98%, 99%, or 100% identity with the consensus sequence shown'in Figure 11 (section 2). However, in functionally significant domains or regions the amino acid percentage identity may be much higher. Putative functionally significant domains or regions can be identified using processes of bioinformatics, including comparison of the sequences of homologues. Functionally significant domains or regions of different polypeptides may be combined for expression from encoding nucleic acid as a fusion protein. For example, particularly advantageous or desirable properties of different homologues may be combined in a hybrid protein, such that the resultant expression product, with RAP2.2 function, may include fragments of various parent proteins. RAP2.2 fragments that are functional in the context of the present invention are also included within the ambit of the invention.

*Rap2.2*-derived oligonucleotide primers (as authentic or degenerate sequences) may be used to isolate *RaP2.2* homologues from many different plants, including dicot and monocots, such as barley, wheat, maize, oats, rice, tomatoes, melons, Brassica's as provided herein, capsicums, lettuces, grape vines, ornamentals. Once a corresponding *RAP2.2* gene is cloned it may be expressed to assess its ability to alter, preferably enhance, the levels of total carotenoid and/or tocotrienol/tocopherol compounds found in plant tissue.

The obtaining of homologues is later discussed herein, but briefly here it should be pointed out that the nucleotide sequence information provided herein, or any part thereof, may be used in a data-base search to find homologous sequences, expression products of which can be tested for *RAP2.2* (or *rap2.2*) function. These may have ability to complement a *RAP2.2* (or *rap2.2*) phenotype in a plant or may, upon expression in a plant, confer such a phenotype.

By sequencing homologues, studying their expression patterns and examining the effect of altering their expression, genes carrying out a similar function to *RAP2.2* are obtainable. Of course, mutants, variants and alleles of these sequences are included within the scope of the present invention in the same terms as discussed above for the *RAP2.2* genes discussed herein, although it should be noted that homologue sequences pre-existing on databases, such as any identified herein including in Table 2, may be excluded from one or more aspects or embodiments of the present invention while included in one or more other aspects.

Homology between the homologues as disclosed herein, may be exploited in the identification of further homologues, for example using oligonucleotides (e.g. a degenerate pool) designed on the basis of sequence conservation or PCR primers.

According to a further aspect, the present invention provides a method of identifying or a method of cloning an *RAP2.2* homologue, e.g. from a species other than Arabidopsis or tomato, the method employing a nucleotide sequence derived from that shown in Figure 4 or Figure 10 (section 2). For instance, such a method may include providing a preparation of plant cell nucleic acid, providing a nucleic acid molecule having a nucleotide sequence substantially as shown herein or complementary to a nucleotide sequence substantially as shown herein, preferably from within the coding sequence (e.g. a sequence coding for amino acid sequences shown in Figures 4, 10 or 11 (section 2)) contacting nucleic acid in said preparation with said nucleic acid molecule under conditions for hybridisation of said nucleic acid molecule to any said gene or homologue in said preparation, and identifying said gene or homologue if present by its hybridisation with said nucleic acid molecule.

Target or candidate nucleic acid may, for example, include genomic DNA, cDNA or RNA (or a mixture of any of these preferably as a library) obtainable from an organism known to contain or suspected of containing such nucleic acid, either monocotyledonous or dicotyledonous. Prior to any PCR that is to be performed, the complexity of a nucleic acid library may be reduced by creating a cDNA library for example using RT-PCR or by using the phenol emulsion reassociation technique (Clarke et al. (1992) NAR 20, 1289-1292) on a genomic library. Successful hybridisation may be identified and target/candidate nucleic acid isolated for further investigation and/or use.

Hybridisation of nucleic acid molecule to a *RAP2.2* gene or homologue may be determined or identified indirectly, e.g. using a nucleic acid amplification reaction, particularly the polymerase chain reaction (PCR). PCR requires the use of two primers to specifically amplify target nucleic acid, so preferably two nucleic acid molecules with sequences characteristic of Rap2.2 are employed. However, if RACE is used only one such primer may be needed. Hybridisation may be also be determined (optionally in conjunction with an amplification technique such as PCR) by probing with nucleic acid and identifying positive hybridisation under suitably stringent conditions (in accordance with known techniques). For probing, preferred conditions are those which are stringent enough for there to be a simple pattern with a small number of hybridisations identified as positive which can be investigated further. It is well known in the art to increase stringency of hybridisation gradually until only a few positive clones remain.

Binding of a probe to target nucleic acid (e.g. DNA) may be measured using any of a variety of techniques at the disposal of those skilled in the art. For instance, probes may be radioactively, fluorescently or enzymatically labelled. Other methods not employing labelling of probe include examination of restriction fragment length polymorphisms, amplification using PCR, RNAase cleavage and allele specific oligonucleotide probing.

Probing may employ the standard Southern blotting technique. For instance DNA may be extracted from cells and digested with different restriction enzymes. Restriction fragments may then be separated by electrophoresis on an agarose gel, before denaturation and transfer to a nitrocellulose filter. Labelled probe may be hybridised to the DNA fragments on the filter and binding determined. DNA for probing may be prepared from RNA preparations from cells by techniques such as reverse-transcriptase-PCR.

Preliminary experiments may be performed by hybridising under low stringency conditions with various probes to Southern blots of DNA digested with restriction enzymes. For probing, preferred conditions are those which are stringent enough for there to be a simple pattern with a small number of hybridisations identified as positive which can be investigated further. It is well known in the art to increase stringency of hybridisation gradually until only a few positive clones remain. Suitable conditions would be achieved when a large number of hybridising fragments were obtained while the background hybridisation was low. Using these conditions nucleic acid libraries, e.g. cDNA libraries representative of expressed sequences, may be searched. Those skilled in the art are well able to employ suitable conditions of the desired stringency for selective hybridisation, taking into account factors such as oligonucleotide length and base composition, temperature and so on.

For instance, screening may initially be carried out under conditions, which comprise a temperature of about 37°C or more, a formamide concentration of less than about 50%, and a moderate to low salt (e.g. Standard Saline Citrate (SSC) =, 0.15 M sodium chloride; 0.15 M sodium citrate; pH 7) concentration.

Alternatively, a temperature of about 50°C or more and a high salt (e.g.SSPE) 0.180 mM sodium chloride; 9 mM disodium hydrogen phosphate; 9 mM sodium dihydrogen phosphate; 1 mM sodium EDTA; pH 7.4). Preferably the screening is carried out at about 37°C, a formamide concentration of about 20%, and a salt concentration of about 5 X SSC, or a temperature of about 50°C and a salt concentration of about 2 X SSPE. These conditions will allow the identification of sequences which have a substantial degree of homology (similarity, identity) with the probe sequence, without requiring the perfect homology for the identification of a stable hybrid.

Suitable conditions include, e.g. for detection of sequences that are about 80-90% identical, hybridization overnight at 42°C in 0.25M Na₂HPO₄, pH 7.2, 6.5% SDS, 10% dextran sulfate and a final wash at 55°C in 0.1X SSC, 0.1% SDS. For detection of sequences that are greater than about 90% identical, suitable conditions include hybridization overnight at 65°C in 0.25M Na₂HPO₄, pH 7.2, 6.5% SDS, 10% dextran sulfate and a final wash at 60°C in 0.1X SSC, 0.1% SDS.

In general, hybridizations may be performed according to the method of Sambrook et al. (below) using a hybridization solution comprising: 5X SSC (wherein SSC = 0.15 M sodium chloride; 0.15 M sodium citrate; pH 7), 5X Denhardt's reagent, 0.5-1.0% SDS, 100 µg/ml denatured, fragmented salmon sperm DNA, 0.05% sodium pyrophosphate and up to 50% formamide. Hybridization is carried out at 37-42°C for at least six hours. Following hybridization, filters are washed as follows: (1) 5 minutes at room temperature in 2X SSC and 1% SDS; (2) 15 minutes at room temperature in 2X SSC and 0.1 % SDS; (3) 30 minutes - 1 hour at 37°C in 1X SSC and 1% SDS; (4) 2 hours at 42-65°C in 1X SSC and 1% SDS, changing the solution every 30 minutes.

One common formula for calculating the stringency conditions required to achieve hybridization between nucleic acid molecules of a specified sequence homology is (Sambrook et al. (1989)): Tₘ = 81.5°C + 16.6Log [Na+] + 0.41 (% G+C) - 0.63 (% formamide) - 600/#bp in duplex.

As an illustration of the above formula, using [Na+] = [0.368] and 50-% formamide, with GC content of 42% and an average probe size of 200 bases, the Tₘ is 57°C. The Tₘ of a DNA duplex decreases by 1 - 1.5°C with every 1% decrease in homology. Thus, targets with greater than about 75% sequence identity would be observed using a hybridization temperature of 42°C. Such a sequence would be considered substantially homologous to the nucleic acid sequence of the present invention.

It is well known in the art to increase stringency of hybridisation gradually until only a few positive clones remain. Other suitable conditions include, e.g. for detection of sequences that are about 80-90% identical, hybridization overnight at 42°C in 0.25M Na₂HPO₄, pH 7.2, 6.5% SDS, 10% dextran sulfate and a final wash at 55°C in 0.1X SSC, 0.1% SDS. For detection of sequences that are greater than about 90% identical, suitable conditions include hybridization overnight at 65°C in 0.25M Na₂HPO₄, pH 7.2, 6.5% SDS, 10% dextran sulfate and a final wash at 60°C in 0.1X SSC, 0.1% SDS.

An alternative, which may be particularly appropriate with plant nucleic acid preparations, is a solution of 5x SSPE (final 0.9 M NaCI, 0.05M sodium phosphate, 0.005M EDTA pH 7.7), 5X Denhardt's solution, 0.5% SDS, at 65C overnight, (for high stringency, highly similar sequences) or 50°C (for low stringency, less similar sequences). Washes in 0.2x SSC/0.1% SDS at 65°C for high stringency, alternatively at 50-60°C in 1x SSC/0.1% SDS for low stringency.

The present invention extends to nucleic acid selectively hybridisable under high stringency with nucleic acid identified herein, e.g. the coding sequence of Figure 4 or 10 (section 2).

PCR techniques for the amplification of nucleic acid are described in US Patent No. 4,683,195 and Saiki et al. *Science* **239**: 487-491 (1988). PCR includes steps of denaturation of template nucleic acid (if double-stranded), annealing of primer to target, and polymerisation. The nucleic acid probed or used as template in the amplification reaction may be genomic DNA, cDNA or RNA. PCR may be used to amplify specific sequences from genomic DNA, specific RNA sequences and cDNA transcribed from mRNA. References for the general use of PCR techniques include Mullis et al, Cold Spring Harbor Symp. Quant. Biol., 51:263, (1987), Ehrlich (ed), PCR technology, Stockton Press, NY, 1989, Ehrlich et al, Science, 252:1643-1650, (1991), "PCR protocols; A Guide to Methods and Applications", Eds. Innis et al, Academic Press, New York, (1990).

Assessment of whether or not a PCR product corresponds to a gene coding for a transcription factor able to influence or alter, generally enhance, the levels of carotenoid and/or tocotrienols/tocopherols in a plant tissue may be conducted in various ways, as discussed, and a PCR band may contain a complex mix of products. Individual products may be cloned and each screened for linkage to such known genes that are segregating in progeny that showed a polymorphism for this probe. Alternatively, the PCR product may be treated in a way that enables one to display the polymorphism on a denaturing polyacrylamide DNA sequencing gel with specific bands that are linked to the gene being preselected prior to cloning. Once a candidate PCR band has been cloned and shown to be linked to a known transcription factor of the invention, it may be used to isolate clones which may be inspected for other features and homologies to RAP2.2 or other related genes. It may subsequently be analysed by transformation to assess its function on introduction into a variety of the plant of interest.

Alternatively, the PCR band or sequences derived by analysing it may be used to assist plant breeders in monitoring the segregation of plants transgenic for the gene.

These techniques are of general applicability to the identification of genes encoding transcription factors of the invention.

Preferred amino acid sequences suitable for use in the design of probes or PCR primers are sequences conserved (completely, substantially or partly) between at least two *Rap2.2* peptides or polypeptides encoded by genes involved in the carotenoid and/or tocotrienol/tocopherol synthesis pathways in a plant. Conserved sequences may be identified using information contained herein, for instance in Figure 11 (section 2).

On the basis of amino acid sequence information or nucleotide sequence information, oligonucleotide probes or primers may be designed (when working from amino acid sequence information, taking into account the degeneracy of the genetic code and where appropriate, codon usage of the organism).

A gene or fragment thereof identified as being that to which a said nucleic acid molecule hybridises, which may be an amplified PCR product, may be isolated and/or purified and may be subsequently investigated for ability to alter, generally enhance the levels of carotenoid and/or tocotrienol/tocopherol levels in plants. If the identified nucleic acid is a fragment of a gene, the fragment may be used (e.g. by probing and/or PCR) in subsequent cloning of the full-length gene, which may be a full-length coding sequence. Inserts may be prepared from partial cDNA clones and used to screen cDNA libraries. The full-length clones isolated may be subcloned into expression vectors and activity assayed by introduction into suitable host cells and/or sequenced. It may be necessary for one or more gene fragments to be ligated to generate a full-length coding sequence.

The present application also provides oligonucleotides based on either a *RAP2.2* nucleotide sequence as provided herein or an *RAP2.2* nucleotide sequence obtainable in accordance with the disclosures and suggestions herein. The oligonucleotides may be of a length suitable for use as primers in an amplification reaction, or they may be suitable for use as hybridization fishing probes. Preferably an oligonucleotide in accordance with the invention, e.g. for use in nucleic acid amplification, has about 10 or fewer codons (e.g. 6, 7 or 8), i.e. is about 30 or fewer nucleotides in length (e.g. 18, 21 or 24). A probe or primer may be about 20-30 nucleotides in length.

Nucleic acid molecules and vectors according to the present invention may be provided in a form isolated and/or purified from their natural environment, in substantially pure or homogeneous, or free or substantially free of nucleic acid and or genes of the species of interest or origin other than the relevant sequence. Nucleic acid according to the present invention may include cDNA, RNA, genomic DNA and may be wholly or partially synthetic. The term "isolate" where used may encompass any of these possibilities.

Nucleic acid as herein provided or obtainable by use of the disclosures herein, may be the subject of alteration by way of one or more of addition, insertion, deletion or substitution of nucleotides with or without altering the encoded amino acid sequence (by virtue of the degeneracy of the genetic code). Such altered forms of *RAP2.2* nucleotide sequences as herein provided or obtainable by use of the disclosures herein can be easily and routinely tested for both *RAP2.2* function and *Rap2.2* function in accordance with standard techniques e.g. as provided herein which basically examine plants or plant cells carrying the mutant, derivative or variant for altered levels of total carotenoid and/or tocotrienol/tocopherol.

The nucleic acid molecule may be in the form of a recombinant and preferably replicable vector for example a plasmid, cosmid, phage or binary vector, e.g. suitable for use with Agrobacterium. The nucleic acid may be under the control of an appropriate promoter and regulatory elements for expression in a host cell such as a microbial, e.g. bacterial, or plant cell. In the case of genomic DNA, this may contain its own promoter and regulatory elements and in the case of cDNA this may be under the control of an appropriate promoter and regulatory elements for expression in the host cell. Thus, the nucleotide sequence of Figures 4 or 10 (section 2) (for example) may be placed under the control of an heterologous promoter. Similarly, a *RAP2.2* homologue sequence from another species may be operably linked to a promoter other than that with which it is naturally associated. However a vector including nucleic acid according to the present invention need not include a promoter, particularly if the vector is to be used to introduce the nucleic acid into cells for recombination into the genome.

The nucleic acid as provided by the present invention as well as a marker gene, respectively, may be placed under the control of any plant-compatible promoter. Those can be plant gene promoters, such as the promoter for the small subunit of ribulose-1,5-bis-phosphate carboxylase (RUBISCO), or promoters from tumour-inducing plasmids of *Agrobacterium tumefaciens*, like that nopaline synthase and octopine synthase promoters, or viral promoters such as the cauliflower mosaic virus (CaMV) 19S and 35S promoters or the figwort mosaic virus 35S promoter. See international application WO 91/19806, for example, for a review of known plant promoters which are suitable for use in the present invention.

"Tissue-specific" promoters provide that accumulation of one or more of said gene products is particularly high in the tissue in which products of the envisaged biosynthetic pathway shall be expressed; some expression may occur in other parts of the plant. Examples of known tissue-specific promoters include the glutelin 1 promoter (Kim et al., 1993; Okita et al., 1989; Zheng et al., 1993), the tuber-directed class I patatin promoter (Bevan et al., 1986); the promoters associated with potato tuber ADPGPP genes (Muller et al., 1990); the soybean promoter of β-conglycinin, also known as the 7S protein, which drives seed-directed transcription (Bray, 1987); and seed-directed promoters from the zein genes of maize endosperm (Pedersen et al., 1982). A further type of promoter which can be used according to the invention is a plant ubiquitin promoter. Plant ubiquitin promoters are well known in the art, as evidenced by Kay et al., (1987), and EP-A 0 342 926. Equally suitable for the present invention are actin promoters, histone promoters and tubulin promoters. Examples of preferred chemically inducible promoters, such as the tobacco PR-1a promoter, are detailed in EP-A 0 332 104. Another preferred category of promoters is that which is wound inducible. Preferred promoters of this kind include those described by Stanford et al., (1989), Xu et al., (1993), Logemann et al., (1989), Rohrmeier & Lehle, (1993), Firek et al., (1993), and Warner et al., (1993).

In a further aspect the present invention provides a gene construct including an inducible promoter operatively linked to a nucleotide sequence provided by the present invention. As discussed, this enables control of expression of the gene. The invention also provides plants transformed with said gene construct and methods including introduction of such a construct into a plant cell and/or induction of expression of a construct within a plant cell, e.g by application of a suitable stimulus, such as an effective exogenous inducer.

The term "inducible" as applied to a promoter is well understood by those skilled in the art. In essence, expression under the control of an inducible promoter is "switched on" or increased in response to an applied stimulus (which may be generated within a cell or provided exogenously). The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus. The preferable situation is where the level of expression increases upon application of the relevant stimulus by an amount effective to alter a phenotypic characteristic. Thus an inducible (or "switchable") promoter may be used which causes a basic level of expression in the absence of the stimulus which level is too low to bring about a desired phenotype (and may in fact be zero). Upon application of the stimulus, expression is increased (or switched on) to a level which brings about the desired phenotype. One example of an inducible promoter is the ethanol inducible gene switch disclosed in Caddick et al (1998) Nature Biotechnology 16: 177-180. Many other examples will be known to those skilled in the art.

Those skilled in the art are well able to construct vectors and design protocols for recombinant gene expression. Suitable vectors can be chosen or constructed, containing appropriate regulatory sequences, including promoter sequences, terminator fragments, polyadenylation sequences, enhancer sequences, marker genes and other sequences as appropriate. For further details see, for example, *Molecular Cloning: a Laboratory Manual:* 2nd edition, Sambrook *et al*, 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in *Current Protocols in Molecular Biology*, Second Edition, Ausubel et al. eds., John Wiley & Sons, 1992. The disclosures of Sambrook et al. and Ausubel et al. are incorporated herein by reference. Specific procedures and vectors previously used with wide success upon plants are described by Bevan (Nucl. Acids Res. 12, 8711-8721 (1984)) and Guerineau and Mullineaux (1993) (Plant transformation and expression vectors. In: Plant Molecular Biology Labfax (Croy RRD ed) Oxford, BIOS Scientific Publishers, pp 121-148).

In a preferred embodiment, the expression vector also contains a gene encoding a selection marker such as, e.g. hygromycin phosphotransferase (van den Elzen et al., 1985), which is functionally linked to a promoter. Additional examples of genes that confer antibiotic resistance and are thus suitable as selectable markers include those coding for neomycin phosphotransferase kanamycin resistance (Velten et al., 1984); the kanamycin resistance (NPT II) gene derived from Tn5 (Bevan et al., 1983); the PAT gene described in Thompson et al., (1987); and chloramphenicol acetyltransferase. For a general description of plant expression vectors and selectable marker genes suitable according to the present invention, see Gruber et al., (1993). As outlined above, selectable genetic markers may be used consisting of chimaeric genes that confer selectable phenotypes such as resistance to antibiotics such as kanamycin, hygromycin, phosphinotricin, chlorsulfuron, methotrexate, gentamycin, spectinomycin, imidazolinones and glyphosate. Alternative selection concepts avoiding introduction of antibiotic resistance genes may alternatively be used, such as the PMI gene (cf. e.g. US Patents 5,994,629 and 5,767,378).

As can be easily achieved by a person skilled in the art the said selection marker can be removed by using co-transformation techniques available in the art.

When introducing a chosen gene construct into a cell, certain considerations must be taken into account, well known to those skilled in the art. The nucleic acid to be inserted should be assembled within a construct which contains effective regulatory elements which will drive transcription. There must be available a method of transporting the construct into the cell. Once the construct is within the cell membrane, integration into the endogenous chromosomal material either will or will not occur. Finally, as far as plants are concerned the target cell type must be such that cells can be regenerated into whole plants.

Plants transformed with the DNA segment containing the sequence may be produced by standard techniques which are already known for the genetic manipulation of plants. DNA can be transformed into plant cells using any suitable technology, such as a disarmed Ti-plasmid vector carried by Agrobacterium exploiting its natural gene transfer ability (EP-A-270355, EP-A-0116718, NAR 12(22) 8711 -87215 1984), particle or microprojectile bombardment (US 5100792, EP-A-444882, EP-A-434616) microinjection (WO 92/09696, WO 94/00583, EP 331083, EP 175966, Green et al. (1987) *Plant Tissue and Cell Culture,* Academic Press), electroporation (EP 290395, WO 8706614) other forms of direct DNA uptake (DE 4005152, WO 9012096, US 4684611), liposome mediated DNA uptake (e.g. Freeman et al. *Plant Cell Physiol*. **29**: 1353 (1984)), or the vortexing method (e.g. Kindle, *PNAS U.S.A.* **87:** 1228 (1990d) Physical methods for the transformation of plant cells are reviewed in Oard, 1991, *Biotech. Adv.* **9**: 1-11.

Thus once a gene has been identified, it may be reintroduced into plant cells using techniques well known to those skilled in the art to produce transgenic plants of the appropriate phenotype.

Agrobacterium transformation is widely used by those skilled in the art to transform dicotyledonous species. Production of stable, fertile transgenic plants in almost all economically relevant monocot plants is also now routine:(Toriyama, et al. (1988) *Bio*/*Technology* **6,** 1072-1074; Zhang, et al. (1988) *Plant Cell Rep.* **7**, 379-384; Zhang, et al. (1988) *Theor Appl Genet* **76,** 835-840; Shimamoto, et al. (1989) *Nature* **338,** 274-276; Datta, et al. (1990) *Bio*/*Technology* **8,** 736-740; Christou, et al. (1991) *Bio*/*Technology* **9**, 957-962; Peng, et al. (1991) International Rice Research Institute, Manila, Philippines 563-574; Cao, et al. (1992) *Plant Cell Rep*. **11,** 585-591; Li, et al. (1993) *Plant Cell Rep.* **12**, 250-255; Rathore, et al. (1993) *Plant Molecular Biology* **21,** 871-884; Fromm, et al. (1990) *Bio*/*Technology* **8,** 833-839; Gordon-Kamm, et al. (1990) *Plant Cell* **2,** 603-618; D'Halluin, et al. (1992) *Plant Cell* **4,** 1495-1505; Walters, et al. (1992) *Plant Molecular Biology* **18,** 189-200; Koziel, et al. (1993) *Biotechnology* **11,** 194-200; Vasil, I. K. (1994) *Plant Molecular Biology* **25,** 925-937; Weeks, et al. (1993) *Plant Physiology* **102,** 1077-1084; Somers, et al. (1992) *Bio*/*Technology* **10,** 1589-1594; WO92/14828). In particular, *Agrobacterium* mediated transformation is now a highly efficient alternative transformation method in monocots (Hiei et al. (1994) *The Plant Journal* **6**, 271-282).

The generation of fertile transgenic plants has been achieved in the cereals rice, maize, wheat, oat, and barley (reviewed in Shimamoto, K. (1994) *Current Opinion in Biotechnology* **5**, 158-162.; Vasil, et al. (1992) *Bio*/*Technology* 10, 667-674; Vain et al., 1995, *Biotechnology Advances* **13** (4): 653-671; Vasil, 1996, *Nature Biotechnology* **14** page 702). Wan and Lemaux (1994) *Plant Physiol.* **104:** 37-48 describe techniques for generation of large numbers of independently transformed fertile barley plants.

Microprojectile bombardment, electroporation and direct DNA uptake are preferred where Agrobacterium is inefficient or ineffective. Alternatively, a combination of different techniques may be employed to enhance the efficiency of the transformation process, e.g. bombardment with Agrobacterium coated microparticles (EP-A-486234) or microprojectile bombardment to induce wounding followed by co-cultivation with Agrobacterium (EP-A-486233).

Following transformation, a plant may be regenerated, e.g. from single cells, callus tissue or leaf discs, as is standard in the art. Almost any plant can be entirely regenerated from cells, tissues and organs of the plant. Available techniques are reviewed in Vasil et al., *Cell Culture and Somatic Cell Genetics of Plants, Vol I, II and III, Laboratory Procedures and Their Applications,* Academic Press, 1984, and Weissbach and Weissbach, *Methods for Plant Molecular Biology,* Academic Press, 1989.

The particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

The invention further encompasses a host cell transformed with a vector as set forth above, especially a plant or a microbial cell. Thus, a host cell, such as a plant cell, including a nucleotide sequence as herein indicated is provided. Within the cell, the nucleotide sequence may be incorporated within the chromosome.

Also according to the invention there is provided a plant cell having incorporated into its genome a nucleotide sequence, particularly a heterologous nucleotide sequence, as provided by the present invention under operative control of a regulatory sequence for control of expression. The coding sequence may be operably linked to one or more regulatory sequences which may be heterologous or foreign to the gene, such as not naturally associated with the gene for its expression. The nucleotide sequence according to the invention may be placed under the control of an externally inducible gene promoter to place expression under the control of the user. A further aspect of the present invention provides a method of making such a plant cell involving introduction of nucleotide sequence or a suitable vector including the sequence of nucleotides into a plant cell and causing or allowing recombination between the vector and the plant cell genome to introduce the sequence of nucleotides into the genome. The invention extends to plant cells containing a nucleotide sequence according to the invention as a result of introduction of the nucleotide sequence into an ancestor cell.

The term "heterologous" may be used to indicate that the gene/sequence of nucleotides in question have been introduced into said cells of the plant or an ancestor thereof, using genetic engineering, ie by human intervention. A transgenic plant cell, i.e. transgenic for the nucleotide sequence in question, may be provided. The transgene may be on an extra-genomic vector or incorporated, preferably stably, into the genome. A heterologous gene may replace an endogenous equivalent gene, ie one which normally performs the same or a similar function, or the inserted sequence may be additional to the endogenous gene or other sequence. An advantage of introduction of a heterologous gene is the ability to place expression of a sequence under the control of a promoter of choice, in order to be able to influence expression according to preference. Furthermore, mutants, variants and derivatives of the wild-type gene, e.g. with higher activity than wild-type, may be used in place of the endogenous gene. Nucleotide sequences heterologous, or exogenous or foreign, to a plant cell may be non-naturally occurring in cells of that type, variety or species. Thus, a nucleotide sequence may include a coding sequence of or derived from a particular type of plant cell or species or variety of plant, placed within the context of a plant cell of a different type or species or variety of plant. A further possibility is for a nucleotide sequence to be placed within a cell in which it or a homologue is found naturally, but wherein the nucleotide sequence is linked and/or adjacent to nucleic acid which does not occur naturally within the cell, or cells of that type or species or variety of plant, such as operably linked to one or more regulatory sequences, such as a promoter sequence, for control of expression. A sequence within a plant or other host cell may be identifiably heterologous, exogenous or foreign.

Plants which include a plant cell according to the invention are also provided, along with any part or propagule thereof, seed, selfed or hybrid progeny and descendants. Particularly provided are transgenic crop plants, which have been engineered to carry genes identified as stated above.

Examples of suitable plants which can be selected from the group as indicated above include tobacco, carrot, vegetable brassica's as provided for above, melons, capsicums, grape vines, lettuce, strawberry, oilseed brassica, sugar beet, wheat, barley, maize, rice, soyabeans, peas, sorghum, sunflower, tomato, cassava, banana, sweet potato and potato.

A plant according to the present invention may be one which does not breed true in one or more properties. Plant varieties may be excluded, particularly registrable plant varieties according to Plant Breeders Rights. It is noted that a plant need not be considered a "plant variety" simply because it contains stably within its genome a transgene, introduced into a cell of the plant or an ancestor thereof.

In addition to a plant, the present invention provides any clone of such a plant, seed, selfed or hybrid progeny and descendants, and any part of any of these, such as cuttings, seed. The invention provides any plant propagule, that is any part which may be used in reproduction or propagation, sexual or asexual, including cuttings, seed and so on. Also encompassed by the invention is a plant which is a sexually or asexually propagated off-spring, clone or descendant of such a plant, or any part or propagule of said plant, off-spring, clone or descendant.

The present invention also encompasses the polypeptide expression product of a nucleic acid molecule according to the invention as disclosed herein or obtainable in accordance with the information and suggestions herein. Also provided are methods of making such an expression product by expression from a nucleotide sequence encoding therefore under suitable conditions in suitable host cells e.g. *E.coli*. Those skilled in the art are well able to construct vectors and design protocols and systems for expression and recovery of products of recombinant gene expression.

Preferred polypeptides include the tomato and Arabidopsis amino acid sequences shown in Figure 11 (section 2). A polypeptide according to the present invention may be an allele, variant, fragment, derivative, mutant or homologue of a polypeptide as shown in Figure 11 (section 2). The allele, variant, fragment, derivative, mutant or homologue may have substantially the Rap2.2 function of the amino acid sequences alluded to above and shown in Figure 11 (section 2) or may be a rap2.2 functional mutant thereof.

"Homology" in relation to an amino acid sequence of the invention may be used to refer to identity or similarity, preferably identity. As noted already above, high level of amino acid identity may be limited to functionally significant domains or regions, e.g. any of the domains identified herein.

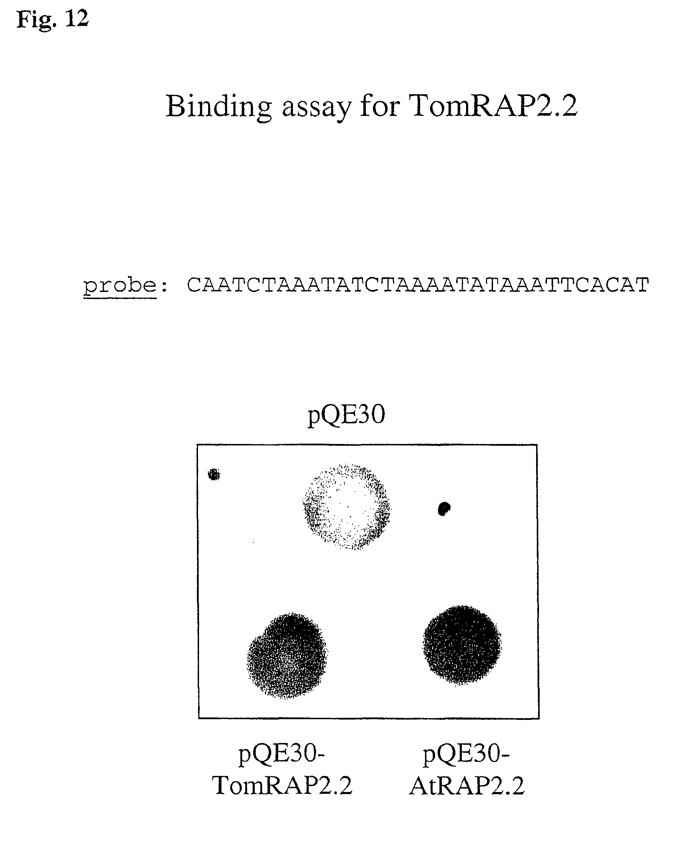

In particular homologues of the particular Rap2.2 polypeptide sequences provided herein are provided by the present invention, as are mutants, variants, fragments and derivatives of such homologues. Such homologues are readily obtainable by use of the disclosures made herein. Thus the present invention also extends to polypetides which include an amino acid sequence with Rap2.2 function obtainable using sequence information as provided herein. The Rap2.2 homologue may at the amino acid level have homology, that is identity, with the amino acid sequences shown in Figure 12, preferably at least about 50%, or at least 51%, 52%, 53%, 54% or 55%, or at least about 60%, or at least about 65%, or at least about 70%, or at least about 75%, or at least about 80% homology, or at least about 85 %, or at least about 88% homology, or at least about 90% homology. Most preferably at least about 95% or greater homology.

In certain embodiments, an allele, variant, derivative, mutant derivative, mutant or homologue of the specific sequence may show little overall homology, say about 20%, or about 25%, or about 30%, or about 35%, or about 40% or about 45%, with the specific sequence. However, in functionally significant domains or regions, the amino acid homology may be much higher. Putative functionally significant domains or regions can be identified using processes of bioinformatics, including comparison of the sequences of homologues. Functionally significant domains or regions of different polypeptides may be combined for expression from encoding nucleic acid as a fusion protein. For example, particularly advantageous or desirable properties of different homologues may be combined in a hybrid protein, such that the resultant expression product, with Rap2.2 function, may include fragments of various parent proteins.

Similarity of amino acid sequences may be as defined and determined by the TBLASTN program, of Altschul *et al*. (1990) *J. Mol. Biol*. **215:** 403-10, which is in standard use in the art. In particular, TBLASTN 2.0 may be used with Matrix BLOSUM62 and GAP penalties: existence: 11, extension: 1. Another standard program that may be used is BestFit, which is part of the Wisconsin Package, Version 8, September 1994, (Genetics Computer Group, 575 Science Drive, Madison, Wisconsin, USA, Wisconsin 53711). BestFit makes an optimal alignment of the best segment of similarity between two sequences. Optimal alignments are found by inserting gaps to maximize the number of matches using the local homology algorithm of Smith and Waterman (*Adv. Appl. Math.* (1981) 2: 482-489). Other algorithms include GAP, which uses the Needleman and Wunsch algorithm to align two complete sequences that maximizes the number of matches and minimizes the number of gaps. As with any algorithm, generally the default parameters are used, which for GAP are a gap creation penalty = 12 and gap extension penalty = 4. Alternatively, a gap creation penalty of 3 and gap extension penalty of 0.1 may be used. The algorithm FASTA (which uses the method of Pearson and Lipman (1988) *PNAS USA* **85**: 2444-2448) is a further alternative.

Use of either of the terms "homology" and "homologous" herein does not imply any necessary evolutionary relationship between compared sequences, in keeping for example with standard use of terms such as "homologous recombination" which merely requires that two nucleotide sequences are sufficiently similar to recombine under the appropriate conditions. Further discussion of polypeptides according to the present invention, which may be encoded by nucleic acid according to the present invention, is found below.

Purified Rap2.2 polypeptides and mutants, variants, fragments, derivatives, alleles and homologues thereof e.g. produced recombinantly by expression from encoding nucleic acid therefor, may be used to raise antibodies employing techniques which are standard in the art. Antibodies and polypeptides including antigen-binding fragments of antibodies may be used in identifying homologues of the sequences specifically provided herein as discussed further below.

Methods of producing antibodies include immunising a mammal (e.g. human, mouse, rat, rabbit, horse, goat, sheep or monkey) with the protein or a fragment thereof. Antibodies may be obtained from immunised animals using any of a variety of techniques known in the art, and might be screened, preferably using binding of antibody to antigen of interest. For instance, Western blotting techniques or immunoprecipitation may be used (Armitage et al, 1992, Nature **357**: 80-82). Antibodies may be polyclonal or monoclonal.

As an alternative or supplement to immunising a mammal, antibodies with appropriate binding specificity may be obtained from a recombinantly produced library of expressed immunoglobulin variable domains, e.g. using lambda bacteriophage or filamentous bacteriophage which display functional immunoglobulin binding domains on their surfaces; for instance see WO92/01047.

Antibodies raised to a polypeptide or peptide can be used in the identification and/or isolation of homologous polypeptides, and then the encoding genes. Thus, the present invention provides a method of identifying or isolating a polypeptide with Rap2.2 function (in accordance with embodiments disclosed herein), including screening candidate peptides or polypeptides with a polypeptide including the antigen-binding domain of an antibody (for example whole antibody or a fragment thereof) which is able to bind a Rarl peptide, polypeptide or fragment, variant or variant thereof or preferably has binding specificity for such a peptide or polypeptide, such as having an amino acid sequence identified herein. Specific binding members such as antibodies and polypeptides including antigen binding domains of antibodies that bind and are preferably specific for a Rarl peptide or polypeptide or mutant, variant or derivative thereof represent further aspects of the present invention, as do their use and methods which employ them.

Candidate peptides or polypeptides for screening may for instance be the products of an expression library created using nucleic acid derived from an plant of interest, or may be the product of a purification process from a natural source.

A peptide or polypeptide found to bind the antibody may be isolated and then may be subject to amino acid sequencing. Any suitable technique may be used to sequence the peptide or polypeptide either wholly or partially (for instance a fragment of a polypeptide may be sequenced). Amino acid sequence information may be used in obtaining nucleic acid encoding the peptide or polypeptide, for instance by designing one or more oligonucleotides (e.g. a degenerate pool of oligonucleotides) for use as probes or primers in hybridisation to candidate nucleic acid, or by searching computer sequence databases, as discussed further below.

The invention further provides a method of enhancing carotenoid and/or tocotrienol/tocopherol levels in a plant which includes expressing a heterologous nucleic acid sequence with Rap2.2 function as discussed, within cells of the plant.

Such methods may be achieved by expression from a nucleotide sequence encoding an amino acid sequence conferring an Rap2.2 function within cells of a plant (thereby producing the encoded polypeptide), following an earlier step of introduction of the nucleotide sequence into a cell of the plant or an ancestor thereof. Such a method may enhance the carotenoid and/or tocotrienol/tocopherol levels in a plant.

Manipulation of expression of a *Rap2.2* transcript or Rap2.2 protein may be used to enhance the carotenoid and/or tocotrienol/tocopherol levels in different plants. This may be achieved by using e.g. a highly active constitutive plant promoter such as the CaMV-35S promoter or the *nos* promoter, or by a highly active tissue-specific plant promoter such as e.g. GTI promoter, which is appropriately selected with respect to the envisaged target tissue, and which can be, for example, endosperm-specific (GTI). Alternatively, *Rap2.2* may be attached to a pathogen-inducible promoter (see discussion below), allowing greater expression in challenged cells.

A gene stably incorporated into the genome of a plant is passed from generation to generation to descendants of the plant, cells of which descendants may express the encoded polypeptide and so may have enhanced levels of carotenoid and/or tocotrienol/tocopherol levels therein.

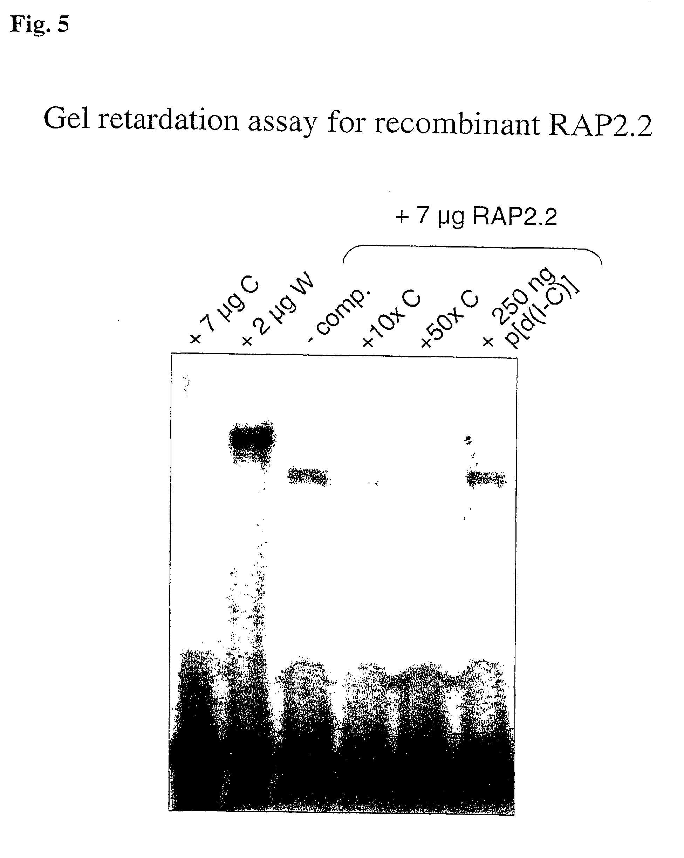

The invention further provides a method which includes expression from a nucleic acid encoding the amino acid sequence of Figure 5 or Figure 11 or a mutant, allele or derivative of the sequence (which may have Rap2.2 function) within cells of a plant (thereby producing the encoded polypeptide), following an earlier step of introduction of the nucleic acid into a cell of the plant or an ancestor thereof. Such a method may raise the plant's total levels of carotenoids and/or tocotrienols/tocopherols.

In a further aspect of the invention there is provided plants transgenic for a nucleic acid sequence comprising the RAP2.2 sequence of Figure 4 or Figure 10 (section 2) having elevated levels of total carotenoids and/or tocotrienols/tocopherols as compared with wild type plants of the same plant species wherein the total carotenoid level is raised from about 5% to about 40% or more, preferably from about 5% to about 35%, most preferably from about 5% to about 30% and/or the total tocotrienol/tocopherol level is raised from about 5% to about 160% or more, preferably from about 5% to about 140%, more preferably from about 30% to about 120%, and most preferably from about 50% to about 100%.

Embodiments and examples relating to the present invention are now described by way of example only with reference to the following figures.

### Brief description of the drawings

### Section 1: Structural and Functional Characterisation of the Phytoene Synthase Promoter

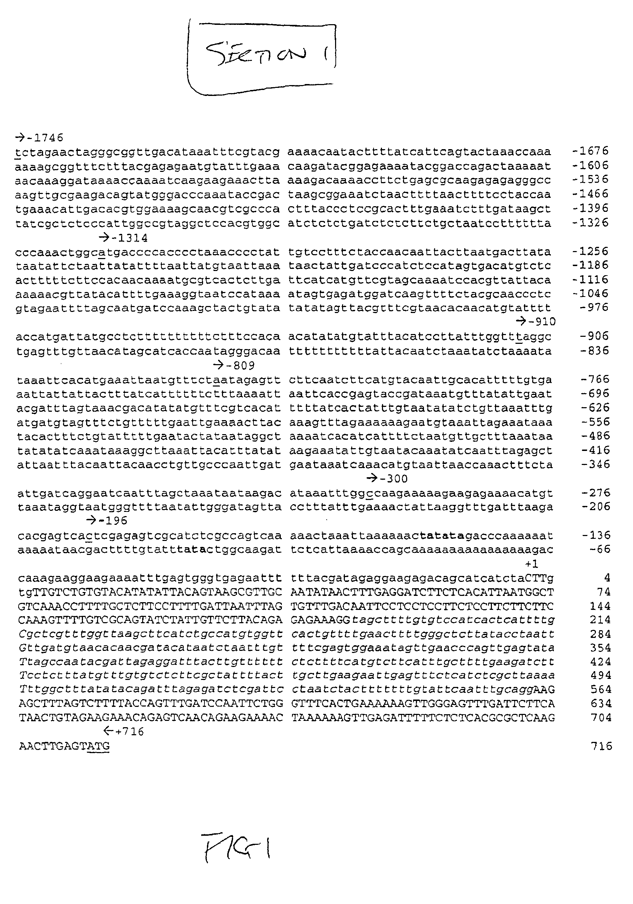

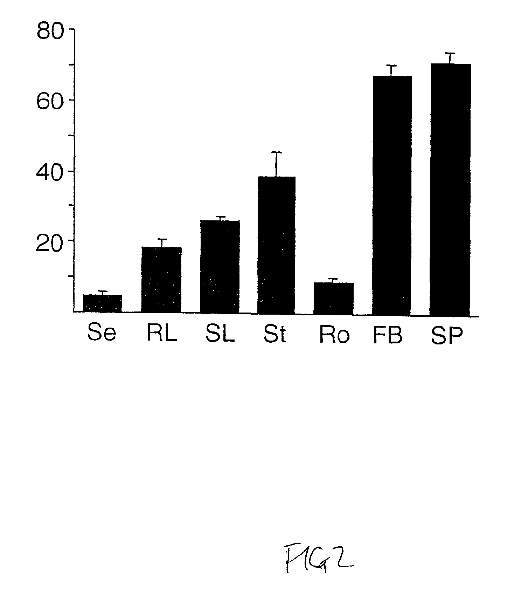

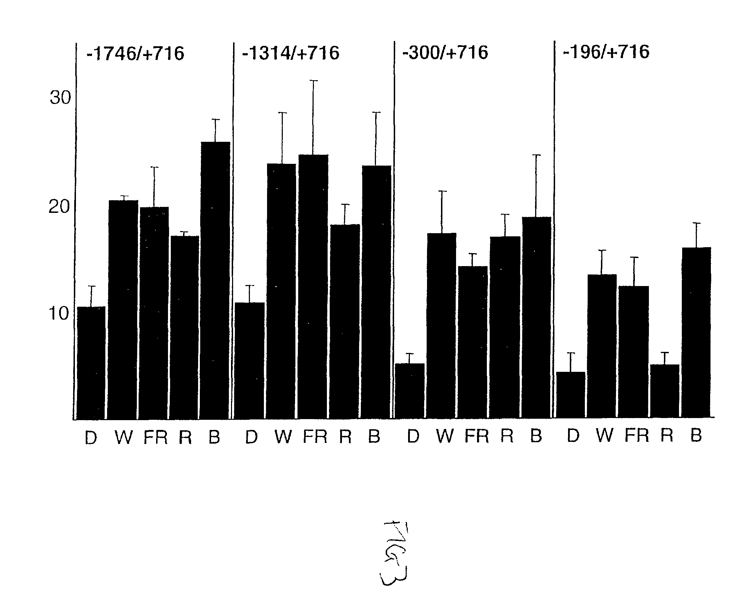

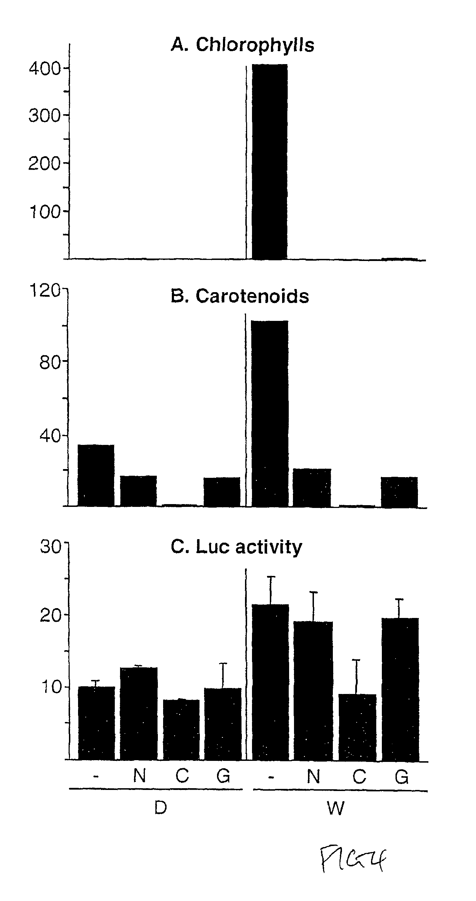

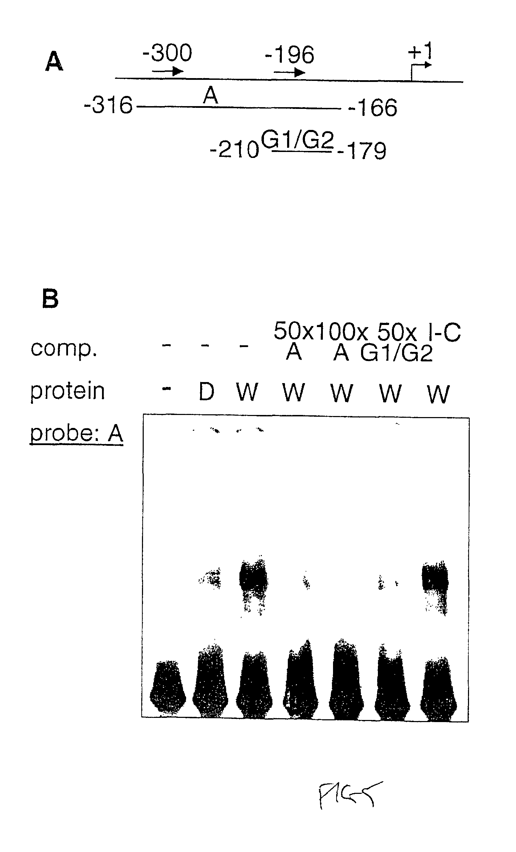

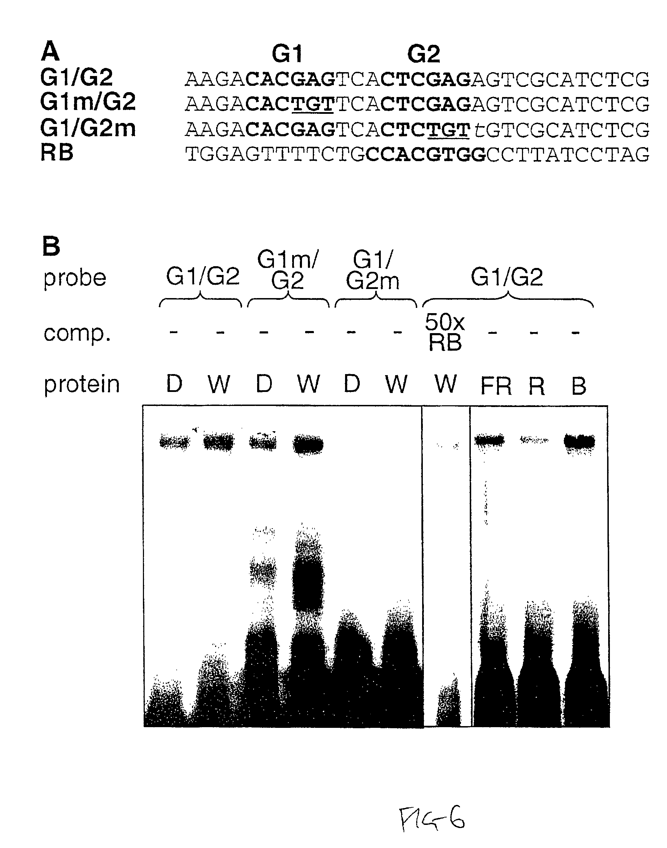

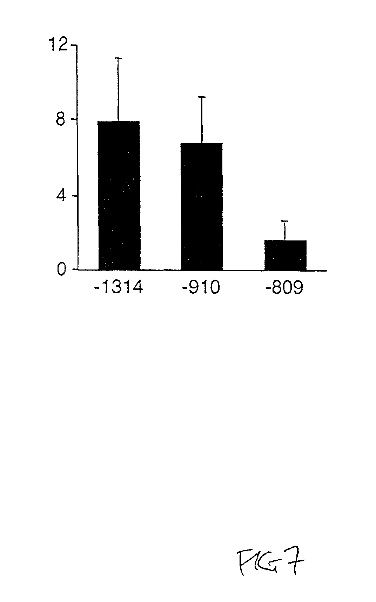

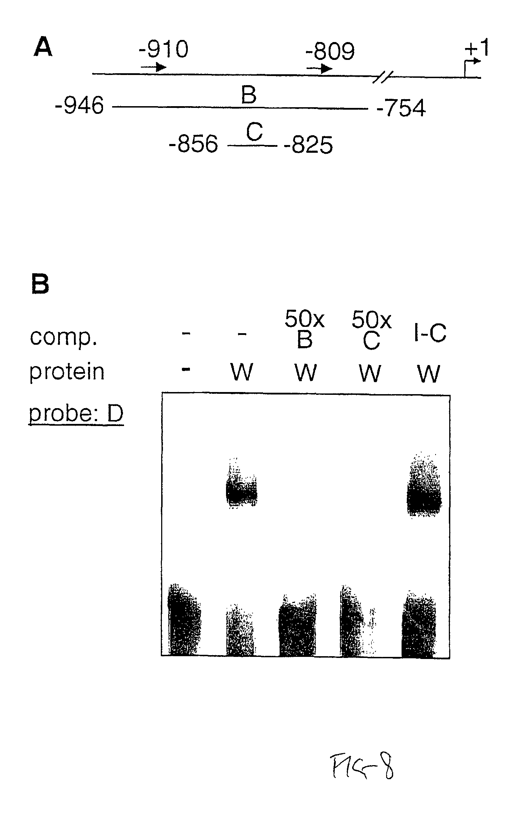

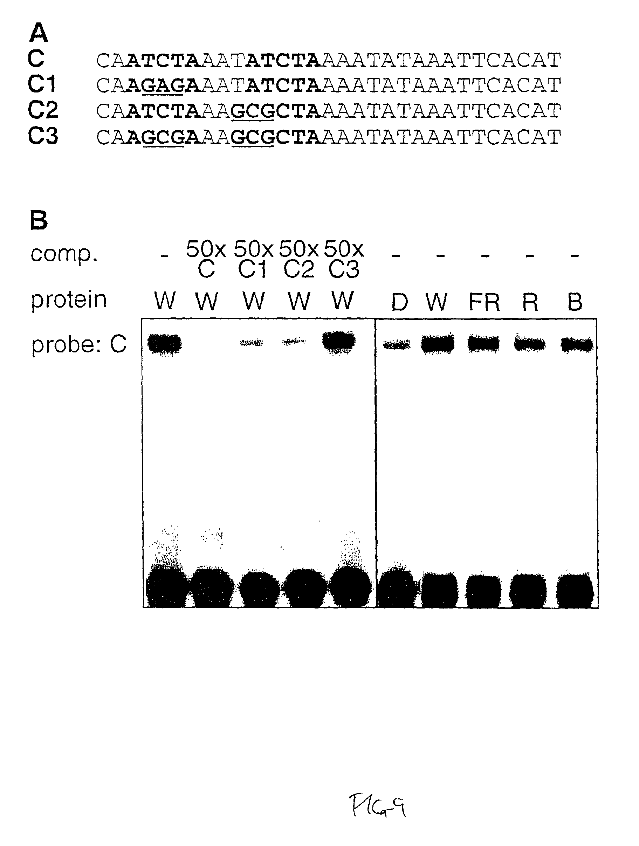

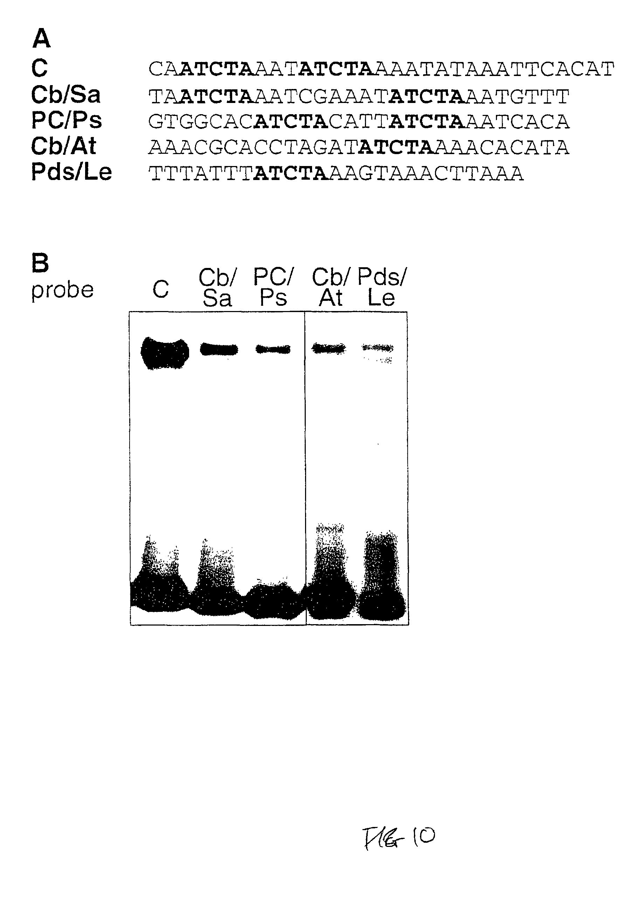

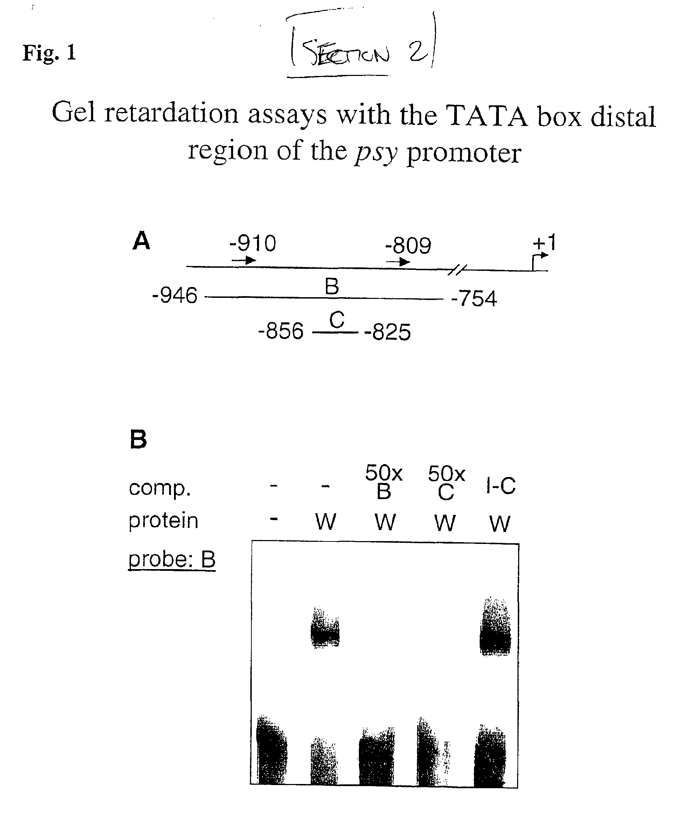

**Figure 1.** Sequence of the *psy* promoter and 5' non-translated region.
   Position +1 represents the first nucleotide of the longest cDNA isolated (Bartley and Scolnik, 1994). Exons are shown in uppercase letters and the first intron is italicized. Breakpoints of the fusions with the *luc* reporter gene are indicated by arrows and underlined letters. Putative TATA boxes are shown in bold, the ATG start codon is underlined. The sequence represents sequence from 21076 to 25521 of the P1 clone MKP11 of chromosome 5 (GenBank accession number AB005238).
**Figure 2.** Activity of the -1746/+716-*psy*/*luc* fusion in tissue extracts from 6 week old transgenic Arabidopsis plants.
   Luc activity was determined by a luminometric assay and expressed in RLU s⁻¹ µg⁻¹ protein. Se, seeds; RL, rosette leaves; SL, stem leaves, St, stem, Ro, roots; FB, flowering buds; SP, seed pods.
**Figure 3.** Activity of different *psy*/*luc* fusions in transgenic Arabidopsis seedlings during de-etiolation.
   The numbers indicate the length of the *psy* promoter as translational fusion with the *luc* gene. Transgenic Arabidopsis seedlings were etiolated for 3 days (D) and thereafter illuminated for 24 h with white light (W), far-red light (FR), red light (R) and blue light (B). Luc activity was measured by a luminometric assay and expressed in RLU s⁻¹ µg⁻¹ protein.
**Figure 4.** Effects of herbicide treatments on carotenoid and chlorophyll content and *psy* promoter activity in Arabidopsis seedlings.
   Wt and transgenic -1746/+716 *psy*/*luc* Arabidopsis seedlings were etiolated for 3 days (D) and thereafter illuminated for 24 h with white light (W). Chlorophyll and carotenoid content (in ng µg⁻¹ protein) are shown in the upper two panels while luc activity (RLU s⁻¹ µg⁻¹ protein) is shown in the bottom panel. N, norflurazon; C, CPTA; G, gabaculine.
**Figure 5. A.** Fragments from the TATA box proximal region of the *psy* promoter used in electrophoretic mobility shift assays (EMSA). The numbers depict the distance in bp to the transcription start site (+1). **B.** EMSA with labeled fragment A and mustard nuclear extracts from etiolated (D) and white light-illuminated mustard seedlings (W). Competitions were performed with the molar excess of unlabelled fragments indicated. I-C, 250 ng poly(dIdC) was added to the incubation.

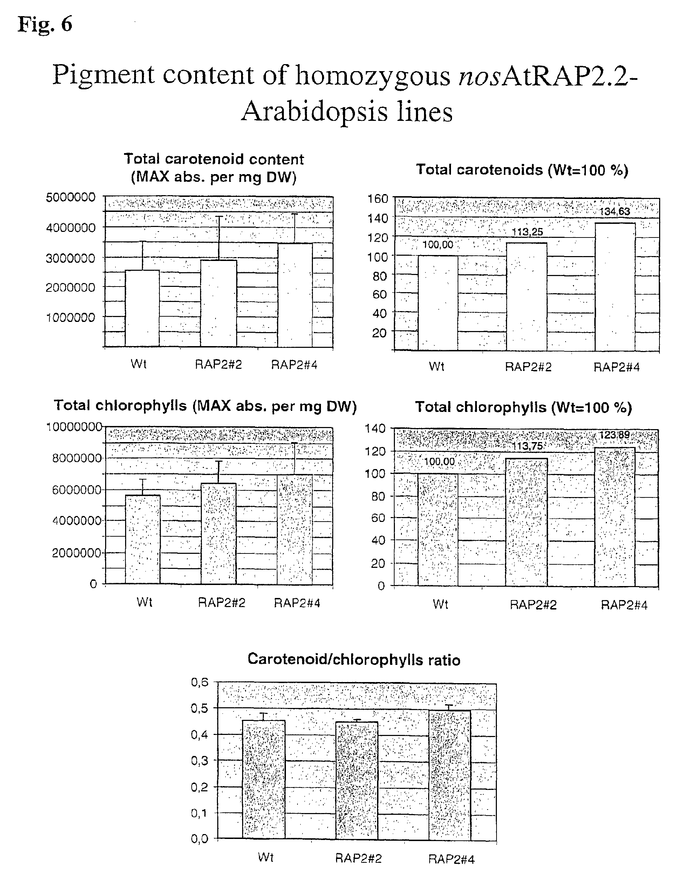

**Figure 6. A.** Sequences of the oligonucleotides used for the electrophoretic mobility shift assays (EMSA). G-box-like elements and the G-box of the *rbcS3B* promoter are shown in bold face; mutations are printed in italics. G1/G2, *psy* promoter region between -210 and -179; RB, *rbcS3B* promoter region between 8212 and 8242 (Dedonder *et al*., 1993). **B.** EMSA with labeled oligonucleotides indicated in A and mustard nuclear extracts from etiolated (D) and mustard seedlings illuminated with white (W), far-red (FR), red (R) and blue light (B), respectively. RB was used as competitor with the molar excesses indicated.

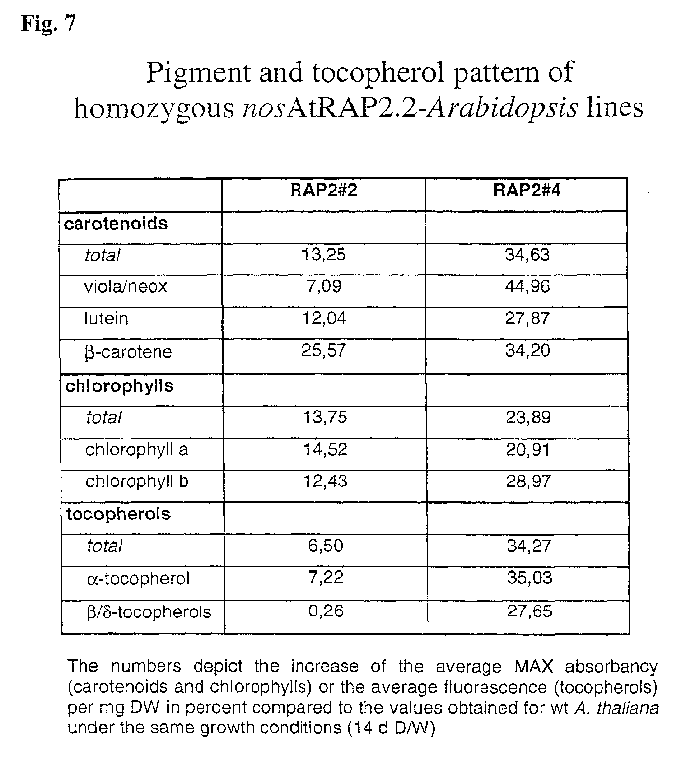

**Figure 7.** Quantification of luciferase activity using transient expression.
   The test plasmids consisted of the *psy* promoter/luciferase fusions indicated. As a control plasmid the vector pHGi35SGUS was used, which leads to constitutive expression of the *GUS* gene. Equimolar amounts of both plasmid were used for transient expression in leaves of *A. thaliana*. Luciferase activity was measured luminometrically and divided through the flourimetrically determined GUS activity.

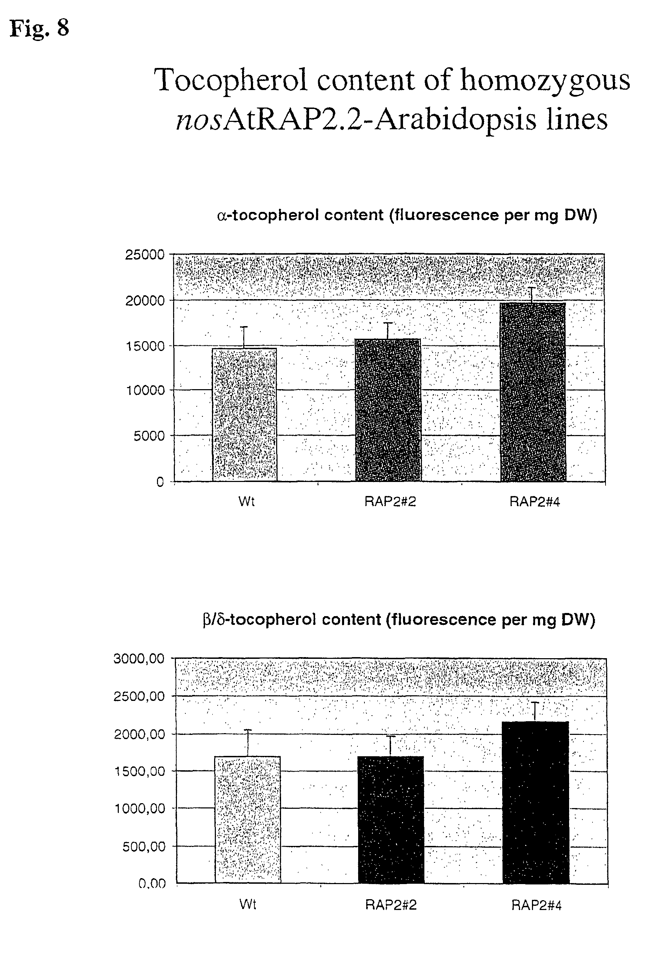

**Figure 8. A.** Fragments from the TATA box distal region of the *psy* promoter used in gel mobility shift assays. The numbers depict the distance in bp to the transcription start site (+1). **B.** Gel mobility shift assays with labeled fragment D and mustard nuclear extracts from illuminated (W) mustard seedlings. Competitions were performed with 50x molar excess of unlabelled fragments B and C, respectively. I-C, 250 ng poly(dIdC) was added to the incubation.

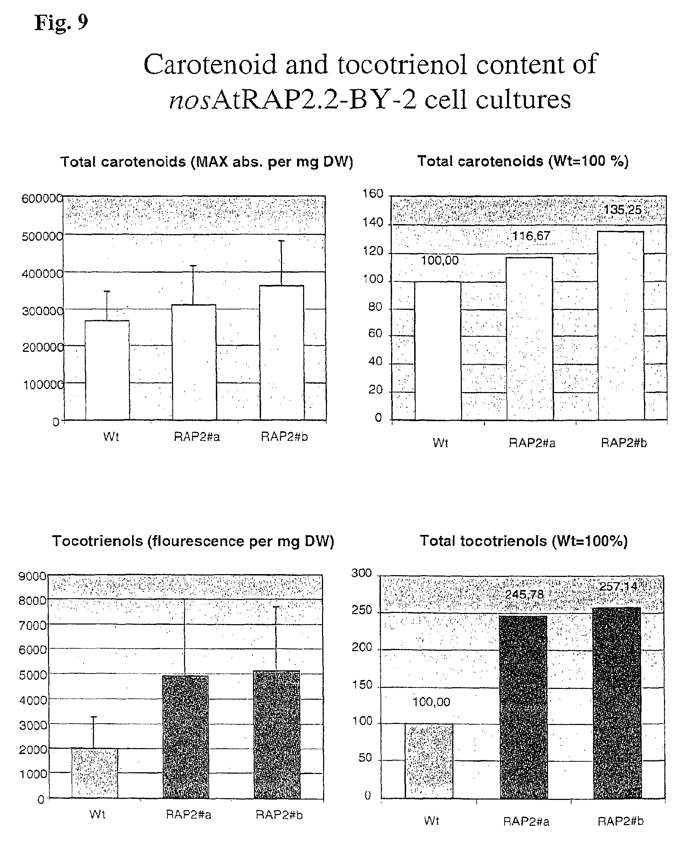

**Figure 9. A.** Sequences of the oligonucleotides used for the gel mobility shift assays. ATCTA elements shown in bold face; mutations are printed in italics. C, *psy* promoter region between -856 and -836. **B.** Gel mobility shift assay with labeled oligonucleotides indicated in A and mustard nuclear extracts from etiolated seedlings (D) or etiolated seedlings illuminated with white (W), far-red (FR), red (R) and blue (B) light, respectively. Competitions were performed with 50x molar excess of unlabelled fragments indicated.
**Figure 10. A.** Sequences of the oligonucleotides used for the gel mobility shift assays. ATCTA elements shown in bold face. C, *psy* promoter region between -856 and -836; Cb/Sa, *cab* promoter/*S. alba* from bp 397 to 423 (GenBank accession number X16436); PC/Ps, plastocyanin promoter/*P. sativum* from bp 637 to 664 (GenBank accession number X16082); Cb/At, *cabl* promoter/*A. thaliana* from bp 1130 to 1157 (GenBank accession number J04098); Pds/Le, *pds* promoter/*L*. *esculentum* from bp 657 to 671 (GenBank accession number U46919). **B.** Gel mobility shift assay with labeled oligonucleotides indicated in A. and nuclear extracts from mustard seedlings illuminated with white light.

### Section 2: Transcription factors RAP2.2

**Figure 1.** Gel retardation assays with the TATA box distal region of the *psy* promoter.
   **A.** Fragments from the TATA box distal region of the *psy* promoter used in gel retardation assays. The numbers depict the distance in bp to the transcription start site (+1). **B.** Gel retardation assays with labeled fragment B and mustard nuclear extracts from illuminated (W) mustard seedlings. Competitions were performed with 50x molar excess of unlabelled fragments B and C, respectively. I-C, 250 ng poly(dIdC) was added to the incubation.

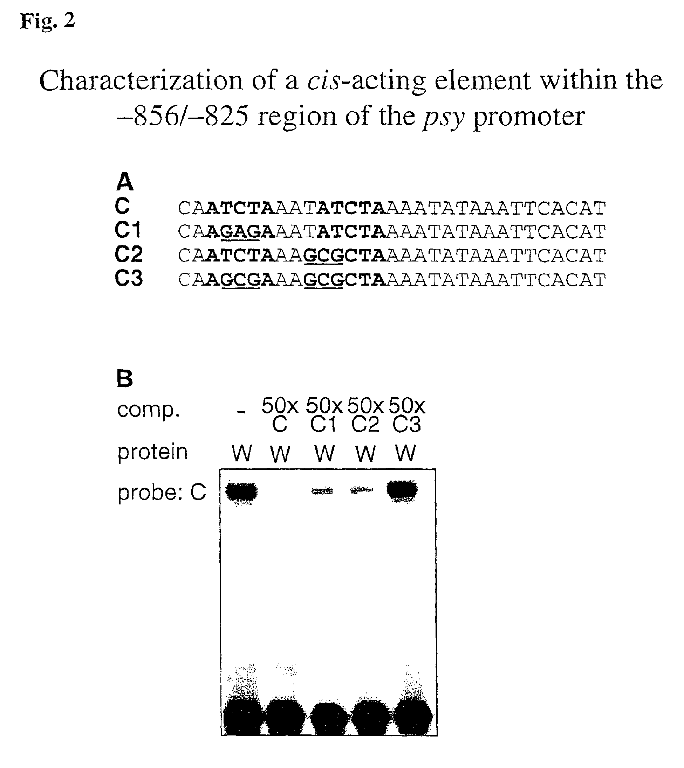

**Figure 2.** Characterization of a *cis*-acting element within the -856/-825 region of the *psy* promoter.
   **A.** Sequences of the oligonucleotides used for the gel retardation assays. ATCTA elements shown in bold face; mutations are printed in italics. C, *psy* promoter region between -856 and -825. **B.** Gel retardation assay with labeled oligonucleotides indicated in A and mustard nuclear extracts from etiolated seedlings illuminated with white (W) light. Competitions were performed with 50x molar excess of unlabelled fragments indicated.

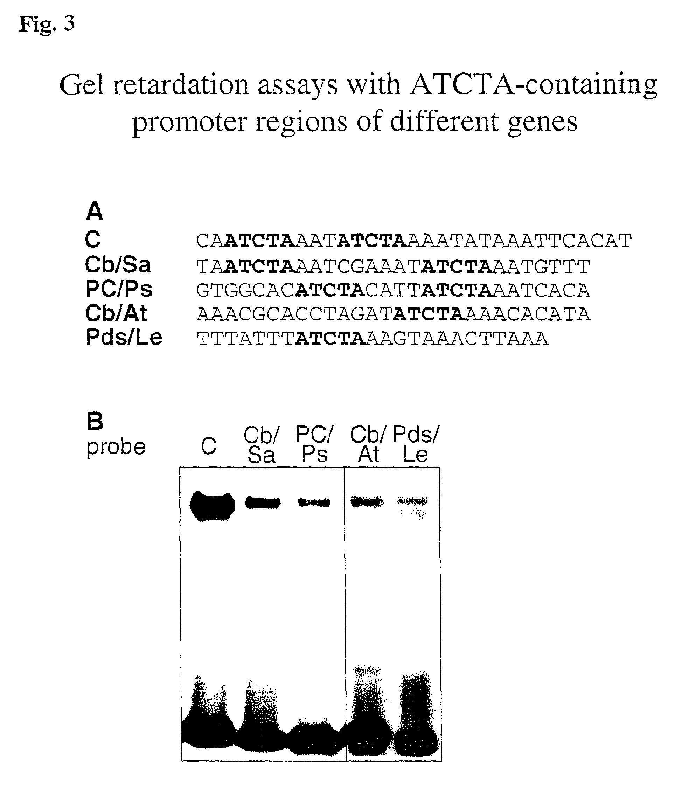

**Figure 3.** Gel retardation assays with ATCTA-containing promoter regions of different genes.
   **A.** Sequences of the oligonucleotides used for the gel retardation assays. ATCTA elements shown in bold face. C, *psy* promoter region between -856 and -825; Cb/Sa, *cab* promoter/*S*. *alba* from bp 397 to 423 (GenBank accession number X16436); PC/Ps, plastocyanin promoter/*P. sativum* from bp 637 to 664 (GenBank accession number X16082); Cb/At, *cabl* promoter/*A. thaliana* from bp 1130 to 1157 (GenBank accession number J04098); Pds/Le, *pds* promoter/*L. esculentum* from bp 657 to 671 (GenBank accession number U46919). **B.** Gel retardation assay with labeled oligonucleotides indicated in A. and nuclear extracts from mustard seedlings illuminated with white light.
**Figure 4.** Nucleotide and deduced amino acid sequence of RAP2.2 from A. thaliana.
   The start and stop codons of the open reading frame are underlined in the nucleotide sequence. Amino acids which form a nuclear location sequence (NLS) are shown in bold and italic letters. A 5' truncated part of the sequence is accessible in the GenBank with the accession number AF003095.
**Fig. 5.** Gel retardation assay for recombinant RAP2.2.
   *E. coli* JM109 transformed with pQE-RAP2.2 were induced with 1 mM IPTG for 4 h. After lysis, the pellet (15 min/13 000 xg) was resuspended in 6 M GuHCl in 1x binding buffer, incubated for 1 h and re-centrifuged. The supernatant was dialyzed over night against binding buffer. 7 µg protein extracts was used for binding reactions. As negative control (C), protein extracts were isolated in the identical way from *E. coli* JM109, transformed with the vector pQE. As a positive control, 2 µg nuclear extracts isolated from illuminated mustard seedlings were used (W). The probe C represents the psy promoter region from -856 to -825 (for sequence, see figs 3 and 4).
**Fig. 6.** Pigment content of homozygous *nos*AtRAP2.2-Arabidopsis lines.
   A. thaliana was transformed with pCamnos-AtRAP2.2. The pigment content of 2 weeks old seedlings of wildtype plants and two homozygous nos-AtRAP2.2 lines (RAP2#2 and RAP2#4) was analyzed via HPLC. The values given depict the average of 4 x 25 seedlings extracted (MAX abs = integrated area values of peaks recorded at their respective λmax).
**Fig. 7.** Pigment and tocopherol patterns of homozygous *nos*AtRAP2.2-Arabidopsis lines.
   The pigment and tocopherol pattern of 2 weeks old seedlings of wildtype plants and two homozygous nos-AtRAP2.2 lines (RAP2#2 and RAP2#4) was analyzed via HPLC. The values given depict the average of 4 x 25 seedlings extracted.
**Fig. 8.** Tocopherol content of homozygous *nos*AtRAP2.2-Arabidopsis lines.
   The tocopherol content of 2 weeks old seedlings of wildtype plants and two homozygous nos-AtRAP2.2 lines (RAP2#2 and RAP2#4) was analyzed via HPLC. The values given depict the average of 4 x 25 seedlings extracted.
**Fig. 9.** Pigment and tocopherol patterns of *nos*AtRAP2.2-BY-2 suspension cell culture
   The total carotenoid and total tocotrienol/tocopherol content of wildtype and two independently transformed nosAtRAP2.2-BY-2 cell cultures (RAP2#a and RAP2#b) was analyzed via HPLC. The values are mean values of 5 measurements.
**Fig. 10.** Nucleotide and amino acid sequence of TomRAP2.2.
**Fig. 11.** Alignment of the amino acid sequences of AtRAP2.2 and TomRAP2.2.
**Fig. 12.** Binding assay for TomRAP2.2.
   *E. coli* JM109 was transformed with pQE, pQE-AtRAP2.2 and pQE-TomRAP2.2, respectively, and induced by growing over night on agar plates containing 1 mM IPTG. Nitrocellulose filter lifts were prepared and incubated with the radiolabelled probe, corresponding to region -856 to -825 of the *psy* promoter (identical with probe C in figs. 2,3,4 and 6).

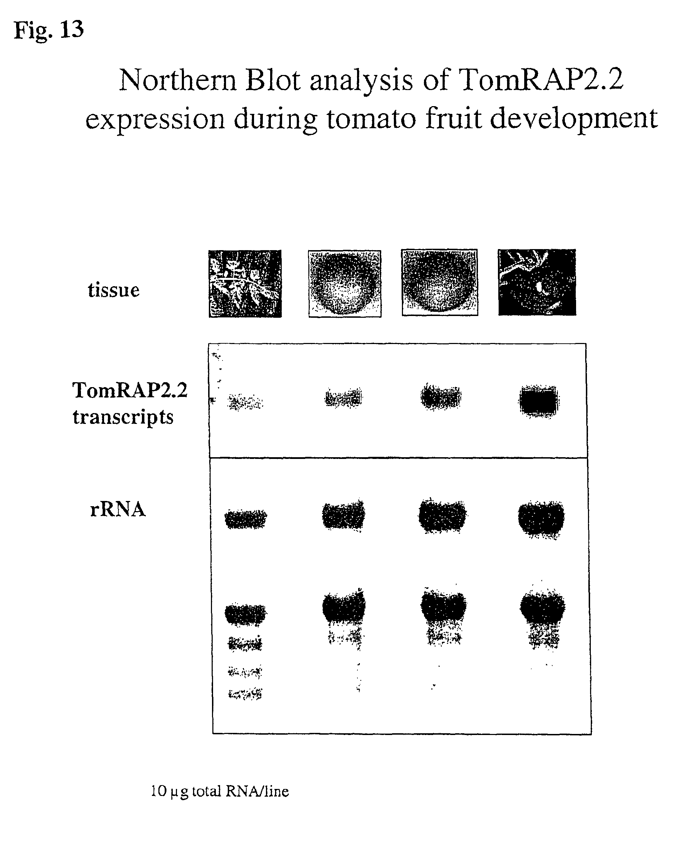

**Fig. 13.** Northern Blot analysis of TomRAP2.2 expression during tomato fruit development.
   10 µg of total RNA isolated from tomato leaves and green, breaker stage and ripe red tomato fruits was separated on a 1,2 % agarose formaldehyde gel (bottom), blotted on nylon membrane and hybridized with the cDNA of TomRAP2.2 (top).

The following examples are illustrative but not limiting of the present invention.

### Examples

### Section 1: Structural and Functional Characterisation of the Phytoene Synthase Promoter

The biosynthesis of carotenoids is regulated in response to developmental stimuli during chromoplast development in flowers and fruits and in response to environmental signals, mainly light, concerted through the processes of chloroplast development. In the former case carotenoids are massively accumulated to exert mainly ecological functions, while in the latter case the coordinated supply of carotenoids and is crucial in physiological/biochemical processes, mainly by their involvement in the photosynthetic electron transport. Carotenoid-free plants cannot survive in the light because in the photosynthetic apparatus, carotenoids function both the acquisition of light energy and the protection against light (Demmig-Adams *et al*., 1996). To perform this task, these pigments are localized together with chlorophyll molecules in the reaction centers of the photosystems as well as in chlorophyll a/b binding proteins (CAB) as part of the light harvesting complexes.

Taking tomato fruit as an example for a chromoplast-bearing tissue, the formation of large amounts of carotenoids involves at least in part a second set of genes differing in their tissue specificity of expression. Two phytoene synthase (PSY) genes as well as two lycopene β-cyclase (CYC-B and LCY-B) genes exist here, differing in their expression pattern. PSY1 plays a predominant role in the non-green tissue of fruit beyond the breaker stage while PSY2 is involved in carotenoid biosynthesis of green leaves. Similarly, CYC-B and LCY-B are predominantly involved in carotenoid biosynthesis in chromoplast or chloroplast-bearing tissues, respectively (for recent reviews, see van den Berg *et al*., 2000; Hirschberg, 2001). The transcriptional regulation of the carotenoid biosynthesis genes seems light-regulated via the phytochrome system (Alba *et al*., 2000), but the coordination with respect to genes involved in photosynthesis, being very strict in leaves, is lost. Thus hitherto unknown developmental factors must be involved. One further determinant of carotenoid accumulation in chromoplasts is given by the formation of lipophilic carotenoid sequestering structures such as lipid globules, crystals, membranes or proteolipid fibrils (Rabbani *et al*., 1998; for review see Camara *et al*., 1995).

Out of the reasons given above we selected *A. thaliana* as a system to investigate the regulation of carotenoid biosynthesis in photosynthetically active tissues. *A. thaliana* does not develop chromoplasts and the expression of *psy* which has been in the focus of our interest is encoded as a single copy gene.

Light, perceived differentially e.g. by the phytochrome system and by cryptochromes (for reviews, see Frankhauser and Chory, 1997; Batschauer, 1998; Lin, 2000) is the most important factor in determining chloroplast development during photomorphogenesis. This process includes mechanisms to protect the emerging photosynthesis against light i.e. to regulate the biosynthesis of carotenoids quantitatively and qualitatively. Consequently, the expression of carotenogenic genes during de-etiolation must be precisely regulated and coordinated to the expression of genes for carotenoid-bearing protein complexes involved in photosynthesis or to the expression of genes involved in chlorophyll biosynthesis etc.

By using quantitative RT-PCR we previously investigated the expression of carotenogenic mRNAs during de-etiolation. This revealed that transcripts of geranylgeranyldiphosphate synthase (*ggps*) and phytoene desaturase (*pds*) remained relatively constant while a significant up-regulation of *psy* was observed during this process. It was concluded that the up-regulation of carotenoid biosynthesis relied essentially on a transcriptional activation of this gene. A detailed analysis of the light qualities mediating the light-induction of *psy* revealed that the phytochrome system was involved. The use of *phyA* and *phyB* mutants allowed to assign a major role to PHYA. In contrast, PHYB was not involved. However, since in *phyA*/*phyB* double mutants increased *psy* transcript amounts were observed under red (R) and blue (B) light, the involvement of other phytochromes (in *A. thaliana* PHYC to E) could not be excluded as well as the participation of cryptochromes (von Lintig *et al*., 1997). In an extension of these studies it was shown later that posttranscriptional as a well as posttranslational events, such as the formation of the prolamellar body and of competent membrane structures are decisive in the regulation of the enzymatic activity of PSY and thus of carotenoid biosynthesis (Welsch *et al*., 2000).

In conclusion, the transcriptional and posttranscriptional regulation of PSY, catalyzing the first reaction specific for carotenoid formation in the pathway appears to be key regulatory, at least in greening tissues. It seemed therefore appropriate to characterize the *psy* promoter to gain a more detailed understanding of *psy* expression. In the present investigation, we report on its cloning and analysis. Using transgenic *A. thaliana* lines, transformed with the promoter fused to the luciferase gene as a reporter, we investigated the promoter activity in different tissues, under different light qualities and in the presence of herbicides. The use of promoter truncations in combination with gel retardation assays allowed the characterization of *cis*-acting elements responsible for the promoter activity. The data indicate a spatial separation of *cis*-acting elements responsible for the mediation of different light responses. Furthermore, *cis*-acting elements were identified which occur also in other photosynthesis-related genes, indicating a possible co-regulation at this level.

### RESULTS

### Cloning of the psy gene and spatial pattern of expression of the luc-reporter gene

The phytoene synthase (*psy*) gene was isolated by screening a genomic library of *A. thaliana* (ecotype Wassilewskija) using a fragment of the *A. thaliana psy* cDNA (GenBank accession number L25812, Bartley and Scolnik, 1994) as a probe. This resulted in a 7 kb DNA fragment which revealed by sequencing the presence of the *psy* gene as part of chromosome 5 (GenBank accession number AB005238 from bp 18150 to bp 25521, Sato *et al*., 1997). The fragment contained the complete transcribed region of 3.1 kb, 525 bp of the 3'-untranscribed region and 3.7 kb of the *psy* promoter region. Figure 1 shows the DNA sequence of the *psy* promoter region up to position -1746. Position +1 represents the first nucleotide of the longest cDNA isolated (GenBank accession number L25812). Two putative TATA motifs are localized at positions -124 and -153.

To investigate regulatory regions within the promoter we constructed six different translational fusions using the luciferase gene as the reporter at the following sites: -1746, -1314, -910, -809, -300 and -196. The fusions -1746/+716, -1314/+716, -300/+716 and -196/+716 were cloned into the vector pBIN121 upstream of the *nos* polyadenylation signal. These constructs were used for *Agrobacterium*-mediated transformation of *A. thaliana* (ecotype Wassilewskija). Homozygous inbreds were produced from several lines of all transformants to be used for expression analysis.

In plants containing the -1746/+716 *psy*/*luc* transgene, luciferase activity was detected in all tissues tested including those which normally contain carotenoids only in trace amounts, if any, such as roots (Figure 2). The highest luciferase activity was measured in flowering buds and ripening seed-pods. Intermediate values were obtained for leaves from the rosette and from leaves (see Discussion).

### Expression during photomorphogenesis

As we have shown previously the phytochrome system mediates the light-induction of PSY as observed on transcript as well as on protein level (von Lintig *et al*., 1997; Welsch *et al*., 2000). Using the different transgenic *psy*/*luc* Arabidopsis lines we aimed at characterizing cis-acting elements within the *psy* promoter responsible for light-induction. For this purpose, luciferase activity was determined from seedlings which were etiolated for 3 d and from etiolated seedlings being illuminated for 24 h with different light qualities (Figure 3). The longest promoter fragment used (-1746/+716) showed two- to three-fold increases in luciferase activity after illumination with white (W), far-red (FR), red (R) and blue (B) light. This correlates well with the increase of *psy* transcript amounts in Arabidopsis seedlings subjected to the same light treatment (von Lintig *et al*., 1997).

Transformants carrying the -1314/+716 *psy*/*luc* fusion or the -1746/+716 *psy*/*luc* fusion were almost indistinguishable in their patterns of luciferase activity. However, further truncation up to position -300 lead to a decrease of about 20 - 30 % in promoter activity under all light conditions. Further truncation up to -196 abolished the induction under R light conditions completely, whereas an increase of luciferase activity under W, FR and B light could still be observed. This indicates the existence of spatially separated *cis*-acting elements responsible for R and FR/B responses: *cis*-acting elements located between -300 and -196 are required for phytochrome response under R while those for phytochrome and/or cryptochrome response under FR and B are located in a TATA box proximal promoter region up to position -196. Furthermore, since both the -300/+716 and -196/+716 *psy*/*luc* fusions mediate the same luciferase activity in etiolated seedlings, the responsible *cis*-acting elements are also localized within the TATA box proximal region up to -196.

Thus, two regulatory regions within the *psy* promoter are mainly involved in the light induction, one being located between -1314 and -300 (in the following TATA box distal) responsible for a basal strong promoter activity the other being located between -300 and the TATA box (in the following TATA box proximal region). This latter region is mainly involved in the differentiated response towards different light qualities.

### Effects of herbicides on the expression

Herbicides that interfere with carotenoid formation have been considered to affect the regulation of carotenoid biosynthesis in a sort of feedback mechanism (Corona *et al*., 1996; Al-Babili *et al*., 1999). Therefore, the influence of different herbicides on carotenoid content and on luciferase activity in herbicide treated *psy*/*luc* plants was analyzed (Figure 4). To include possible effects of herbicides on light induction, the herbicide treatment was carried out with etiolated seedlings as well as with seedlings being illuminated for 24h with W. The two compounds acting on carotenogenic enzymes were norflurazon, inhibiting phytoene desaturase (PDS) and leading to phytoene-accumulation and CPTA inhibiting lycopene cyclase (LCY) yielding lycopene. Furthermore, to compare to effects of gabaculine on the *pds* promoter reported in the literature (Corona *et al*., 1996) we included this compound into our studies. Gabaculine is an inhibitor of chlorophyll biosynthesis acting at the level the enzyme glutamate 1-semialdehyd aminotransferase (Werck-Reichhart *et al*., 1988).

As determined by quantitative HPLC analysis, in etiolated seedlings treated with norflurazon and gabaculine, carotenoid content reached only half of the amount being present in the untreated controls. Furthermore, the carotenoid content remained constant after illumination in the herbicide treated seedlings, whereas a triplication of carotenoids occurred in untreated seedlings. In seedlings grown on CPTA the carotenoid amount was further decreased consisting of only traces of lycopene. Just like with all other herbicides tested, this remained unchanged after illumination. Thus, in the system we used all herbicides led to a decrease in carotenoid content and a complete loss in light-induced accumulation.

This loss, however is not in all cases related to equivalent responses in promoter activity. Interestingly, the decrease in the carotenoid content affected by norflurazon and gabaculine in the dark did not significantly change the luciferase activity in -1746/+716 *psy*/*luc* plants. This indicates that a feedback mechanism did not act in the system used. However, the regulation of signal transduction pathways leading to light-induction of the *psy* promoter occurred undisturbed since an increase in luciferase activity was observed in the respective illuminated seedlings.

CPTA, just like norflurazon and gabaculine did not change the reporter's activity in dark grown seedlings, but in contrast, it abolished light-induction almost absolutely.

### Characterization of cis-acting elements of the TATA box proximal promoter region

The analysis of luciferase expression of the *psy*/*luc* fusions lead to the conclusion that *cis*-acting elements mediating differentiated light responses are located within the TATA box proximal promoter region up to position -300. To further characterize these elements, enhanced mobility shift assays (EMSAs) using nuclear extracts from *S. alba* seedlings were performed. Using the region from -316 to -166 as a probe, the formation of specific protein/DNA complexes was observed, as shown by successfully competing complex detection with the same unlabelled probe (Figure 5). Consistent with the increased promoter activities during de-etiolation (see above), extracts from seedlings illuminated with W showed increased binding activity compared to nuclear extracts from etiolated seedlings. This indicated the involvement of light-induced modifications in the binding activities of corresponding transcription factors. Competition assays with the -210/-179 region revealed that the corresponding cis-acting elements are located within this region (Figure 5). Confirming this finding, EMSAs using the -210/-179 promoter region as a probe led to the formation of protein/DNA complexes (Figure 6). Furthermore, the amounts of complexes formed differed depending on the mustard nuclear extracts. When obtained from etiolated seedlings or R light illuminated seedlings only weak protein/DNA complexes were formed, increased binding of the corresponding *trans*-acting factors was observed with extracts from seedlings illuminated with W, FR and B light.

Although motifs of well-known plant transcription factors could not be found within this -210 to -179 promoter region, two short motifs share some similarity to G-box motifs which are known to be involved in the light regulation of several genes (in the following G1 and G2, see Figure 6; Giuliano *et al*., 1988). According to Schindler *et al*. (1992), mutations in these motifs affect the affinity towards G-box binding factors (GBFs). Therefore we decided to investigate possible function of these two motifs by analyzing the binding activities of DNA fragments successively mutated in these motifs (Figure 6A). The mutation in motif G1 led to the appearance of an additional protein/DNA complex with different migration behavior, whereas the mutation in G2 prevented complex formation. Thus, mutations in both motifs effected complex formation and the involvement of GBFs seems likely. A further clue was obtained by a competition assay using the perfect palindromic G-box of the *rbcS3b* promoter (GenBank accession number X14564, Dedonder *et al*., 1993) as competitor. In this case, the complex formed with the *psy* promoter region from -210 to -179 disappeared almost completely.

### Characterization of cis-acting elements of the TATA box distal promoter region

Beside the TATA box proximal promoter region, which mediates a differential response towards different light qualities, further *cis*-acting elements are located between -1314 and -300 which are responsible for a strong basal i.e. light quality-independent promoter activity (see above). To further restrict the position of these elements we analyzed the transient luciferase expression of additional *psy*/*luc* fusions in green leaves from adult plants (grown under 8 h D/16 h W conditions). In this assay, for each test construct containing the *psy*/*luc* fusion, equimolar amounts of a control plasmid mediating a constitutive expression of GUS (driven by the CaMV-35S promoter) were co-transformed. This allowed to quantify the luciferase values relative to the flourimetrically obtained values for GUS activity, thus to correct for different transformation rates and/or tissue conditions (Norris *et al*., 1993). As shown in Figure 7 the *psy*/*luc* fusion -910/+716 mediated almost the same promoter activity as -1314/+716 (which was already analyzed in stabile transformations, see above). However, a strong decrease in luciferase activity was observed for the -809/+716 *psy*/*luc* fusion. Therefore, *cis*-acting elements responsible for the strong basal promoter activity are located between -910 and -809.

EMSAs were conducted again in order to identify cis-acting elements within this region with nuclear extracts from mustard seedlings (Figure 8). Using the region -946 to -754 of the *psy* promoter as radiolabelled probe the formation of protein/DNA complexes were observed with extracts from etiolated seedlings illuminated for 24 h with W. The specificity of this complex was demonstrated by the competition with the same unlabelled probe. Furthermore, the complex formed could also be competed with a 31 bp fragment, corresponding to the promoter region -856 to -825.

Using this shorter fragment as radiolabelled probe, EMSAs with nuclear extracts isolated from differentially illuminated seedlings revealed that the binding activity of the corresponding *trans*-acting factors increased under all light conditions examined as compared to experiments conducted with extracts from etiolated seedlings (Figure 9). Thus, this region is indeed light responsive but does not confer differentiated responses towards different light qualities.

Although no consensus motifs from known plant transcription factors were detected in the promoter sequence investigated, the repeated occurrence of a pentameric sequence ATCTA seemed indicative. Therefor we decided to investigate a possible involvement of these sequence motifs on complex formation. Both motifs were successively mutated and these fragments were used as competitors. As shown in Figure 9 the individual mutation of the first and of the second ATCTA motif both led to a decrease in binding activity while simultaneous mutations in both motifs completely abolished binding ability. Therefore, the sequence ATCTA represents at least in part the *cis*-acting element for a yet unknown transcription factor mediating a strong *psy* promoter activity.

To allow some generalization, we screened promoter sequences of other carotenogenic genes and of photosynthesis-related genes for the occurrence of the newly characterized motif. Interestingly, it was found also in the promoter regions of *pds* from *L. esculentum* (GenBank accession number U46919; Corona *et al*., 1996) in the *cab* promoters from *S. alba* (GenBank accession number X16436; Gauly *et al*., 1992)and *A. thaliana* (GenBank accession number J04098; Ha and An, 1988) as well as in the plastocyanin (*pc*) promoter of *P. sativum* (GenBank accession number X16082; Last *et al*., 1989). Only in the *cab*/*S. alba* and *pc*/*P. sativum* promoters the ATCTA motif appears in a similar tandem manner as observed in the *psy* promoter. Interestingly, with all of these promoter regions the formation of protein/DNA complexes was observed (Figure 10). Their electrophoretic mobility corresponded to the one observed with the probe from the *psy* promoter however, the amount of retarded radiolabelled probe was different.

### DISCUSSION

Photomorphogenesis requires a coordinated synthesis of carotenoids with all other constituents involved in photosynthesis to allow the formation of a functional photosynthetic apparatus. The enzyme phytoene synthase (PSY) catalyzes the first reaction specifically devoted to carotenoid formation. Therefore the finding that PSY represents the first light-induced step in the pathway on the transcriptional, protein and enzymatic level appears meaningful (von Lintig *et al*., 1997; Welsch *et al*., 2000). In a consequence we decided to focus on the *psy* promoter from *A. thaliana* and analyzed transgenic plants carrying promoter/luciferase fusions.

Correlating to the accumulation of significant amounts of PSY in the prolamellar bodies of etioplasts and the light-induced increase of protein amounts, *psy* promoter activity is clearly detectable in etiolated seedlings and doubles after 24 h of illumination with white light (W) (Figure 3). This parallelism in transcript and protein amounts indicates that translation is not involved in controlling *psy* expression. Posttranslational mechanisms, however, are involved. It must be kept in mind that the increased promoter activity observed with far-red (FR), for instance yields an enzymatically inactive protein which can be activated by the decay of the prolamellar body and the development of thylakoid membranes. In an interpretation this mechanism provides a fast supply of photoprotective carotenoids during the initial photomorphogenesis that is thought to be more responsive than an increase in transcriptional and translational activity (Welsch *et al*., 2000). But apart from this exception, it can be stated here that regulation of carotenoid biosynthesis occurs mainly on the transcriptional level. This is in support of several studies monitoring the steady-state concentrations of carotenogenic mRNAs in different systems (reviewed in Hirschberg *et al*., 1997).

In adult plants, the pattern of tissue specific promoter activity did not in all cases meet the expectations (Figure 2). The signal obtained for roots, for instance, is surprising but may be related to an ongoing small level of carotenoid biosynthesis. Carotenoids produced here may yield abscisic acid (ABA) which, as revealed recently, is formed via carotenoid cleavage (Schwartz *et al*., 1997). In fact, the synthesis of ABA also occurs also in roots (Giraudat *et al*., 1994) so that a weak *psy* promoter activity may account for the synthesis of this phytohormone.

Similarly, in developing seeds as being present in developing seed pods the observed high level *psy* promoter activity may relate to ABA formation, this phytohormone being known to prevent premature germination. (Le Page-Degrivry *et al*., 1990). In addition, mature Arabidopsis seeds contain detectable amounts of carotenoids which are produced during seed development. Therefore in mature ripe seeds the luciferase activity is low.

In flowering buds, their petals being uncolored, the tissues of anthers and pistils probably contribute to the observed high expression of the *psy*/*luc* transgene. While in Arabidopsis separate analysis of these tissues is difficult, a remarkably strong carotenoid biosynthesis can be concluded from experiments with transgenic tomato and tobacco plants expressing GUS under control of the *pds* promoter. In these plants, beside anthers, corollae and pistils showed the strongest expression of the *pds*/GUS transgene (Corona *et al*., 1996). These results were related to a structural function of carotenoids which has been discussed for long (Brooks and Shaw, 1968). However, there is no evidence for possible functions in anthers and stigmas. Beside attracting insects, a photoprotection of germline DNA may play a role in these tissues, analogous to the function of flavonoids in pollen to protect against UV light (Mo *et al*., 1992). In the leaves and in the stem, the high expression of the *psy*/*luc* transgene reflects the necessity to provide carotenoids involved in photosynthesis, besides ABA production.

Carotenoid biosynthesis takes place exclusively in plastids catalyzed by enzymes that are encoded by nuclear genes. It is well-known that inhibitions of normal plastid genesis may affect the expression of such proteins. For instance, plastid defects induced by norflurazon lead to differential alteration in the expression of the nuclear encoded plastid proteins in barley seedlings (Batschauer *et al*., 1986). We therefore studied the effects of norflurazon, CPTA and gabaculine on the expression of the reporter gene. Norflurazon, a classical bleaching herbicide and gabaculine, an inhibitor of chlorophyll biosynthesis did not affect *psy* promoter activity during etiolation and after illumination (Figure 4). This is quite surprising at least in the case of norflurazon, since one would expect increased activity because of the photooxidative consequences it exerts. Thus, neither the accumulation of pathway intermediates nor the reduction of final products at this point seem to exert notable feedback regulatory activity. In this respect the regulation of the *psy* promoter is different from the *pds* promoter from tomato, where an increase in promoter activity was reported in response to these two compounds. Similarly, the lycopene cyclase inhibitor CPTA, leading to lycopene accumulation, was reported to mediate a positive *pds* promoter response in the dark and in the light, whereas our results show that the *psy* promoter did not respond to CPTA in the dark. But interestingly, it lost the ability to respond positively to white light. It remains to be clarified whether this is due to a feedback-signaling character of lycopene itself (or its derivatives). Such a role of lycopene can also be concluded from unexpected results obtained in recent transformation experiments. In rice endosperm, the formation of trans-lycopene, mediated by *psy* and a bacterial *crtI* (a trans-lycopene forming carotene desaturase) led to the establishment of the entire carotenogenic pathway including the formation of xanthophylls (Ye *et al*., 2000). Similarly, the expression of *crtI* in tomatoes did not lead to increased synthesis of total carotenoids, but resulted in an increase of β-carotene. Endogenous carotenoid genes were concurrently up-regulated, expect for *psy*, which was repressed. (Romer *et al*., 2000). This correlates to the effect of lycopene on the *psy* promoter demonstrated here. This, however conflicts with CPTA experiments carried out with daffodil flowers, where lycopene accumulation increased the abundance of *psy* transcripts and protein (Al-Babili *et al*., 1999).

Light induces the accumulation of carotenoids. At the level of the *psy* promoter, all light qualities tested in the present investigation were able to increase *psy* promoter-mediated luciferase activity in transgenic seedlings carrying the -1746/+716 *psy*/*luc* fusion (Figure 3). However, the strongest induction was observed for blue (B) light which indicates a decisive function of cryptochromes for the regulation of *psy*. Besides these photoreceptors, we recently reported the involvement of the phytochrome system in the light-induction of *psy* transcript amounts. Analysis of *phyA* and *phyB* mutants of Arabidopsis demonstrated that mainly PHYA is involved (von Lintig *et al*., 1997). In agreement with these findings, FR light led to a strong increase in promoter activity. The same response pattern toward different light qualities, albeit to a reduced extent than the -1746/+716 *psy*/*luc* fusion, is mediated by the -300/+716 *psy*/*luc* fusion. However, a truncation of 104 bp leading to the -196/+716 *psy*/*luc* fusion abolished the induction in R light completely. This indicates that *cis*-acting elements responsible for phytochrome response under R light are located between -300 and -196 of the promoter, whereas elements mediating responses to FR and B light and a minimal promoter activity in the dark are located in the first 196 bp. The differentiation of light responses occurs also at the level of *trans*-acting factors. EMSAs using nuclear extracts from mustard seedlings illuminated for 24 h with different light qualities demonstrate that the amounts of protein/DNA complexes formed with the 215/-166 promoter region correspond to the *psy* promoter activity measured under the same light qualities (Figure 6). Therefore, the binding activities of the *trans*-acting factors involved here are regulated by FR- and B-receptor mediated mechanisms. The most intensely investigated group of transcription factors involved in light regulation is represented by G-box binding factors (GBFs; Giuliano *et al*., 1988). Indeed, a competition assay with the perfect palindromic G-box of the *rbcS3b* promoter of *A. thaliana* demonstrated the involvement of GBFs in the protein/DNA complexes formed within this *psy* promoter region (Figure 6). Known mechanisms involved in an increase of GBF binding activity like light-induced translocation from the cytoplasm to the nucleus (Harter *et al*., 1994) or phosphorylation (Klimczak *et al*., 1992) may be responsible for the effects observed here.

The *psy* promoter region from -215 to -166 contains two G-box like motifs (named G1 and G2). As shown by EMSAs with probes carrying mutations in G1 and G2, both motifs are involved in the formation of the protein/DNA complex observed (Figure 5). Since GBFs belong to the group of basic leucine zipper transcription factors (bZIP) it is generally assumed that a central ACGT core is necessary for binding (Foster *et al*., 1994) while flanking sequences determine sequence specificity (Williams *et al*., 1992; Izawa *et al*., 1993). Interestingly, the G-box like motifs in the *psy* promoter do not meet this criteria, because they contain the sequences ACGA and TCGA as a core in G1 and G2, respectively. However, there are some examples that a central ACGT core is not obligatory (de Pater *et al*., 1994). The exact identity of the GBFs involved in the *psy* promoter's G1 and G2 binding remain to be investigated.

Apart from the minimal *psy* promoter activity mediated by the -300/+716 promoter region, further *cis*-acting elements upstream up to -1314 are necessary for a strong promoter activity, as reflected by the strong luciferase activity in the -1314/+716 *psy*/*luc* plants. Quantification of transient expression revealed that these elements are located between position -910 and -809 (Figure 7). Competition EMSAs with nuclear extracts from mustard seedlings illuminated with W restricts the localization of the corresponding *cis*-acting elements between -856 and -825. The amounts of protein/DNA complexes formed with this promoter region when nuclear extracts from differently illuminated mustard seedlings were used remained constant (Figure 9). This indicates that the corresponding *trans*-acting factors are constitutively required for high basal level of *psy* promoter activity, as reflected by the decrease in *psy* promoter mediated luciferase activity in the -300/+716 fusion observed for all light qualities tested.

The sequence of the DNA fragments used for these EMSAs contained two pentameric ATCTA motifs ordered in a tandem manner. Competition EMSAs with DNA fragments carrying mutations within both motifs demonstrated that these boxes represent the binding motif for a unknown *trans*-acting factor (Figure 9). Furthermore, the existence of one ATCTA motif seems to be sufficient for a weak binding as a mutation in either the first or the second box could not compete the complexes formed with the wildtype sequence completely.

The presence of the ATCTA motif in other promoter regions from genes involved in carotenoid biosynthesis and photosynthesis indicates a general regulatory importance of this site. This is further corroborated by the observation that with the different ATCTA-containing promoter regions used, protein/DNA complexes - although differing in amount - with identical migration behavior were obtained (Figure 10).

The occurrence of the ATCTA box in photosynthesis-related genes and the existence of a transcription factor binding here points to its more general relevance. Work is in progress for its identification.

### MATERIALS AND METHODS

### Cloning of the psy gene

For the isolation of the *psy* gene, a genomic library of *A. thaliana*, ecotype Wassilewskija (Schulz *et al*., 1994) was screened. As a probe, a PCR fragment was amplified from a plasmid carrying the *A. thaliana psy*-cDNA (Bartley and Scolnik, 1994) with the oligonucleotides Psysen (TTG TGG GTT GGT AAG GGT TC) and Psyasen (CGT AGA TTG CCC AAA TCG CC) and radiolabelled using the Klenow fragment (Klenow and Henningsen, 1970). From the isolated genomic clone, a 7 kb *Eco*RI fragment containing the *psy* gene was subcloned into pBSK-. 5' subclones were produced using restriction sites and by exonuclease method with the enzymes *Bam*HI and *Pst*I (Henikoff 1984) and sequenced.

Translational fusions of different promoter truncations with the luciferase gene were produced by subcloning corresponding promoter fragment into the vector pSP*luc*^{*+*} (Promega, Mannheim, Germany). For binary constructs, *psy*/*luc* fusions were isolated and subcloned into the vector pBIN-35S-mGFP (kindly provided from Dr. M. Rodriguez), thereby replacing the 35S-mGFP region.

### Luciferase and GUS measurement

Luciferase activity in seedlings from homozygous T2 lines was determined according to lida *et al*. (1995) using a luminometer (lumat LB9501, Berthold, Wildbad, Germany). Protein concentration in the supernatants was determined according to Bradford (1976). All measurements were repeated three times for each T2 line; the values represent the average of all experiments for at least two strong expressing line each transformation.

Quantification of transient expression was performed according to Norris *et al*., (1993) using the plasmid pHGi35SGUS (kindly provided by Dr. M. Rodriguez) as control plasmid. Leaves of 3 week old Arabidopsis plants were bombarded with 14 pmol of an equimolar mixture of control plasmid and test plasmid (i.e. *psy*/*luc* fusions in the vector pSP*luc*⁺) using a self-made particle influence gun (Feiner *et al*., 1992). After incubation for 24 h in dim light, extracts were prepared and protein concentration (Bradford 1976) and luciferase activity (Iida *et al*., 1995) were determined immediately. Remaining extracts were kept for 24 h at -80°C, re-centrifuged and GUS activity was measured using a fluorescence spectrometer (LS50B, Perkin Elmer, Rodgau-Jügesheim, Germany).

### Enhanced mobility shift assay

Nuclear extracts were isolated from cotyledons of mustard seedlings according to Dignam *et al*. (1983) and Jensen *et al*. (1988). Protein concentration was performed according to Bradford (1976) and aliquots were stored at -80°C.

DNA fragments used as probes which were larger than 30 bp were end-labeled with α-³²PdATP or α-³²PdCTP. Smaller probes were prepared using partially overlapping oligonucleotides and filled-in with α-³²PdATP or α-³²PdCTP using the Klenow fragment (Klenow and Henningsen 1970). DNA fragments used as competitors were prepared in the identical procedure except that all nucleotides were supplied as non-radioactive. All DNA fragments were purified by gel-chromatography (MicroSpin S-200, Amersham Pharmacia Biotech, Freiburg, Germany), incorporation of radioactivity was quantified by scintillation counting.

Binding reaction were done in a final volume of 30 µl and contained 15 µl 2x binding buffer (24 mM Tris/HCl, pH 7,9; 24 % glycerol (v/v), 70 mM KCI; 0,14 mM EDTA; 0,95 mM PMSF, 2,15 mM DTT; 15 mM MgCl₂; 0,01 % bromphenol blue (w/v)), 6-10 nmol probe, 2 µg poly(dIdC).poly(dIdC), 2 µg nuclear proteins and specific competitor DNA as indicated in the figure legends. After incubation at room temperature for 10 min, the binding mixtures were loaded on 4-8% polyacrylamid gels and run in 25 mM Tris/HCl, pH 8,3, 190 mM glycin and 1 mM EDTA at 4°C and 200 V. Gels were wrapped in cellophane, dried at 60 °C under vacuum and autoradiographed.

### Plant transformation and growth conditions

*A. thaliana* (ecotype Wassilewskija) plants were transformed by vacuum infiltration (Bechtold *et al*., 1993) with agrobacteria strain GV3101 (Koncz and Schell, 1986) containing the binary *psy* promoter/luciferase constructs (see above). Putative T₁ transformants were selected on kanamycin-containing (50 µg ml⁻¹) Murashige-Skoog (MS) agar Petri plates. Of the initially identified T1 transformants, 5 lines per transformation were propagated. Homozygous T2 progenies were identified by the selection pattern of the corresponding T3 progenies on kanamycin-containing agar plates.

Seeds of white mustard (*Sinapis alba* L., harvest 1982) used for isolation of nuclear extracts were obtained from Asgrow Company (Freiburg, Germany).

Seeds from homozygous Arabidopsis lines were surface-sterilized, plated on MS agar plates (1 x MS salts; 0,5 g MES/KOH, pH 5,7; 0,4 % phytoagar), vernalized for 4 d at 4°C and germinated for 3 days in darkness. Mustard seedlings were germinated and grown for 3 days on moist paper in darkness. Subsequently, seedlings were illuminated for 24 h with white light or the following light conditions: white light (W), Osram L40W (73 lamps) + Philips TLD40W (18 lamps), fluence rate 10.9 W/m²; red light (R), λₘₐₓ = 660 nm, fluence rate 5 W/m²; far-red (FR), λₘₐₓ = 730 nm, fluence rate 3 W/m^{2;} blue light (B), Philips TLD36W/18 lamps, plexiglass filter 627 (Röhm & Haas, Darmstadt, Germany), λₘₐₓ = 436 nm, fluence rate 4.1 W/m^{2.}

### Herbicide treatments and HPLC analysis

For herbicide-containing agar plates, autoclaved MS agar was cooled to 40°C, stock solutions of the corresponding chemicals were added and the media poured in Petri plates. The following substances were used: CPTA (2-(4-chlorophenylthio)triethylammonium chloride; synthesized according to Scheutz and Baldwin, 1958); norflurazon (SAN 9789; 4-chloro-5-(methylamino)-2-(α,α,α-trifluoro-m-tolyl)-3(2H)-pyridazinon; Mayer *et al*., 1989); gabaculine (2-amino-2,3-dihydrobenzolic acid; Sigma, Germany).

Seeds from *A thaliana* (ecotype Wassilewskija) were surface-sterilized and plated on MS agar plates and herbicides-containing MS agar plates, respectively. After vernalization for 4 days at 4°C, seeds were germinated for 3 days in darkness and thereafter illuminated for 24 h with white light. Seedlings were harvested, immediately frozen in liquid nitrogen, grinded to powder and resuspended in 500 µl 100 mM Tris. An aliquot of 50 µl was taken for protein determination (Bradford, 1976). The remaining suspension was extracted with an equal amount of CHCl₃/methanol (2/1, v/v). The organic phase was separated by centrifugation (5 min, 10 000*g), collected and the aqueous phases was re-extraction twice with CHCl₃. The collected organic phases were subjected to quantitative HPLC analysis. As internal standards lycopin (Hoffmann-LaRoche, Basel, Switzerland) was used for control seedling and seedlings treated with norflurazon and gabaculine whereas β-carotene was used for CPTA-treated seedlings. The HPLC-system consisting of a C30 reverse phase column (YMC 200, CROM, Herrenberg, Germany) and a gradient system using (A) methanol/*tert*-butyl-methyl ether/H₂O (75:15:15, v/v/v) as the polar solvent and (B) methanol/*tert*-butyl-methyl ether (50/50, v/v) as the nonpolar solvent. The gradient profile was 100 % A linear to 0 % A in 100 min, then isocratic for an additional 10 min at a constant flow-rate of 1 ml/min. UV/VIS spectra and radioactivity were monitored simultaneously by a photodiode array detector (Waters 986, Eschborn, Germany) and a flow-through liquid scintillation counter (Ramona, Raytest, Wildbad, Germany). For analysis the Millenium software package version 2.1 (Waters) was used. Products were identified by chromatographic comparison to authentic reference substances isolated from *tangerine* tomatoes (Clough and Pattenden 1979) and by their spectral characteristics.

### LITERATURE CITED

Alba R, Cordonnier-Pratt MM, Pratt LH (2000) Fruit-localized phytochromes regulate lycopene accumulation independently of ethylene production in tomato. Plant Physiol 123: 363-370.
Al-Babili S, Hartung W, Kleinig H, Beyer P (1999) CPTA modulates levels of carotenogenic proteins and their mRNAs and affects carotenoid and ABA content as well as chromoplast structure in *Narcissus pseudonarcissus* flowers. Plant Biol 1: 607-612.
Bartley GE, Scolnik PA (1994) Nucleotide sequence of an *Arabidopsis* cDNA for phytoene synthase. Plant Physiol 104: 1471-1472.
Batschauer A (1998) Photoreceptors of higher plants. Planta 206: 479-492.
Bechtold N, Ellis J, Pelletier G (1993) *In planta Agrobacterium* mediated gene transfer by infiltration of adult *Arabidopsis thaliana* plants. C R Acad Sci Paris 316: 1194-1199.
Bradford MM (1976) A rapid and sensitive method for the quantitation of microgram quantities of protein utilizing the principle of protein-dye binding. Anal Biochem 72: 248-54.
Camara B, Hugueney P, Bouvier F, Kuntz M, Moneger R (1995) Biochemistry and molecular biology of chromoplast development. Int Rev Cytol 163: 175-247.
Clough J, Pattenden MG (1979) Naturally occurring poly-cis carotenoids. Stereochemistry of poly-cis lycopene and its congeners in *tangerine* tomato fruits. J Chem Soc Chem Commun: 616-619.
Corona V, Aracri B, Kosturkova G, Bartley GE, Pitto L, Giorgetti L, Scolnik P, Giuliano G (1996) Regulation of a carotenoid biosynthesis gene promoter during plant development. Plant J 9: 505-512.
De Pater S, Katagiri F, Kijne J, Chua NH (1994) bZIP proteins bind to a palindromic sequence without an ACGT core located in a seed-specific element of the pea lectin promoter. Plant J 6: 133-140.
Dedonder A, Rethy R, Fredericq H, van Montagu M, Krebbers E (1993) *Arabidopsis rbcS* genes are differentially regulated by light. Plant Physiol 101: 801-808.
Demmig-Adams B, Gilmore AM, Adams WWIII (1996) *In vivo* functions of carotenoids in higher plants. FASEB J 10: 403-412.
Dignam JD, Lebovitz RM, Roeder RG (1983) Accurate transcription initiation by RNA polymerase II in a soluble extract from isolated mammalian nuclei. Nucleic Acids Res 11: 1475-1489.
Feiner JJ, Vain P, Jones MW, McMullen MD (1992) Development of the particle inflow gun for DNA delivery to plant cells. Plant Cell Rep 11: 323-328.
Foster R, Izawa T, Chua NH (1994) Plant bZIP proteins gather at ACGT elements. FASEB J 8: 192-200.
Frankhauser C, Chory J (1997) Light control of plant development. Ann Rev Cell Dev Biol 13: 203-229.
Gauly A, Batschauer A, von Amim A, Kössel H (1992) Isolation and characterization of a gene encoding a chlorophyll *a*/*b*-binding protein from mustard and the targeting of the encoded protein to the thylakoid membrane of pea chloroplasts *in vitro.* Plant Mol Biol 19: 277-287.
Giuliano G, Pichersky E, Malik VS, Timko MP, Scolnik PA, Cashmore AR (1988) An evolutionarily conserved protein binding sequence upstream of a plant light-regulated gene. Proc Natl Acad Sci USA 85: 7089-7093.
Ha SBB, An G (1988) Identification of upstream regulatory elements involved in the developmental expression of the *Arabidopsis thaliana cabl* gene. Proc Natl Acad Sci USA 85: 8017-8021.
Harter K, Kircher S, Frohnmeyer H, Krenz M, Nagy F, Schäfer E (1994) Light-regulated modification and nuclear translocation of cytosolic G-box binding factors in parsley. Plant Cell 6: 545-559.
Henikoff S (1984) Unidirectional digestion with exonuclease III creates targeted breakpoints for DNA sequencing. Gene 28: 351-359.
Hirschberg J (2001) Carotenoid biosynthesis in flowering plants. Curr Op Plant Biol 4: 210-218.
Iida A, Nagasawa A, Oeda K (1994) Positive and negative *cis*-regulatory regions in the soybean glycinin promoter identified by quantitative transient gene expression. Plant Cell Rep 14: 539-544.
Izawa T, Foster R, Chua NH (1993) Plant bZIP protein DNA binding specificity. J Mol Biol 233: 1131-1144.
Jensen EO, Marcker KA, Schell J, de Bruijn F (1988) Interaction of a nodule specific, *trans*-acting factor with distinct DNA elements in the soybean leghaemoglobin Ibc₃ 5' upstream region. EMBO J 7: 1265-1271.
Klenow H, Henningsen L (1970) Selective elimination of the exonuclease activity of the deoxyribonucleic acid polymerase from *Escherichia coli* by limited proteolysis. Proc Natl Acad Sci USA 65: 168-175.
Klimczak LJ, Schindler U, Cashmore AR (1992) DNA binding activity of the *Arabidopsis* G-box binding factor GBF1 is stimulated by phosphorylation by casein kinase II from broccoli. Plant Cell 4: 87-98.
Koncz C, Schell J (1986) The promoter of T_{L}-DNA gene 5 controls the tissue specific expression of chimeric genes carried by a novel type of *Agrobacterium* binary vector. Mol Gen Genetics 204: 383-396.
Last DI, Gray J.C. (1989) Plastocyanin is encoded by a single-copy gene in the pea haploid genome. Plant Mol Biol 12: 655-666.
Lin C (2000) Plant blue-light receptors. Trends Plant Sci 5: 337-42.
Mo Y, Nagel C, Taylor L (1992) Biochemical complementation of chalcone synthase mutants defines a role for flavonols in functional pollen. Proc Natl Acad Sci USA 89: 7213-7217.
Norris SR, Meyer SE, Callis J (1993) The intron of *Arabidopsis thaliana* polyubiquitin genes is conserved in location and is a quantitative determinant of chimeric gene expression. Plant Mol Biol. 21: 895-906.
Rabbani S, Beyer P, von Lintig J, Hugueney P, Kleinig H (1998) Induced β-carotene synthesis driven by triacylglycerol deposition in the unicellular alga *Dunaliella bardawil*. Plant Physiol 116: 1239-1248.
Romer S, Fraser PD, Kiano JW, Shipton CA, Misawa N, Schuch W, Bramley PM (2000) Elevation of the provitamin A content of transgenic tomato plants. Nat Biotechnol 18: 666-669.
Sato S, Kotani H, Nakamura Y, Kaneko T, Asamizu E, Fukami M, Miyajima N, Tabata S (1997) Structural analysis of *Arabidopsis thaliana* chromosome 5.1. Sequence features of the 1.6 Mb regions covered by twenty physically assigned P1 clones. DNA Res 30: 215-230.
Schindler U, Menkens AE, Beckmann H, Ecker JR, Cashmore AR (1992) Heterodimerization between light-regulated and ubiquitously expressed *Arabidopsis* GBF bZIP proteins. EMBO J 11: 1261-1273.
Schulz B, Bennett MJ, Dilkes BP, Feldmann KA (1994) T-DNA tagging in Arabidopsis thaliana: cloning by gene disruption. Plant Mol Biol Manual K3; Kluwer Academic Publishers, Belgium, 1-17.
Schwartz SH, Tan BC, Gage DA, Zeevaart JAD, McCarty DR (1997) Specific oxidative cleavage of carotenoids by VP14 of maize. Science 276: 1872-1874.
Van den Berg H, Faulks R, Fernando Granado H, Hirschberg J, Olmedilla B, Sandmann G, Southon S, Stahl W (2000) The potential for the improvement of carotenoid levels in foods and the likely systemic effects. J Sci Food Agric 80: 880-912.
Von Lintig J, Welsch R, Bonk M, Giuliano G, Batschauer A, Kleinig H (1997) Light-dependent regulation of carotenoid biosynthesis occurs at the level of phytoene synthase expression and is mediated by phytochrome in *Sinapis alba* and *Arabidopsis thaliana* seedlings. Plant J 12: 625-634.
Welsch R, Beyer P, Hugueney P, Kleinig H, von Lintig J (2000) Regulation and activation of phytoene synthase, a key enzyme in carotenoid biosynthesis, during photomorphogenesis. Planta 211: 846-854.
Werck-Reichhart D, Jones OT, Durst F (1988) Haem synthesis during cytochrome P-450 induction in higher plants. 5-Aminolaevulinic acid synthesis through a five-carbon pathway in *Helianthus tuberosus* tuber tissues aged in the dark. Biochem J 249: 473-480.
Williams ME, Foster R, Chua NH (1992) Sequences flanking the hexameric G-box core CACGTG affect the specificity of protein binding. Plant Cell 4: 485-496.
Ye GN, Stone D, Pang SZ, Creely W, Gonzalez K, Hinchee M (1999) *Arabidopsis* ovule is the target for *Agrobacterium in planta* vacuum infiltration transformation. Plant J 19: 249-57.

**Section 2:** Transcription Factor RAP2.2

### 1. Characterization of the cis-acting element

Although no consensus motifs from known plant transcription factors were detected in the promoter sequence investigated, the repeated occurrence of a pentameric sequence ATCTA seemed indicative. Therefore both motifs were successively mutagenized and the resulting fragments were used as competitors. As shown in fig. 2 the individual mutation of the first and of the second ATCTA motif both led to a decrease in binding activity while simultaneous mutations in both motifs completely abolished binding ability. Therefore, the sequence ATCTA represents at least in part the *cis*-acting element for a transcription factor mediating a strong *psy* promoter activity.

To allow some generalization, we screened promoter sequences of other carotenogenic genes and of photosynthesis-related genes for the occurrence of the newly characterized motif. Interestingly, among others, it was found in the promoter regions of *pds* from *L. esculentum* (GenBank accession number U46919; Corona *et al*., 1996) in the chlorophyll a/b binding protein (*cab*) promoters from *S. alba* (GenBank accession number X16436; Gauly *et al*., 1992) and *A. thaliana* (GenBank accession number J04098; Ha and An, 1988) as well as in the plastocyanin (*pc*) promoter of *P. sativum* (GenBank accession number X16082; Last *et al*., 1989). Only in the *cab*/*S. alba* and *pc*/*P. sativum* promoters the ATCTA motif appears in a similar tandem manner as observed in the *psy* promoter. With all of these promoter regions the formation of protein/DNA complexes was observed (fig. 3). Their electrophoretic mobility corresponded to the one observed with the probe from the *psy* promoter, however, the amount of retarded radiolabelled probe was different.

Further investigations of other promoters of plastid-localized proteins revealed that the ATCTA motif exists also in genes involved in tocotrienol/tocopherol biosynthesis. Therefore, It can be noted that the motif characterized is involved in the regulation of genes involved in carotenoid biosynthesis, photosynthesis and tocotrienol/tocopherol biosynthesis. Table 1 summarizes the position and number of ATCTA elements in several genes of different organisms which are involved in the pathways mentioned; table 2 lists the corresponding nucleotide sequences of the corresponding genes. Interestingly, in most cases also the sequences neighboring the ATCTA element which was characterized to be crucial for the binding show similarities.

### 2. South-Western Screening

Since no plant transcription factor was known which binds to a cis-acting element with the sequence ATCTA, a chapter of a λZAPII-cDNA-library of *A. thaliana* (Kieber *et al*., 1993) comprising cDNAs of 1-2 kb in size was screened via South-Western screening. As a radiolabelled probe, the primers 5' - CAA TCT AAA TAT CTA AAA TAT AAA - 3' and 5' - TTT ATA TTT TAG ATA TTT AGA TTG - 3 were used to produce concatenated oligonucleotides according to Sambrook *et al*. (1989) resulting in concatemers of the sequence 5' - CAA TCT AAA TAT CTA AAA TAT AAA - 3'. Nitrocellulose filters were prepared in two sets; one set was used for the incubation with the radiolabelled probe directly, whereas the second copy was subjected to denaturation using 6 M GuHCl followed by renaturation (Sambrook *et al.,* 1989). Both filter sets were incubated with binding buffer (25 mM HEPES/KOH, pH 7,9; 7,5 mM MgCl2; 20 mM KCI; 0,07 mM EDTA) containing 1 mM DTT, 0,25 % fat-free milk powder and 10 µg/ml calf thyme DNA for 12 h at 4°C.

The screening process revealed several positive signals in the first round. However, only one clone from a filter set which was subjected to re-/denaturation revealed a positive signal in the second round of screening. The clone was *in vivo* excised and the plasmid was sequenced using reverse and universal primers. This revealed sequence identity with the sequence of RAP2.2 from *A. thaliana* (accession number af003095), starting with bp number 78. Therefore, a 5'-truncated cDNA of RAP2.2 was isolated, termed pblue-RAPΔ.

In order to get the full-length cDNA of RAP2.2, the 5'-part was cloned from the same chapter used for the screening via PCR. The oligonucleotides reverse and RAPasen (5' - GCC ACG GAT TCT GCG TGC - 3') were used as primers. This revealed a 450 bp fragment which was digested with *Bam*HI and *Pst*I and ligated into pblue-RAPΔ, digested with *Bam*HI and *Pst*I, thereby using an internal *Pst*I site within RAPΔ downstream the annealing site for RAPasen. A sequencing reaction again performed with the reverse primer verified that the 5'-end of RAP2.2 of *A. thaliana* was cloned. Fig. 4 shows the nucleotide and the deduced amino acid sequence of the RAP2.2 cDNA cloned from *A. thaliana*.

### 3. Binding assay with recombinant RAP2.2

In order to confirm that RAP2.2 binds to the cis-acting element characterized in the gel retardation experiments and used for the South-Western screening, gel retardation assays were performed using the recombinant protein. For this, the RAP2.2 cDNA was subcloned into the expression vector pQE30 (Quiagen, Hilden, Germany), thereby providing the recombinant protein with an N-terminal His-tag. Via site-directed mutagenesis using the primer 5' - CGT GAA GGA TCC ATG TGT GG - 3', a *Bam*HI site was introduced at position 67 of the RAP2.2 cDNA. The fragment amplified was digested with *Bam*HI and *Xho*I and ligated into pQE30, digested with *Bam*HI and *Sal*I.

The purification of the recombinant protein revealed not to be possible via metal affinity under native conditions. Similarly, gel retardation assays using the lysate of bacteria expressing RAP2.2 were unsuccessful. The analysis of different bacterial fractions using antibodies directed against the His-tag revealed that the recombinant protein was accumulated in inclusion bodies. Therefore and in a correspondence to the screening conditions required to identify RAP2.2, it was decided to use a denaturation/renaturation cycle before using the extracts for gel retardation. For this, bacteria were lysed by 2 passages through the French Press, centrifuged (13 000*g/15 min.) and the pellet was resuspended into 6 M GuHCl in binding buffer (see above). After incubation of 1 h at RT, the mixture was re-centrifuged and dialyzed against 50x volumes binding buffer at 4°C over night. Protein concentration was determined according to Bradford (1976).

For gel retardation assays, 7 µg of proteins were incubated with 10 nmol (10 000 cpm) radiolabelled probe and 2 µg poly[d(I-C)] in a total volume of 30 µl in the following reaction buffer: 12 mM Tris/HCl, pH 7,9; 12 % glycerol; 35 mM KCI; 0,07 mM EDTA; 0,45 mM PMSF; 1,1 mM DTT; 7,5 mM MgCl₂; 0,005% brom phenolblue. After incubation for 10 min. at RT, samples were separated on a 8 % PAA gel with running buffer (25 mM Tris/HCl, pH 8,3; 190 mM glycin; 1 mM EDTA). Electrophoresis was carried out in running buffer at 4°C and 200 V for 2 h, the gel was vacuum-dried and an X-ray film was exposed.

Fig. 5 shows that recombinant RAP2.2 specifically binds the region from -856 to -825 of the *psy* promoter, whereas with protein extracts from *E. coli* cells transformed with the empty vector pQE30, no complex formation occurred.

### 4. Transformation of A. thaliana

To investigate the involvement of RAP2.2 in the regulation of carotenoid biosynthesis and to investigate its role in photosynthesis-linked biosynthetic pathways, transgenic *A. thaliana* lines were produced which constitutively expressed the RAP2.2 cDNA under control of the *nos* promoter. For the construction of the binary vector pCamnos-RAP2.2, the *Xba*I/*Eco*RV-fragment of pblue-RAP2.2 was subcloned into pCambial390/ntII/nosP (kindly provided by P. Schaub), digested with *Spe*I/*Pml*I. By this, the cDNA of RAP2.2 gets positioned downstream of the *nos*-promoter and upstream of the *nos*-terminator. This plasmid was used to transform competent agrobacteria, strain GV3101 (Koncz and Schell, 1986).

*A. thaliana* (ecotype Wassilewskija) plants were transformed by vacuum infiltration (Bechtold *et al*., 1993) with the transformed agrobacteria. Putative T₁ transformants were selected on kanamycin-containing (50 µg ml⁻¹) Murashige-Skoog (MS) agar Petri plates. Out of the initially identified T1 transformants, 5 lines per transformation were propagated. Homozygous T2 progenies were identified by the segregation pattern of the corresponding T3 progenies on kanamycin-containing agar plates.

### 5. Transformation of BY-2 cell suspension culture

A *N*. *tabacum* BY-2 cell suspension culture (Nagata *et al*., 1992) was grown in MS culture medium (1x MS salts, pH 5,8; 0,2 g/l KH₂PO₄; 30 g/l sucrose; 0,2 mg/l 2,4-D; 1 mg/l Thiamin-HCl; 0,1 g/l myo-Inositol) at 28 °C/130 rpm in the dark. The culture was subcultured weekly by transferring 2 ml of culture into 100 ml fresh media.

For transformation, 1 ml of cell culture from the 6^{th} day after inoculation was mixed with 12 ml MS medium and 200 µl of Agrobacteria (strain GV3101), transformed with pCamnos-RAP2.2. After 4 days of co-incubation at 28°C in the dark, the cells were centrifuged (5 min./2000*g), the supernatant was discarded and the cell pellet washed twice with culture medium containing 125 µg/ml β-bactyl (SmithKline Beecham, München, Germany). Thereafter, the cells were transferred into 50 ml fresh culture medium containing 125 µg/ml β-bactyl and 100 µg/ml kanamycin. After the culture reached the stationary phase, 1 ml was subcultured into fresh medium containing antibiotics. Cells were grown and transferred once more in fresh media before pigment analysis.

### 6. Pigment analysis

### 6.1. Pigment analysis of A. thaliana seedlings

For the analysis of pigment content and pigment pattern of transgenic *A. thaliana* lines expressing RAP2.2 under control of the *nos* promoter, same amounts of seeds of transgenic lines and wildtype *A. thaliana* (ecotype Wassilewskija), respectively, were surface-sterilized with bleach and equally spread on MS-agar containing Magenta boxes (Sigma, Germany). Seeds were vernalized for 4 days and transferred to the growth chamber (8 h D, 16 h W; 25°C). After 14 days, 25 seedlings were harvested, frozen in liquid nitrogen and lyophilized. The dry weight was determined and the pigments were extracted three times by adding 2 ml of acetone and sonication. After centrifugation (5 min./6000*g), the acetone phases were pooled, dried under a stream of nitrogen and the pigments redissolved in 200 µl of chloroform.

### 6.2. Pigment analysis of BY-2 cell suspension culture

BY-2 cell suspension cells were harvested 7 days after subculture, excessive culture medium was removed by filtration through Whatman paper and the cells were lyophilized. Dry weight was determined and the cells were resuspended in 35 ml of acetone. At this step, 100 µl of α-tocopherol (100 ng/ml in chloroform; Sigma, Germany) were added as an internal standard. Pigments were extracted twice by sonication. Cell debris was removed by centrifugation (5 min./6000*g) and filtration through membrane filters (0,2 µm; Schleicher & Schuell, Dassel, Germany), the acetone phases were dried by rotary evaporation and the pigments were dissolved in 30 µl chloroform.

### 6.3 HPLC system

30 µl of the *A. thaliana* pigment extracts and the complete sample from the BY-2 cell suspension cell culture were analyzed by a HPLC-system. This consisted of a C30 reverse phase column (YMC 200, CROM, Herrenberg, Germany) and a gradient system using (A) methanol/*tert*-butyl-methyl ether (50:50, v/v) as the nonpolar solvent and (B) methanol/*tert*-butyl-methyl ether/H₂O (75:15:15, by vol.) as the polar solvent. The column was developed at a constant flow-rate of 1 ml/min with 0 % A linear to 57 % A in 45 min, then isocratic for an additional 55 min. UV/VIS spectra and fluorescence signals were monitored simultaneously by a photodiode array detector (Waters 986, Eschborn, Germany) and a scanning fluorescence detector (Waters 474, Eschborn, Germany; excitation at 293 nm, emission at 324 nm). For analysis, the Millenium software package version 2.1 (Waters) was used. Products were identified by chromatographic comparison to authentic reference substances and by their spectral characteristics.

From each transgenic *A. thaliana* line and from the wildtype, 4 x 25 seedlings were analyzed, respectively. The data given represent the mean values of all measurements done in MAX absorbency per mg dry weight. Tocopherol data are described in fluorescence per mg dry weight. Fig. 6 shows total carotenoid and chlorophyll content of two homozygous transgenic nosAtRAP2.2 *A. thaliana*-lines compared to the values obtained for the wildtype, demonstrating that both carotenoid and chlorophyll content increase up to 30%. Fig.7 shows that the increase in carotenoid content seems not to be due to an increase of one compound as the pigment pattern remains relatively unchanged. Corresponding results for α-, β- and χ- tocopherols are shown in fig.8, revealing also an induction of about 30%.

The carotenoid (xanthophylls and β-carotene) and tocotrienols (α-, β-, γ- and δ-tocotrienols) content of two independently transformed nosAtRAP2.2-BY-2 cell cultures and of untransformed cell culture was analyzed five times each. The internal standard α-tocopherol was used as a reference to determine the amounts of total carotenoid and tocotrienols/tocopherols. This was feasible, as wildtype BY-2 cell culture do not contain this tocopherol in detectable amounts.

Fig. 9 shows the mean values for the carotenoids and tocotrienols for the two nosAtRAP2.2-BY-2 and wildtype cell cultures. Interestingly, the total carotenoid content shows an increase of up to 30% and is therefore comparable with the increase observed for nosAtRAP2.2-*A. thaliana* (see above). In contrast to the transgenic *A. thaliana* lines, in nosAtRAP2.2-BY-2 cell culture, tocotrienols show the strongest increase of nearly 160% compared to wildtype.

### 7. Cloning of the RAP2.2 homologous from tomato

The ripening process of tomatoes is accompanied by the differentiation of chloroplasts to chromoplasts, accompanied by a massive accumulation of carotenoids. This process is mirrored by a strong increase of PSY1 both on transcript as well as on protein levels (Bartley and Scolnik, 1995). In order to investigate whether RAP2.2 is not only involved in pigment biosynthesis in green systems, but also plays a role in the up-regulation of carotenoid biosynthesis during tomato fruit ripening, the AtRAP2.2 homologous cDNA of tomato (TomRAP2.2) was cloned and used to analyze its regulation during tomato fruit development.

### 7.1. Cloning of RAP 2.1 from L. esculentum

As a template for the cloning of RAP2.2 from *L. esculentum*, a lambda-ZAP cDNA library (mRNA isolated from breaker stage of ripening fruit; Stratagene, USA) was *in vivo* excised according to the manufacture's instructions. Using the primer 5'- ATG TGT GGT GGT GCA ATT CT - 3 and universal and an annealing temperature of 60°C, one specific product of ca. 1200 bp in size was amplified by PCR, isolated and cloned into the vector pBAD-TOPO (Invitrogen, Groningen, The Netherlands), revealing pBAD-TomRAP. The fragment was sequenced and revealed a cDNA with homologies to several ESTs from tomato. Fig. 9 shows the nucleotide and the deduced amino acid sequence of the TomRAP2.2 cDNA cloned from *L. esculentum*. A translation into the aa sequence revealed 52% identity and 60% similarity to AtRAP2.2 (see fig. 10).

### 7.2. Expression of TomRAP2.2

In order to check whether the translation product of pBADTRAP binds to the same *cis*-acting element as AtRAP2.2, the cDNA was expressed in the same background as AtRAP2.2. Therefor, the cDNA was first subcloned into the vector pQE30 by site-directed mutagenesis. For the subcloning, the PCR product with TRAPBam (5' - CAA GCT CGC CCT TGG ATC CGG- 3') and BADasen (5' - GAT TTA ATC TGT ATC AGG- 3') using pBAD-TomRAP as a template, was digested with *Bam*HI and *Hin*dIII and ligated into the vector pQE30 (*Bam*HI/*Hin*dIII), revealing pQE30-TomRAP. For expression, *E. coli* JM109 were transformed with pQE30-TomRAP, induced with 1 mM IPTG and grown for further 3 h at 37°C. The protein could not be detected by Coomassie staining, however the expression of the recombinant His-tagged RAP2.2 was demonstrated by Western blotting using anti-RGS-6*His-antibodies.

### 7.3. Binding Assay

*E. coli* JM109 were transformed with pQE30-TomRAP, pQE30-AtRAP and pQE30, respectively. Dilutions of overnight cultures were spread on agar plates containing 1 mM IPTG and 100 µg/ml ampicillin. After over night incubation, nitrocellulose filter lifts were prepared and incubated for 30 min. in prehybridization buffer (binding buffer containing 1 mM DTT and 5% fat-free milk powder). Filters were incubated in 100 ml hybridization buffer (binding buffer with 0,25 % fat-free milk powder and 10 µg/ml calf thyme DNA) containing 20 pmol of radiolabelled probe. The probe used corresponds to the region from -856 to -825 of the *psy* promoter (see probe C in figs. 1,2,3 and 5).

After an incubation for 2 h at 4°C, the filters were washed three times for 10 min. with washing buffer (binding buffer with 0,25 % fat-free milk powder) and an X-ray film was exposed. The binding assay revealed that both TomRAP2.2 and AtRAP2.2 were able to bind the probe, whereas the control (*E. coli* transformed with the control vector pQE) showed only a weak unspecific binding (see fig. 11). Therefor it can concluded that TomRAP2.2 specifically binds to the same *cis*-acting element as AtRAP2.2.

### 8. Northern-Blot analysis

Total RNA was isolated from tomato leaves and green, breaker stage and ripe red tomato fruits using TRIZOL (GibcoBRL, USA) according to the manufacture's instruction. 10 µg of total RNA each was separated on a 1,2% agarose formaldehyde gel in 1x MOPS buffer (200 mM MOPS, pH 7,0; 50 mM natrium acetate; 10 mM EDTA), blotted on Hybond-N+ nylon membrane (amersham aharmacia, Freiburg, Germany) over night using 10x SSC. RNA was fixed to the membrane by incubating at 80°C for 2 h. Prehybridisation was performed in hybridisation buffer (Sambrock *et al*., 1989) containing 100 µg/ml freshly denatured salmon sperm DNA for 3 h. As a probe, the cDNA of TomRAP2.2 was isolated as *Eco*RI fragment of pQE30-TomRAP and radiolabelled with Klenow exo- (Klenow and Henningsen, 1970). After hybridisation over night at 42°C, the membrane was washed twice with 2xSSC in 0,5 % SDS and one time with 0,2x SSC in 0,5 % SDS at 60 °C and an X-ray film was exposed.

Fig. 12 shows that TomRAP2.2-mRNA amounts are induced during tomato fruit ripening process indicating that RAP2.2 may also be involved also in the chromoplast-specific accumulation of carotenoids.

### 9. Constructs for tomato transformation

The strong induction of TomRAP2.2 indicates that RAP2.2 may be involved also in the massive accumulation of carotenoids during tomato fruit development. Therefor, the overexpression of RAP2.2 in tomato fruits could lead to a further increase of carotenoid content in ripe fruits. In the constructs which will be used for the transformation of tomato plants, RAP2.2 is placed upstream the fibrillin promoter of *C. annuum*, a fruit-specific, strong promoter. These transformations will be carried out using the cDNA of TomRAP2.2 (vector pTCV2001-FibTRAP2.2) and - to avoid possible co-suppression effects - also with the cDNA of AtRAP2.2 (binary vector: pTCV2001-FibAtRAP2.2). VectorNTI Maps of both constructs are attached in the Appendix part (figures 1 and 2, respectively).

### Tables

**Table 1:**

| The ATCTA element in different promoters of plastid-localized proteins | | | | |
|---|---|---|---|---|
| Gene (acc.nr.) Organism | | 1^{st} ATCTA-element | 2^{nd}ATCTA-element | Literature |
| *Carotenoid biosynthesis:* | | | | |
| *DXS (*AL161542) | *A. thaliana* | 9093 | 9494 | |
| *PSY* (Ab005238) | *A. thaliana* | **22637/ 22645** | | |
| *PDS* (U46919) | *L. esculentum* | **656** | | Corona *et al*., 1996 |
| *PDS* (AF039585) | *Z. mays* | 862 | 1003 | Li *et al*., 1996 |

| *Photosynthesis:* | | | | |
|---|---|---|---|---|
| *CAB* (X16436) | *S. alba* | 398/412 | **431/439** | Gauly *et al*., 1992 |
| *CAB1* (J04098) | *A. thaliana* | 902 | **1144** | Ha and An, 1988 |
| *PC* (X16082) | *P. sativum* | 553 | **644/653** | Last *et al*., 1989 |
| *PC* (S67901) | *A. thaliana* | 47 | **385** | Vorst *et al*., 1993 |
| *PC* (Z28347) | *H. vulgare* | 684 | | Nielsen and Gausing, 1993 |

| *Tocopherol biosynthesis:* | | | | |
|---|---|---|---|---|
| *HPD* | *A. thaliana* | 2010993 | 2011487 | |
| (NC_003070) | | | | |
| *GGR* | *A. thaliana* | 46562 | 47439 | |
| (AC011765) | | | | |
| *PT* (AC007651) | *A. thaliana* | 91309 | 92066 | |
| *TMT* (AC006193) | *A. thaliana* | 97643 | | |

The position of the ATCTA is denoted according to the numbering given in the accession numbers. In those cases where more than one ATCTA motif occurred, they were numbered downstream and termed 1^{st} and 2^{nd} ATCTA element; wheras ATCTA elements occurring in tandem manner were considered as a unity. Numbers in bold indicate ATCTA elements of sequences which were used as radiolabelled probes for gel retardation experiments in fig. 4.

*CAB:* chlorophyll a/b-binding protein, *DXS:* 1-deoxy-D-xylulose 5-phosphate synthase, *GGR:* geranylgeranyl reductase, *HPD:* hydroxyphenylpyruvic acid dioxygenase, *PC:* plastocyanin, *PDS:* phytoene desaturase, *PT:* prenyl transferase, *TMT:* γ-tocopherol methyltransferase.

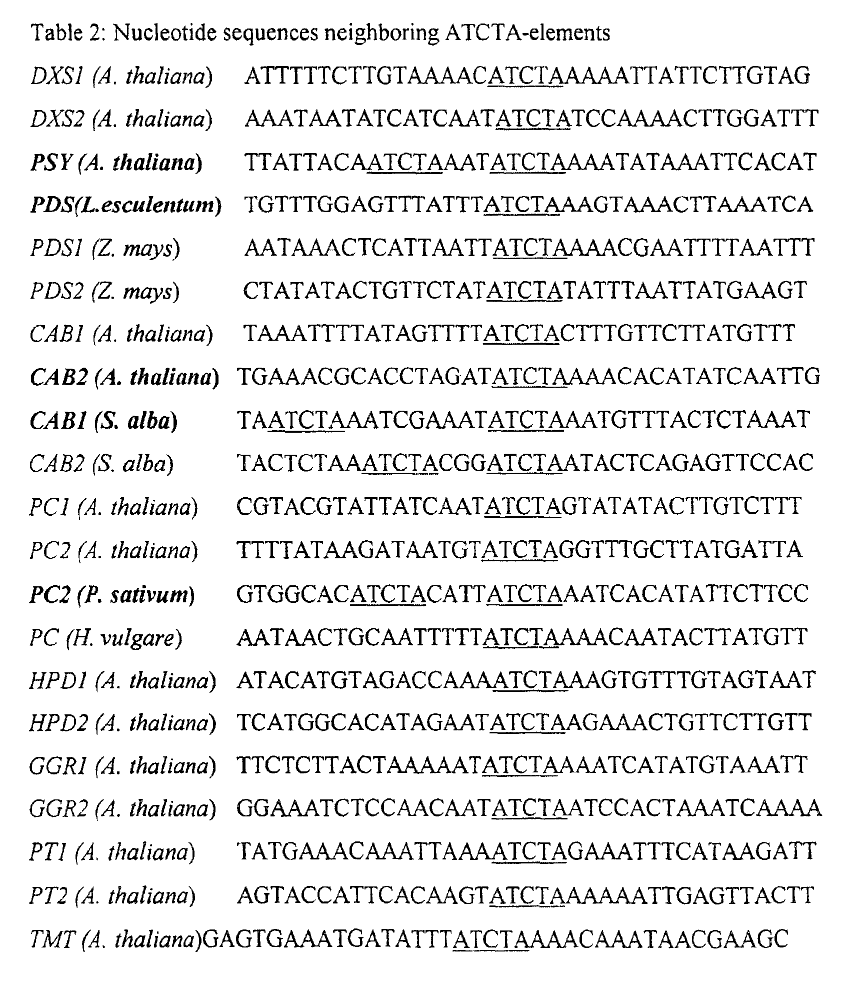

Nucleotide sequences neighboring ATCTA-elements (underligned) of promoters listed in table 1. Numbers following gene names indicate 1^{st} and 2^{nd} ATCTA element, as indicated in table 1. Gene names in bold denote that ATCTA-containing promoter fragments were used as radiolabelled probes for gel retardation experiments in fig. 4. For abbreviations, see table 1.

### LITERATURE CITED

Bartley G.E. and Scolnik P.A. (1995) Plant carotenoids: pigments for photoprotection, visual attraction, and human health. Plant Cell 7: 1027 - 1038.
Bechtold N., Ellis J. and Pelletier G. (1993) *In planta Agrobacterium* mediated gene transfer by infiltration of adult *Arabidopsis thaliana* plants. CR Acad Sci Paris Life Sciences 316: 1194-1199.
Bradford M.M. (1976) A rapid and sensitive method for the quantitation of microgram quantities of protein utilizing the principle of protein-dye binding. Anal Biochem 72: 248-54.
Corona V., Aracri B., Kosturkova G., Bartley G.E., Pitto L., Giorgetti L., Scolnik P. and Giuliano G. (1996) Regulation of a carotenoid biosynthesis gene promoter during plant development. Plant J 9: 505-512.
Dignam J.D., Lebovitz R.M. and Roeder R.G. (1983) Accurate transcription initiation by RNA polymerase II in a soluble extract from isolated mammalian nuclei. Nucleic Acids Res 11: 1475-1489.
Gauly A., Batschauer A., von Arnim A. and Kössel H. (1992) Isolation and characterization of a gene encoding a chlorophyll *a*/*b*-binding protein from mustard and the targeting of the encoded protein to the thylakoid membrane of pea chloroplasts *in vitro.* Plant Mol Biol 19: 277-287.
Ha S.B.B. and An G. (1988) Identification of upstream regulatory elements involved in the developmental expression of the *Arabidopsis thaliana cabl* gene. Proc Natl Acad Sci USA 85: 8017-8021.
Jensen E.O., Marcker K.A., Schell J. and de Bruijn F. (1988) Interaction of a nodule specific, *trans*-acting factor with distinct DNA elements in the soybean leghaemoglobin lbc₃ 5' upstream region. EMBO J 7: 1265-1271.
Kieber J.J., Rothenberg M., Roman G., Feldmann K.A. and Ecker J.R. (1993) CTR1, a negative regulator of the ethylene response pathway in *Arabidopsis,* encodes a member of the raf family of protein kinases. Cell 72: 427-441.
Klenow H. and Henningsen L. (1970) Selective elimination of the exonuclease activity of the deoxyribonucleic acid polymerase from *Escherichia coli* by limited proteolysis. Proc Natl Acad Sci USA 65: 168-175.
Koncz C. and Schell J. (1986) The promoter of T_{L}-DNA gene 5 controls the tissue specific expression of chimeric genes carried by a novel type of *Agrobacterium* binary vector. Mol Gen Genetics 204: 383-396.
Last D.I. and Gray J.C. (1989) Plastocyanin is encoded by a single-copy gene in the pea haploid genome. Plant Mol Biol 12: 655-666.
Nagata T., Nemoto Y. and Hasezawa S. (1992) Tobacco BY-2 cell line as the "HeLa" cell in the cell biology of higher plants. Int Rev Cytol 132: 1-30.
Sambrook J., Fritsch E.F. and Maniatis T. (1989) Molecular cloning: a laboratory manual, (Ed 2) Cold Spring Harbor Laboratory, Cold Spring Harbor, NY.
von Lintig J., Welsch R., Bonk M., Giuliano G., Batschauer A. and Kleinig H. (1997) Light-dependent regulation of carotenoid biosynthesis occurs at the level of phytoene synthase expression and is mediated by phytochrome in *Sinapis alba* and *Arabidopsis thaliana* seedlings. Plant J 12: 625-634.

SEQUENCE LISTING

<110> greenovation GmbH

<120> Method of Altering Plant Secondary Metabolite
      Biosynthesis in Plant Cells

<130> 164

<140> 01122252.8
<141> 2001-09-17

<160> 15

<170> PatentIn Ver. 2.1

<210> 1
<211> 1639
<212> DNA
<213> Arabidopsis thaliana

<220>
<221> CDS
<222> (70)..(1191)
<223> Note: equals "Figure 4" (Section 2), "claims 6, 8, 10 and 27"
      (as originally filed);derived AA SEQ of SEQ ID NOS 1 and 2 equals
      "Figure 4" (Section 2), "claim 5" (as originally filed)

<400> 1
tttcgccccg aagattctat ttctgatcat ttacacccct aaaaagagta gagctttcgt 60

gaagccacc atg tgt gga gga gct ata atc tcc gat ttc ata cct ccg ccg 111
          Met Cys Gly Gly Ala Ile Ile Ser Asp Phe Ile Pro Pro Pro
            1               5                  10

agg tcc ctc cgc gtc act aac gag ttt atc tgg ccg gat ctg aaa aac    159
Arg Ser Leu Arg Val Thr Asn Glu Phe Ile Trp Pro Asp Leu Lys Asn
 15                  20                  25                  30

aaa gtg aaa gct tca aag aag aga tcg aat aag cga tcc gat ttc ttc    207
Lys Val Lys Ala Ser Lys Lys Arg Ser Asn Lys Arg Ser Asp Phe Phe
                 35                  40                  45

gat ctt gac gat gat ttc gaa gct gat ttc caa ggg ttt aag gat gac    255
Asp Leu Asp Asp Asp Phe Glu Ala Asp Phe Gln Gly Phe Lys Asp Asp
             50                  55                  60

tcg gct ttt gac tgc gaa gac gat gat gat gtc ttc gtc aat gtt aag    303
Ser Ala Phe Asp Cys Glu Asp Asp Asp Asp Val Phe Val Asn Val Lys
         65                  70                  75

cct ttc gtc ttc acc gca act act aag ccc gta gct tcc gct ttc gtc    351
Pro Phe Val Phe Thr Ala Thr Thr Lys Pro Val Ala Ser Ala Phe Val
     80                  85                  90

tcc act gta ggt tca gca tat gcc aag aaa act gta gag tcc gct gag    399
Ser Thr Val Gly Ser Ala Tyr Ala Lys Lys Thr Val Glu Ser Ala Glu
 95                 100                 105                 110

caa gct gag aaa tct tct aag agg aag agg aag aat caa tac cga ggg    447
Gln Ala Glu Lys Ser Ser Lys Arg Lys Arg Lys Asn Gln Tyr Arg Gly
                115                 120                 125

att agg cag cgt cct tgg gga aaa tgg ctt gcg gag atc cgt gat ccg    495
Ile Arg Gln Arg Pro Trp Gly Lys Trp Leu Ala Glu Ile Arg Asp Pro
            130                 135                 140

164TF.APP
aga aaa ggc tcc cga gaa tgg ctt gga cat tca cct gct gag gaa gca     543
Arg Lys Gly Ser Arg Glu Trp Leu Gly His Ser Pro Ala Glu Glu Ala
        145                 150                 155

gca aga gct tat gat gct gca gca cgc aga atc cgt ggc acg aaa gct     591
Ala Arg Ala Tyr Asp Ala Ala Ala Arg Arg Ile Arg Gly Thr Lys Ala
    160                 165                 170

aag gtg aat ttt ccc gag gag aag aac cct agc gtc gta tcc cag aaa     639
Lys Val Asn Phe Pro Glu Glu Lys Asn Pro Ser Val Val Ser Gln Lys
175                 180                 185                 190

cgt cct agt gct aag act aat aat ctt cag aaa tca gtg gct aaa cca     687
Arg Pro Ser Ala Lys Thr Asn Asn Leu Gln Lys Ser Val Ala Lys Pro
                195                 200                 205

aac aaa agc gta act ttg gtt cag cag cca aca cat ctg agt cag cag     735
Asn Lys Ser Val Thr Leu Val Gln Gln Pro Thr His Leu Ser Gln Gln
            210                 215                 220

tac tgc aac aac tcc ttt gac aac tct ttt ggt gat atg agt ttc atg     783
Tyr Cys Asn Asn Ser Phe Asp Asn Ser Phe Gly Asp Met Ser Phe Met
        225                 230                 235

gaa gag aag cct cag atg tac aac aat cag ttt ggg tta aca aac tcg     831
Glu Glu Lys Pro Gln Met Tyr Asn Asn Gln Phe Gly Leu Thr Asn Ser
    240                 245                 250

ttc gat gct gga ggt aac aat gga tac cag tat ttc agt tcc gat cag     879
Phe Asp Ala Gly Gly Asn Asn Gly Tyr Gln Tyr Phe Ser Ser Asp Gln
255                 260                 265                 270

ggc agt aac tcc ttc gac tgt tct gag ttc ggg tgg agt gat cac ggc     927
Gly Ser Asn Ser Phe Asp Cys Ser Glu Phe Gly Trp Ser Asp His Gly
                275                 280                 285

cct aaa aca ccc gag atc tct tca atg ctt gtc aat aac aac gaa gca     975
Pro Lys Thr Pro Glu Ile Ser Ser Met Leu Val Asn Asn Asn Glu Ala
            290                 295                 300

tca ttt gtt gaa gaa acc aat gca gcc aag aag ctc aaa cca aac tct     1023
Ser Phe Val Glu Glu Thr Asn Ala Ala Lys Lys Leu Lys Pro Asn Ser
        305                 310                 315

gat gag tca gac gat ctg atg gca tac ctt gac aac gcc ttg tgg gac     1071
Asp Glu Ser Asp Asp Leu Met Ala Tyr Leu Asp Asn Ala Leu Trp Asp
    320                 325                 330

acc cca cta gaa gtg gaa gcc atg ctt ggc gca gat gct ggt gct gtg     1119
Thr Pro Leu Glu Val Glu Ala Met Leu Gly Ala Asp Ala Gly Ala Val
335                 340                 345                 350

act cag gaa gag gaa aac cca gtg gag cta tgg agc tta gat gag atc     1167
Thr Gln Glu Glu Glu Asn Pro Val Glu Leu Trp Ser Leu Asp Glu Ile
                355                 360                 365

aat ttc atg ctg gaa gga gac ttt tgaagtgatc gatggttcct tagtttgtaa     1221
Asn Phe Met Leu Glu Gly Asp Phe
            370

ataaagctgt gttggtgagt tacagttaca agctcttctt gttgaatcgt aaagacaaac 1281

aacctaagtg ttggtttcag gattaatctt ctgtcgttct tttttggttg tttcaggatt 1341

ttgctgttgg gggatggtac aagtcacacc tcaagctcta tgcattggta tctcatgagc 1401

cttctcttcc atagagagtt tctcttttaa ttttgtcgaa ataaaaaagg tgtgatgaag 1461

164TF.APP
taaatagagg tataataata tctatctatt aagtcttgtt ttgttctttc attttgtat 1521

ttcttttcta tttaaaagac agtttattag tcttctgagc tctcttttg atctttgtta 1581

tagcgtatca tcaccctcga aagtgtaatg ttttgtaccc ccaaacttgt ttagcatt 1639

<210> 2
<211> 374
<212> PRT
<213> Arabidopsis thaliana

<400> 2
Met Cys Gly Gly Ala Ile Ile Ser Asp Phe Ile Pro Pro Pro Arg Ser
1               5                   10                  15

Leu Arg Val Thr Asn Glu Phe Ile Trp Pro Asp Leu Lys Asn Lys Val
            20                  25                  30

Lys Ala Ser Lys Lys Arg Ser Asn Lys Arg Ser Asp Phe Phe Asp Leu
        35                  40                  45

Asp Asp Asp Phe Glu Ala Asp Phe Gln Gly Phe Lys Asp Asp Ser Ala
    50                  55                  60

Phe Asp Cys Glu Asp Asp Asp Asp Val Phe Val Asn Val Lys Pro Phe
65                  70                  75                  80

Val Phe Thr Ala Thr Thr Lys Pro Val Ala Ser Ala Phe Val Ser Thr
                85                  90                  95

Val Gly Ser Ala Tyr Ala Lys Lys Thr Val Glu Ser Ala Glu Gln Ala
            100                 105                 110

Glu Lys Ser Ser Lys Arg Lys Arg Lys Asn Gln Tyr Arg Gly Ile Arg
        115                 120                 125

Gln Arg Pro Trp Gly Lys Trp Leu Ala Glu Ile Arg Asp Pro Arg Lys
    130                 135                 140

Gly Ser Arg Glu Trp Leu Gly His Ser Pro Ala Glu Glu Ala Ala Arg
145                 150                 155                 160

Ala Tyr Asp Ala Ala Ala Arg Arg Ile Arg Gly Thr Lys Ala Lys Val
                165                 170                 175

Asn Phe Pro Glu Glu Lys Asn Pro Ser Val Val Ser Gln Lys Arg Pro
            180                 185                 190

Ser Ala Lys Thr Asn Asn Leu Gln Lys Ser Val Ala Lys Pro Asn Lys
        195                 200                 205

Ser Val Thr Leu Val Gln Gln Pro Thr His Leu Ser Gln Gln Tyr Cys
    210                 215                 220

Asn Asn Ser Phe Asp Asn Ser Phe Gly Asp Met Ser Phe Met Glu Glu
225                 230                 235                 240

Lys Pro Gln Met Tyr Asn Asn Gln Phe Gly Leu Thr Asn Ser Phe Asp
                245                 250                 255

Ala Gly Gly Asn Asn Gly Tyr Gln Tyr Phe Ser Ser Asp Gln Gly Ser
            260                 265                 270

Asn Ser Phe Asp Cys Ser Glu Phe Gly Trp Ser Asp His Gly Pro Lys
        275                 280                 285

Thr Pro Glu Ile Ser Ser Met Leu Val Asn Asn Asn Glu Ala Ser Phe

164TF.APP
290                 295                 300

Val Glu Glu Thr Asn Ala Ala Lys Lys Leu Lys Pro Asn Ser Asp Glu
305                 310                 315                 320

Ser Asp Asp Leu Met Ala Tyr Leu Asp Asn Ala Leu Trp Asp Thr Pro
                325                 330                 335

Leu Glu Val Glu Ala Met Leu Gly Ala Asp Ala Gly Ala Val Thr Gln
            340                 345                 350

Glu Glu Glu Asn Pro Val Glu Leu Trp Ser Leu Asp Glu Ile Asn Phe
        355                 360                 365

Met Leu Glu Gly Asp Phe
    370

<210> 3
<211> 1289
<212> DNA
<213> Lycopersicon esculentum

<220>
<221> CDS
<222> (1)..(1098)
<223> Note:equals "Figure 10" (section 2), "claims 3, 8, 9 and 27"
      (as originally filed);derived AA SEQ of SEQ ID NOS 3 and 4 equals
      "Figure 10" (section 2), "claim 2" (as originally filed)

<400> 3
atg tgt ggt ggt gca att atc tcc gat ttg gta cct cct agc cgg att      48
Met Cys Gly Gly Ala Ile Ile Ser Asp Leu Val Pro Pro Ser Arg Ile
  1               5                  10                  15

tct cgc cgg tta acc gct gat ttt cta tgg ggt aca tcc gat ctg aac      96
Ser Arg Arg Leu Thr Ala Asp Phe Leu Trp Gly Thr Ser Asp Leu Asn
             20                  25                  30

aag aag aag aag aac cct agt aat tac cac tca aag ccc ttg agg tct     144
Lys Lys Lys Lys Asn Pro Ser Asn Tyr His Ser Lys Pro Leu Arg Ser
         35                  40                  45

aag ttt att gac ctt gaa gat gaa ttt gaa gct gac ttt cag cac ttc     192
Lys Phe Ile Asp Leu Glu Asp Glu Phe Glu Ala Asp Phe Gln His Phe
     50                  55                  60

aag gat aat tct gat gat gat gat gat gtg aag gca ttt ggc ccc aaa     240
Lys Asp Asn Ser Asp Asp Asp Asp Asp Val Lys Ala Phe Gly Pro Lys
 65                  70                  75                  80

tcc gtg aga tct ggt gat tca aac tgc gaa gct gac aga tcc tcc aag     288
Ser Val Arg Ser Gly Asp Ser Asn Cys Glu Ala Asp Arg Ser Ser Lys
                 85                  90                  95

aga aag agg aag aat cag tac cgg ggg atc aga cag cgt cct tgg ggt     336
Arg Lys Arg Lys Asn Gln Tyr Arg Gly Ile Arg Gln Arg Pro Trp Gly
            100                 105                 110

aag tgg gca gct gaa ata cgt gat cca agg aaa ggt att cga gtc tgg     384
Lys Trp Ala Ala Glu Ile Arg Asp Pro Arg Lys Gly Ile Arg Val Trp
        115                 120                 125

ctt ggt act ttc aat tca gcc gaa gag gca gcc aga gct tat gat gct     432
Leu Gly Thr Phe Asn Ser Ala Glu Glu Ala Ala Arg Ala Tyr Asp Ala
    130                 135                 140

164TF.APP
gag gcg cga agg atc aga ggc aag aaa gct aag gtg aac ttt cct gat 480
Glu Ala Arg Arg Ile Arg Gly Lys Lys Ala Lys Val Asn Phe Pro Asp
145 150 155 160

gaa gct cca gtg tct gtt tca aga cgt gct att aag caa aat ccc caa 528
Glu Ala Pro Val Ser Val Ser Arg Arg Ala Ile Lys Gln Asn Pro Gln
165 170 175

aag gca ctt cgt gag gaa acc ctg aac aca gtt cag ccc aac atg act 576
Lys Ala Leu Arg Glu Glu Thr Leu Asn Thr Val Gln Pro Asn Met Thr
180 185 190

tat att agt aac ttg gat ggt gga tct gat gat tcg ttc agt ttt ttc 624
Tyr Ile Ser Asn Leu Asp Gly Gly Ser Asp Asp Ser Phe Ser Phe Phe
195 200 205

gaa gag aaa cca gca acc aag cag tac ggc ttc gag aat gtg tct ttt 672
Glu Glu Lys Pro Ala Thr Lys Gln Tyr Gly Phe Glu Asn Val Ser Phe
210 215 220

act gct gta gat atg gga ctg ggc tca gtt tcc cct tca gct ggt aca 720
Thr Ala Val Asp Met Gly Leu Gly Ser Val Ser Pro Ser Ala Gly Thr
225 230 235 240

aat gtt tac ttc agc tct gat gaa gca agt aac act ttt gac tgc tct 768
Asn Val Tyr Phe Ser Ser Asp Glu Ala Ser Asn Thr Phe Asp Cys Ser
245 250 255

gat ttc ggt tgg gct gaa ccg tgt gca agg act cca gag atc tca tct 816
Asp Phe Gly Trp Ala Glu Pro Cys Ala Arg Thr Pro Glu Ile Ser Ser
260 265 270

gtt ctg tcg gaa gtt ctg gaa acc aat gag act cat ttt gat gat gat 864
Val Leu Ser Glu Val Leu Glu Thr Asn Glu Thr His Phe Asp Asp Asp
275 280 285

tcc aga cca gag aaa aaa ctg aag tcc tgt tcc agc act tca ttg aca 912
Ser Arg Pro Glu Lys Lys Leu Lys Ser Cys Ser Ser Thr Ser Leu Thr
290 295 300

gtt gac ggt aac act gtg aac acg cta tct gaa gag cta tcg gct ttt 960
Val Asp Gly Asn Thr Val Asn Thr Leu Ser Glu Glu Leu Ser Ala Phe
305 310 315 320

gaa tcc cag ata agt tct tgc aga tcc cat atc tcg agg gaa att ggg 1008
Glu Ser Gln Ile Ser Ser Cys Arg Ser His Ile Ser Arg Glu Ile Gly
325 330 335

atg cat cgg ttg atg cct tcc tca ata caa gtg caa ttc agg atg gtg 1056
Met His Arg Leu Met Pro Ser Ser Ile Gln Val Gln Phe Arg Met Val
340 345 350

gaa acg cca tgg acc ttt ggt cct tcg atg atg tac ctt ctt 1098
Glu Thr Pro Trp Thr Phe Gly Pro Ser Met Met Tyr Leu Leu
355 360 365

taatgggagg tgcctactaa gctgcataca catcttccct tgctaagttt tgtaaataac 1158

gcttcatttg agtgaagttt gcgcctgcgt ttacgtttat caccaaacta aaagactata 1218

tatgtgttgt attaatttat tcaaaattta ctcgtttgat atatgtaagt atgtatcctt 1278

gttttcataa a 1289

<210> 4
<211> 366
<212> PRT

164TF.APP
<213> Lycopersicon esculentum

<400> 4
Met Cys Gly Gly Ala Ile Ile Ser Asp Leu Val Pro Pro Ser Arg Ile
1               5                   10                  15

Ser Arg Arg Leu Thr Ala Asp Phe Leu Trp Gly Thr Ser Asp Leu Asn
            20                  25                  30

Lys Lys Lys Lys Asn Pro Ser Asn Tyr His Ser Lys Pro Leu Arg Ser
        35                  40                  45

Lys Phe Ile Asp Leu Glu Asp Glu Phe Glu Ala Asp Phe Gln His Phe
    50                  55                  60

Lys Asp Asn Ser Asp Asp Asp Asp Asp Val Lys Ala Phe Gly Pro Lys
65                  70                  75                  80

Ser Val Arg Ser Gly Asp Ser Asn Cys Glu Ala Asp Arg Ser Ser Lys
                85                  90                  95

Arg Lys Arg Lys Asn Gln Tyr Arg Gly Ile Arg Gln Arg Pro Trp Gly
            100                 105                 110

Lys Trp Ala Ala Glu Ile Arg Asp Pro Arg Lys Gly Ile Arg Val Trp
        115                 120                 125

Leu Gly Thr Phe Asn Ser Ala Glu Glu Ala Ala Arg Ala Tyr Asp Ala
    130                 135                 140

Glu Ala Arg Arg Ile Arg Gly Lys Lys Ala Lys Val Asn Phe Pro Asp
145                 150                 155                 160

Glu Ala Pro Val Ser Val Ser Arg Arg Ala Ile Lys Gln Asn Pro Gln
                165                 170                 175

Lys Ala Leu Arg Glu Glu Thr Leu Asn Thr Val Gln Pro Asn Met Thr
            180                 185                 190

Tyr Ile Ser Asn Leu Asp Gly Gly Ser Asp Asp Ser Phe Ser Phe Phe
        195                 200                 205

Glu Glu Lys Pro Ala Thr Lys Gln Tyr Gly Phe Glu Asn Val Ser Phe
    210                 215                 220

Thr Ala Val Asp Met Gly Leu Gly Ser Val Ser Pro Ser Ala Gly Thr
225                 230                 235                 240

Asn Val Tyr Phe Ser Ser Asp Glu Ala Ser Asn Thr Phe Asp Cys Ser
                245                 250                 255

Asp Phe Gly Trp Ala Glu Pro Cys Ala Arg Thr Pro Glu Ile Ser Ser
            260                 265                 270

Val Leu Ser Glu Val Leu Glu Thr Asn Glu Thr His Phe Asp Asp Asp
        275                 280                 285

Ser Arg Pro Glu Lys Lys Leu Lys Ser Cys Ser Ser Thr Ser Leu Thr
    290                 295                 300

Val Asp Gly Asn Thr Val Asn Thr Leu Ser Glu Glu Leu Ser Ala Phe
305                 310                 315                 320

Glu Ser Gln Ile Ser Ser Cys Arg Ser His Ile Ser Arg Glu Ile Gly
                325                 330                 335

Met His Arg Leu Met Pro Ser Ser Ile Gln Val Gln Phe Arg Met Val
            340                 345                 350

164TF.APP

Glu Thr Pro Trp Thr Phe Gly Pro Ser Met Met Tyr Leu Leu
        355                 360                 365

<210> 5
<211> 379
<212> PRT
<213> Artificial Sequence

<220>
<223> Note: equals "consensus sequence depicted in Figure 11" (section 2),
      "consensus sequence in claim 1" (as originally filed)

<220>
<223> Description of Artificial Sequence: consensus sequence; X=Lys,
      Arg,His,Asp,Glu,Asn,Gln,Ser,Thr,Tyr,Gly,Ala,Val,Leu,Ile,Pro,Phe,
      Met,Trp or Cys; X do not represent parts of the consensus sequence

<220>
<221> NON_CONS
<222> 10,(14)..(16),18,22,(27)..(29),32,33,35,(37)..(43),(45)..(46),
      49,51,63,(67)..(72),(79)..(106),108,111,(114..116),141,151,153,
      (157..160),171,178,(188..190),(193..204),(206..233),(236..238),
      (240..242),244,245,(247..255),(258..262),266,(268..270),276,
      (291..293),(303..308),311,312,314,(318..320),325,326,328,329,
      331,332,334,(336..338),340,342,(344..348),351,353,(355..358),
      361,(363..368),370,(372..374),378

<400> 5
Met Cys Gly Gly Ala Ile Ile Ser Asp Xaa Ile Pro Pro Xaa Xaa Xaa
  1               5                  10                  15

Ser Xaa Arg Leu Thr Xaa Asp Phe Ile Trp Xaa Xaa Xaa Asp Leu Xaa
             20                  25                  30

Xaa Lys Xaa Lys Xaa Xaa Xaa Xaa Xaa Xaa Xaa Lys Xaa Xaa Arg Ser
        35                  40                  45

Xaa Phe Xaa Asp Leu Asp Asp Asp Phe Glu Ala Asp Phe Gln Xaa Phe
    50                  55                  60

Lys Asp Xaa Xaa Xaa Xaa Xaa Xaa Asp Asp Asp Asp Asp Val Xaa Xaa
 65                  70                  75                  80

Xaa Xaa Xaa Xaa Xaa Xaa Xaa Xaa Xaa Xaa Xaa Xaa Xaa Xaa Xaa Xaa
                 85                  90                  95

Xaa Xaa Xaa Xaa Xaa Xaa Xaa Xaa Xaa Xaa Ala Xaa Lys Ser Xaa Asp
            100                 105                 110

Ser Xaa Xaa Xaa Ala Asp Lys Ser Ser Lys Arg Lys Arg Lys Asn Gln
        115                 120                 125

Tyr Arg Gly Ile Arg Gln Arg Pro Trp Gly Lys Trp Xaa Ala Glu Ile
    130                 135                 140

Arg Asp Pro Arg Lys Gly Xaa Arg Xaa Trp Leu Gly Xaa Xaa Xaa Xaa
145                 150                 155                 160

Ala Glu Glu Ala Ala Arg Ala Tyr Asp Ala Xaa Ala Arg Arg Ile Arg
                165                 170                 175

Gly Xaa Lys Ala Lys Val Asn Phe Pro Asp Glu Xaa Xaa Xaa Ser Val
            180                 185                 190

164TF.APP
Xaa Xaa Xaa Xaa Xaa Xaa Xaa Xaa Xaa Xaa Xaa Xaa Leu Xaa Xaa Xaa
        195                 200                 205

Xaa Xaa Xaa Xaa Xaa Xaa Xaa Xaa Xaa Xaa Xaa Xaa Xaa Xaa Xaa Xaa
    210                 215                 220

Xaa Xaa Xaa Xaa Xaa Xaa Xaa Xaa Xaa Phe Asp Xaa Xaa Xaa Ala Xaa
225                 230                 235                 240

Xaa Xaa Phe Xaa Xaa Glu Xaa Xaa Xaa Xaa Xaa Xaa Xaa Xaa Xaa Gly
                245                 250                 255

Leu Xaa Xaa Xaa Xaa Xaa Ala Ala Gly Xaa Asn Xaa Xaa Xaa Tyr Phe
            260                 265                 270

Ser Ser Asp Xaa Ala Ser Asn Ser Phe Asp Cys Ser Asp Phe Gly Trp
        275                 280                 285

Ala Asp Xaa Xaa Xaa Lys Thr Pro Glu Ile Ser Ser Met Leu Xaa Xaa
    290                 295                 300

Xaa Xaa Xaa Xaa Asn Glu Xaa Xaa Phe Xaa Asp Asp Ser Xaa Xaa Xaa
305                 310                 315                 320

Lys Lys Leu Lys Xaa Xaa Ser Xaa Xaa Ser Xaa Xaa Leu Xaa Ala Xaa
                325                 330                 335

Xaa Xaa Asn Xaa Leu Xaa Asp Xaa Xaa Xaa Xaa Xaa Glu Ala Xaa Ile
            340                 345                 350

Xaa Ala Xaa Xaa Xaa Xaa Ile Ser Xaa Glu Xaa Xaa Xaa Xaa Xaa Xaa
        355                 360                 365

Leu Xaa Ser Xaa Xaa Xaa Ile Asn Phe Xaa Leu
    370                 375


<210> 6
<211> 24
<212> DNA
<213> Artificial Sequence

<220>
<223> Description of Artificial Sequence: Primer depicted on page 49,
      line 8 (as originally filed)

<400> 6
caatctaaat atctaaaata taaa                                          24


<210> 7
<211> 24
<212> DNA
<213> Artificial Sequence

<220>
<223> Description of Artificial Sequence: Primer depicted on page 49,
      lines 8,9 (as originally filed)


<400> 7
tttatatttt agatatttag attg                                          24


<210> 8
<211> 20
<212> DNA
<213> Artificial Sequence

164TF.APP

<220>
<223> Description of Artificial Sequence: Primer Psyasen depicted on page 41,
      line 13 (as originally filed)

<400> 8
cgtagattgc ccaaatcgcc                                                    20


<210> 9
<211> 20
<212> DNA
<213> Artificial Sequence

<220>
<223> Description of Artificial Sequence: Primer Psysen depicted on page 41,
      line 13 (as originally filed)


<400> 9
ttgtgggttg gtaagggttc                                                    20


<210> 10
<211> 18
<212> DNA
<213> Artificial Sequence

<220>
<223> Description of Artificial Sequence: Primer RAPasen depicted on page 49,
      lines 26,27 (as originally filed)


<400> 10
gccacggatt ctgcgtgc                                                      18


<210> 11
<211> 20
<212> DNA
<213> Artificial Sequence

<220>
<223> Description of Artificial Sequence: Primer depicted on page 50,
      lines 6,7 (as originally filed)


<400> 11
cgtgaaggat ccatgtgtgg                                                    20


<210> 12
<211> 20
<212> DNA
<213> Artificial Sequence

<220>
<223> Description of Artificial Sequence: Primer depicted on page 54,
      lines 7,8 (as originally filed)


<400> 12
atgtgtggtg gtgcaattct                                                    20


<210> 13
<211> 21
<212> DNA

164TF.APP
<213> Artificial Sequence

<220>
<223> Description of Artificial Sequence: Primer TRAPBam depicted on page 54,
      line 20 (as originally filed)


<400> 13
caagctcgcc cttggatccg g                                                21


<210> 14
<211> 18
<212> DNA
<213> Artificial Sequence

<220>
<223> Description of Artificial Sequence: Primer BADasen depicted on page 54,
      line 21 (as originally filed)


<400> 14
gatttaatct gtatcagg                                                    18


<210> 15
<211> 2462
<212> DNA
<213> Arabidopsis thaliana

<220>
<221> misc_feature
<222> (1)..(2462)
<223> Note: equals "Figure 1" (section 1)(as originally filed)

<220>
<221> 5'UTR
<222> (1)..(1746)

<220>
<221> misc_feature
<222> (1747)..(1933)
<223> exon 1

<220>
<221> intron
<222> (1934)..(2307)

<220>
<221> misc_feature
<222> (2308)..(2459)
<223> exon 2

<220>
<221> misc_signal
<222> (2460)..(2462)
<223> Start codon of phytoene synthase


<400> 15
tctagaacta gggcggttga cataaatttc gtacgaaaac aatactttta tcattcagta 60
ctaaaccaaa aaaagcggtt tctttacgag agaatgtatt tgaaacaaga tacggagaaa 120
atacggacca gactaaaaat aacaaaggat aaaaccaaaa tcaagaagaa acttaaaaga 180
caaaaccttc tgagcgcaag agagagggcc aagttgcgaa gacagtatgg gacccaaata 240
ccgactaagc ggaaatctaa cttttaactt ttcctaccaa tgaaacattg acacgtggaa 300
aagcaacgtc gcccacttta ccctccgcac tttgaaatct ttgataagct tatcgctctc 360
ccattggccg taggctccac gtggcatctc tctgatctct cttctgctaa tcctttttta 420
cccaaactgg catgacccca cccctaaacc cctattgtcc tttctaccaa caattactta 480

164TF.APP
atgacttata taatattcta attatatttt aattatgtaa ttaaataact attgatccca 540
tctccatagt gacatgtctc actttttctt ccacaacaaa atgcgtcact cttgattcat 600
catgttcgta gcaaaatcca cgttattaca aaaaacgtta tacattttga aaggtaatcc 660
ataaaatagt gagatggatc aagttttcta cgcaaccctc gtagaatttt agcaatgatc 720
caaagctact gtatatatat agttacgttt cgtaacacaa catgtatttt accatgatta 780
tgcctctttt tttttctttt ccacaacata tatgtattta catccttatt tggtttaggc 840
tgagtttgtt aacatagcat caccaatagg gacaattttt tttttttatta caatctaaat 900
atctaaaata taaattcaca tgaaattaat gtttctaata gagttcttca atcttcatgt 960
acaattgcac atttttgtga aattattatt actttatcat tttttcttta aaattaattc 1020
accgagtacc gataaatgtt tatattgaat acgatttagt aaacgacata tatgtttcgt 1080
cacattttta tcactatttg taatatatct gttaaatttg atgatgtagt ttctgttttt 1140
gaattgaaaa cttacaaagt ttagaaaaaa gaatgtaaat tagaaataaa tacactttct 1200
gtatttttga atactataat aggctaaaat cacatcattt tctaatgttg ctttaaataa 1260
tatatatcaa ataaaggctt aaattacatt tatataagaa atattgtaat acaaatatca 1320
atttagagct attaatttac aattacaacc tgttgcccaa ttgatgaata aatcaaacat 1380
gtaattaacc aaactttcta attgatcagg aatcaattta gctaaataat aagacataaa 1440
tttggccaag aaaaagaaga gaaaacatgt taaataggta atgggtttta atattgggat 1500
agttaccttt atttgaaaac tattaaggtt tgatttaaga cacgagtcac tcgagagtcg 1560
catctcgcca gtcaaaaact aaattaaaaa actatataga cccaaaaaat aaaaataacg 1620
acttttgtat ttatactggc aagattctca ttaaaaccag caaaaaaaaa aaaaaaagac 1680
caaagaagga agaaaatttg agtgggtgag aattttttac gatagaggaa gagacagcat 1740
catctacttg tgttgtctgt gtacatatat tacagtaagc gttgcaatat aactttgagg 1800
atcttctcac attaatggct gtcaaacctt ttgctcttcc ttttgattaa tttagtgttt 1860
gacaattcct cctccttctc cttcttcttc caaagttttg tcgcagtatc tattgttctt 1920
acagagagaa aggtagcttt tgtgtccatc actcattttg cgctcgtttg gttaagcttc 1980
atctgccatg tggttcactg ttttgaactt ttgggctctt atacctaatt gttgatgtaa 2040
cacaacgata cataatctaa tttgttttcg agtggaaata gttgaaccca gttgagtata 2100
ttagccaata cgattagagg atttacttgt tttttctctt ttcatgtctt catttgcttt 2160
tgaagatctt tcctctttat gtttgtgtct cttcgctatt ttacttgctt gaagaattga 2220
gtttctcatc tcgcttaaaa tttggcttta tatacagatt tagagatctc gattcctaat 2280
ctactttttt tgtattcaat ttgcaggaag agctttagtc ttttaccagt ttgatccaat 2340
tctgggtttc actgaaaaaa gttgggagtt tgattcttca taactgtaga agaaacagag 2400
tcaacagaag aaaactaaaa aagttgagat ttttctctca cgcgctcaag aacttgagta 2460
tg 2462

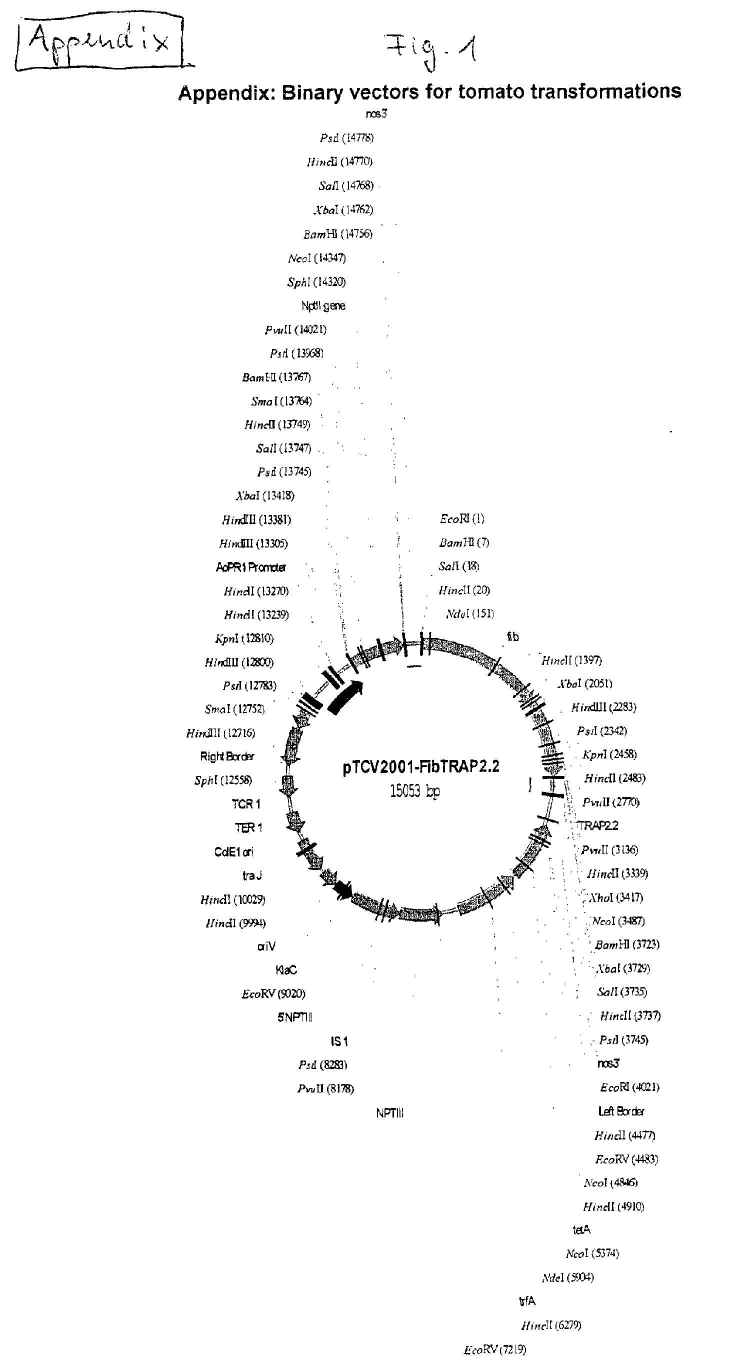

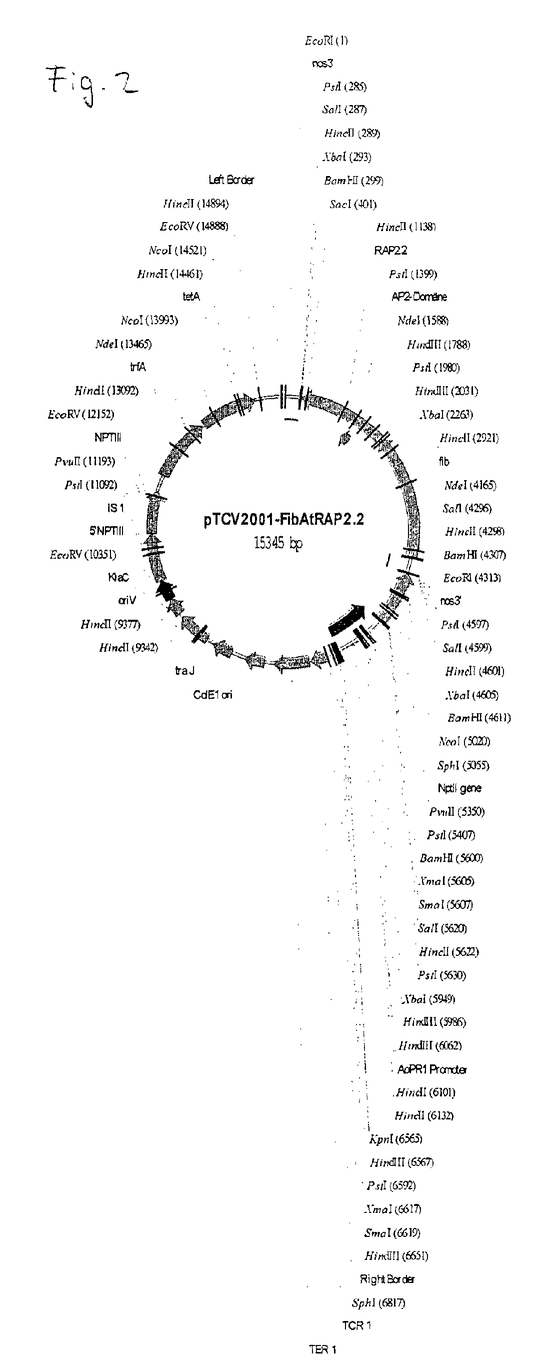

## Claims

1. An isolated polynucleotide encoding a polypeptide comprising the consensus amino acid sequence shown in Figure 11 and capable of binding to a promoter region comprising at least one ATCTA domain wherein said promoter region is operably associated with a coding sequence of a gene of a carotenoid synthesis pathway and/or a tocotrienol/tocopherol synthesis pathway such that when the said polypeptide is bound to the promoter region carotenoid and/or tocotrienol/tocopherol synthesis is enhanced.

2. An isolated polynucleotide according to claim 1 wherein the polypeptide has the tomato polypeptide sequence shown in Figure 10.

3. An isolated polynucleotide according to claim 2 wherein the polynucleotide has the coding sequence shown in Figure 10.

4. An isolated polynucleotide according to claim 1 comprising genomic DNA.

5. An isolated polynucleotide according to claim I wherein the polypeptide has the Arabidopsis thaliana polypeptide sequence shown in Figure 4.

6. An isolated polynucleotide according to claim 5 wherein the polynucleotide has the coding sequence shown in Figure 4.

7. An isolated polynucleotide according to claim 5 comprising genomic DNA.

8. An isolated polynucleotide encoding a polypeptide which binds to at least one ATCTA DNA motif of a promoter that functions in a plant carotenoid and/or tocotrienol/tocopherol synthesis pathway, which polynucleotide selectively hybridizes under stringent conditions with a probe which is the complement of the a *RAP2.2* coding nucleotide sequence shown in Figure 4 or 10 said stringent conditions comprising hybridization overnight at 42°C in 0.25M Na₂HPO₄ pH7.2, 6.5% SDS, 10% dextran sulfate and a final wash at 55°C in 0.1xSSC, 0.1%SDS.

9. An isolated polynucleotide encoding a polypeptide which functions in a plant carotenoid and/or tocotrienol/tocopherol synthesis pathway, which polynucleotide comprises tomato cDNA sequence comprising the sequence identified in Figure 10.

10. An isolated polynucleotide encoding a polypeptide which functions in a plant carotenoid and/or tocotrienol/tocopherol synthesis pathway, which polynucleotide comprises tomato cDNA sequence comprising the sequence identified in Figure 4.

11. A nucleic acid vector suitable for transformation of a plant cell and including a polynucleotide according to any one of claims 1 to 10.

12. A host cell containing a heterologous polynucleotide or nucleic acid vector according to any one of claims 1 to 11.

13. A host cell according to claim 12 which is microbial.

14. A host cell according to claim 13 which is a plant cell.

15. A plant cell according to claim 14 having heterologous said polynucleotide within its chromosome.

16. A plant cell according to claim 14 or 15 which is comprised in a plant, a plant part or a plant propagule, or an extract or derivative of a plant.

17. A method of producing a cell according to any of claims 12 to 16, the method including incorporating said polynucleotide or nucleic acid vector into the cell by means of transformation.

18. A method according to claim 17 which includes recombining the polynucleotide with the cell genome nucleic acid such that it is stably incorporated therein.

19. A method according to claim 17 or claim 18 which includes regenerating a plant from one or more transformed cells.

20. A plant comprising a plant cell according to claim 14 or 15.

21. A part or propagule of a plant comprising a plant cell according to claim 14 or 15.

22. A method of producing a plant, the method including incorporating a polynucleotide or nucleic acid vector according to any of claims 1 to 10 into a plant cell and regenerating a plant from said plant cell.

23. A method according to claim 22 including sexually or asexually propagating or growing off-spring or a descendant of the plant regenerated from said plant cell.

24. A method of influencing a characteristic of a plant, the method including causing or allowing transcription from a heterologous polynucleotide according to any of claims 1 to 10 within cells of the plant.

25. Use of a polynucleotide according to any of claims I to 10 in the production of a transgenic plant.

26. A method of identifying or obtaining a polynucleotide encoding a polypeptide which functions in a plant carotenoid and/or tocotrienol/tocopherol synthesis pathway, the method including screening candidate nucleic acid using a nucleic acid molecule which selectively hybridises under stringent conditions with a polynucleotide according to claim 8 or the complement thereof.

27. A nucleic acid probe or primer which has a nucleotide sequence of at least about 20-30 nucleotides, which sequence is shown in, or is complementary to, the coding region of Figure 4 or Figure 10.

28. A pair of primers according to claim 27 suitable for amplification of a fragment of the coding region of a polynucleotide according to any one of claims 1 to 10.

29. A method for identifying in or obtaining from a plant or plant cell a polynucleotide encoding a polypeptide which functions in a plant carotenoid and/or tocotrienol/tocopherol synthesis pathway, which method employs a nucleic acid probe or primer according to claim 27 or claim 28.

30. A method as claimed in claim 29 including:
(a) providing a nucleic acid preparation from a plant cell;
(b) providing a probe according to claim 27;
(c) contacting the nucleic acid preparation with the probe under conditions for selective hybridisation of the probe to any nucleic acid encoding a said polypeptide;
(d) identifying said nucleic acid encoding a said polypeptide if present by its hybridisation with the probe; and optionally
(e) confirming the identity of said polypeptide encoded by the nucleic acid by expression in an expression system and determination of ability to function in a plant carotenoid and/or tocotrienol/tocopherol synthesis pathway.

31. A method according to claim 30 including:
(a) providing a preparation of nucleic acid from a plant cell;
(b) providing a pair of primers according to claim 28;
(c) contacting nucleic acid in said preparation with said primers under conditions for performance of PCR;
(d) performing PCR and determining the presence or absence of an amplified PCR product; and optionally
(e) confirming the identity of the amplified PCR product by expression in an expression system to produce a polypeptide and determination of ability of the produced polypeptide to function in a plant carotenoid and/or tocotrienol/tocopherol synthesis pathway.

32. An isolated polypeptide encoded by a polynucleotide according to any of claims 1 to 10.

33. An isolated antibody including an antigen-binding site with specific binding affinity for the polypeptide according to claim 32.

34. A polypeptide including the antigen-binding site of an antibody according to claim 33.

35. A method of identifying or obtaining a polypeptide according to claim 32, the method including screening candidate polypeptides with an antibody or polypeptide according to claim 33 or claim 34.
